# EUROPEAN PATENT APPLICATION

(11) **EP 4 349 867 A1**
(43) Date of publication of application: **10.04.2024**
(21) Application number: 22810551.6
(22) Date of filing: 24.05.2022
(51) Int. Cl.: C07K 16/46, A61K 39/395, C07K 16/28, C12N 15/13

(54) **SPECIFIC BINDING PROTEIN TARGETING PD-L1 AND CD73**

(30) Priority: 28.05.2021 CN 202110594756
(71) Applicant: Harbour Biomed (Shanghai) Co., Ltd, Shanghai 201203 (CN)
(72) Inventor: SHAN, Qianqian, Shanghai 201203 (CN); ZHANG, Xuekun, Shanghai 201203 (CN); GAN, Xin, Shanghai 201203 (CN); LUO, Haishan, Shanghai 201203 (CN); SHI, Lei, Shanghai 201203 (CN); WANG, Yongqiang, Shanghai 201203 (CN); JIA, Xingxing, Shanghai 201203 (CN); WANG, Rongchao, Shanghai 201203 (CN); RONG, Yiping, Shanghai 201203 (CN)
(74) Representative: Latscha Schöllhorn Partner AG
(86) International application number: PCT/CN2022/094703
(87) International publication number: WO 2022/247826

(57) **Abstract**

The present invention relates to a specific binding protein targeting PD-L1 and CD73, wherein the specific binding protein is capable of specifically binding to PD-L1 and/or a fragment thereof, and CD73 and/or a fragment thereof. The present invention further relates to use of the specific binding protein in the treatment, prevention and/or diagnosis of diseases such as immune diseases, acute and chronic inflammatory diseases, and tumor diseases.

## Description

### TECHNICAL FIELD

The present invention relates to the field of biomedicine, and in particular to the field of antibody treatment.

### BACKGROUND

Programmed death ligand 1 (PD-L1) one of two cell surface glycoprotein ligands of programmed death receptor 1 (PD-1) (the other is PD-L2), down regulates T cell activation and cytokine secretion when binding to PD-1. PD-L1, also known as a cluster of differentiation 274 (CD274) or B7 homologue 1 (B7-H1), is a 40 kDa type I transmembrane protein, and the PD-L1 contains an IgV (immunoglobulin variable region)-like region, an IgC (immunoglobulin constant region)-like region, a transmembrane region, and a cytoplasmic tail region, wherein the cytoplasmic tail region is associated with intracellular signal transduction; and the IgV region and the IgC region are associated with intercellular signal transduction. When the PD-L1 binds to the PD-1, protein tyrosine phosphatase containing a Src homology region 2 (SHP-2) is recruited, so that zeta-chain-related protein (ZAP70) phosphorylation induced by lymphocyte specific protein tyrosine kinase (LCK) is weakened; a RAS-MEK-ERK/PI3K-Akt-mTOR pathway is inhibited; transcription of T cells is further effectively inhibited; the immune function of the T cells is finally inhibited, and the PD-L1 plays an important role in negative regulation of immune response. Therefore, it is generally considered that blocking the PD-1/PD-L1 signaling pathway can up regulate the activation of T cells, activate endogenous anti-tumor immune response, and thus exert a therapeutic effect on tumors.

The first-generation immune checkpoint inhibitors represented by PD-1/PD-L1 and CTLA-4 monoclonal antibodies have gradually become the representative of tumor immunotherapy, and particularly play an important role in adoptive cell transfer therapy and immune checkpoint blockade (ICB). Tumor immunotherapy targeting the PD-1/PD-L1 has been approved for treatment of a variety of different solid tumors, and received the Nobel Prize in Physiology or Medicine in 2018. However, clinical data from anti-PD-1/PD-L1 treatment show limited response rates. Some patients developed primary resistance and did not respond to PD-1/PD-L1 treatment, and some responders developed acquired resistance after the initial response.

The search for new-generation tumor immune targets which are safe and effective and can generate synergistic effects with PD-1/PD-L1 is currently the focus of attention in tumor immunotherapy, and therefore, a series of immunosuppressive or agonistic targets such as LAG3, TIGIT, TIM3, CD47, OX40, 4-1BB and the like emerge. White blood cell differentiation antigen 73 (CD73), also known as 5'-nucleotidase, is an enzyme encoded by a NT5E gene in the human body. The CD73 is a dimer consisting of two identical 70-KD subunits, linked to the outer surface of a plasma membrane by glycosyl phosphatidyl inositol. It is known that the CD73 catalyzes the dephosphorylation of extracellular monophosphate nucleosides to nucleosides, such as adenosine. The accumulation of extracellular adenosine in cancer tissue constitutes an important mechanism for tumor immune escape. Among other effects, tumor-derived adenosine largely inhibits infiltrating effector T cells through A2A receptors activated by adenosine cyclase (Ohta et al., (2006) Proc Natl Acad Sci USA 103:13132-13137). The CD73 has been reported to be expressed in a variety of tumor cells, including leukemia, bladder cancer, glioma, glioblastoma, ovarian cancer, melanoma, prostate cancer, thyroid cancer, esophageal cancer and breast cancer (Jin et al., Cancer Res 2010; 70:2245-55 and Stagg et al., PNAS 2010; 107:1547-52).

For the treatment of solid tumors, for purposes of overcoming drug resistance and improving the curative effect, one important aspect is to relieve the inhibitory effects of the tumor micro-environment (TME) on immune effector cells. TME is a very complex system consisting of various cells, intercellular matrix, enzymes, cytokines, metabolites, and the like. TME has the characteristics of significant low hydrogen, low pH and high pressure, and is very different from normal tissue. There exists one immunosuppressive mechanism of great importance which is mediated by a CD73-adenosine metabolic signaling pathway. Adenosine can inhibit the immune killing effects of T cells through an adenosine receptor (A2AR), so that tumors can achieve immune escape, and the CD73 is a key enzyme that catalyzes the production of adenosine.

The immune checkpoint blockade of the PD-1/PD-L1 has become a milestone for immunotherapy. However, although anti-PD-1/PD-L1 treatment has shown impressive efficacy in solid tumor therapy, a long lasting response only occurs in a small fraction of patients, and some patients who initially respond to treatment eventually develop acquired resistance, and therefore, there is an urgent need to develop more effective PD-1/PD-L1 inhibitors and tumor immune targets that can produce synergistic effects with PD-1/PD-L1.

### SUMMARY

The present invention provides an antigen-binding protein capable of specifically binding to PD-L1 or a fragment thereof, which is capable of blocking the binding of PD-1 to the PD-L1. The invention also provides a multispecific binding protein capable of binding to one or more antigens with high affinity and high specificity, and the antigens are PD-L1 or a fragment thereof, and/or CD73 or a fragment thereof. The invention also provides a nucleic acid molecule encoding the antigen-binding protein and the multispecific binding protein, an expression vector for producing the antigen-binding protein and the multispecific binding protein, host cells, and methods for preparing the antigen-binding protein and the specific binding protein. The invention also relates to the use of the antigen-binding protein and multispecific binding protein in the treatment, prevention and/or diagnosis of diseases, such as immune diseases, acute and chronic inflammatory diseases, and tumor diseases.

In a first aspect, the present invention provides an isolated antigen-binding protein specifically binding to PD-L1 or a fragment thereof.

The isolated antigen-binding protein specifically binding to PD-L1 or a fragment thereof has one or more of the following properties: (a) capable of binding to human PD-L1 and/or cynomolgus monkey PD-L1 with high affinity; (b) having an inhibitory effect on a PD-1 signaling pathway; (c) capable of enhancing activation of T lymphocytes; and (d) showing *in vivo* tumor inhibitory activity.

In some embodiments, the isolated antigen-binding protein specifically binding to PD-L1 and/or a fragment thereof comprises an antibody heavy chain variable region VH, and the VH comprises the following complementary determining regions, or mutants thereof:
HCDR1 set forth in an amino acid sequence of SEQ ID NO: 9;
HCDR2 set forth in an amino acid sequence of SEQ ID NO: 23; and/or
HCDR3 set forth in an amino acid sequence of SEQ ID NO: 36.

The mutants are an insertion, deletion or substitution of 1, 2, 3 or 4 amino acids on the amino acid sequences of the HCDR1, HCDR2 and HCDR3 of the VH. It should be known to those skilled in the art that insertion, deletion or substitution mutations of 1, 2, or 3 amino acids can be performed on 1, 2, or 3 CDRs in the HCDR1, the HCDR2, and the HCDR3, respectively. For example, a mutation of 1 amino acid may be performed on the HCDR1, but no amino acid mutations on the HCDR2 and the HCDR3; or a mutation of 1 amino acid may be performed on the HCDR1 and the HCDR2, respectively, but no amino acid mutations on the HCDR3.
In some embodiments, the HCDR1 has an amino acid sequence of GFX₁FSX₂Y; the HCDR2 has an amino acid sequence of X₃YX₄GX₅X₆, and the HCDR3 has an amino acid sequence of NRAX₇FGVX₈PDX_{g}SDI, wherein X₁, X₂, X₃, X₄, X₅, X₆, X₇, X₈, or X₉ is any one selected from N, T, D, S, W, R, K, E, I, A, V and L. In some embodiments, X₁ is T, D or N; X₂ is N or S; X₃ is W or R; X₄ is D or T; X₅ is T or S; X₆ is K, R or E; X₇ is I or L; X₈ is V or I; and/or, X₉ is A or D.

In some embodiments, the amino acid sequence of the HCDR1 is GF(N/T/D)FS(N/S)Y In some embodiments, the amino acid sequence of the HCDR2 is (W/R)Y(D/T)G(T/S)(K/R/E). In some embodiments, the amino acid sequence of the HCDR3 is NRA(I/L)FGV(V/I)PD(A/D)SDI.

In some embodiments, the mutant of the HCDR1 has an amino acid sequence set forth in any one of SEQ ID NO: 10, SEQ ID NO: 11, SEQ ID NO: 13 and SEQ ID NO: 14.

In some embodiments, the mutant of the HCDR2 has an amino acid sequence set forth in SEQ ID NO: 24 or SEQ ID NO: 27.

In some embodiments, the mutant of the HCDR3 has an amino acid sequence set forth in any one of SEQ ID NO: 39, SEQ ID NO: 40 and SEQ ID NO: 41.

Preferably, the isolated antigen-binding protein specifically binding to PD-L1 and/or a fragment thereof comprises an antibody heavy chain variable region VH, and the VH comprises the following complementary determining regions, or mutants thereof:
HCDR1 set forth in an amino acid sequence of any one of SEQ ID NOs: 9-11 and SEQ ID NOs: 13-14;
HCDR2 set forth in an amino acid sequence of any one of SEQ ID NOs: 23-24 and SEQ ID NO: 27; and/or
HCDR3 set forth in an amino acid sequence of any one of SEQ ID NO: 36 and SEQ ID NOs: 39-41.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 9, SEQ ID NO: 23 and SEQ ID NO: 36, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 10, SEQ ID NO: 23 and SEQ ID NO: 36, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 11, SEQ ID NO: 24 and SEQ ID NO: 36, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 11, SEQ ID NO: 23 and SEQ ID NO: 36, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 13, SEQ ID NO: 23 and SEQ ID NO: 36, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 10, SEQ ID NO: 23 and SEQ ID NO: 39, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 10, SEQ ID NO: 23 and SEQ ID NO: 40, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 10, SEQ ID NO: 23 and SEQ ID NO: 41, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 10, SEQ ID NO: 27 and SEQ ID NO: 36, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 10, SEQ ID NO: 27 and SEQ ID NO: 39, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 10, SEQ ID NO: 27 and SEQ ID NO: 40, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 10, SEQ ID NO: 27 and SEQ ID NO: 41, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 14, SEQ ID NO: 27 and SEQ ID NO: 36, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 14, SEQ ID NO: 27 and SEQ ID NO: 39, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 14, SEQ ID NO: 27 and SEQ ID NO: 40, respectively.

In some embodiments, the isolated antigen-binding protein specifically binding to PD-L1 and/or a fragment thereof comprises an antibody heavy chain variable region VH, and the VH comprises the HCDR1, the HCDR2 and the HCDR3, whose amino acid sequences are set forth in SEQ ID NO: 14, SEQ ID NO: 27 and SEQ ID NO: 41, respectively.

Preferably, the VH further comprises a heavy chain variable region framework region (VH FWR), and the VH FWR may comprise VH FWR1, VH FWR2, VH FWR3 and VH FWR4.

In some embodiments, the VH FWR1 has an amino acid sequence set forth in any one of SEQ ID NOs: 3-5.

In some embodiments, the VH FWR2 has an amino acid sequence set forth in SEQ ID NO: 17 or SEQ ID NO: 20.

In some embodiments, the VH FWR3 has an amino acid sequence set forth in SEQ ID NO: 30 or SEQ ID NO: 31.

In some embodiments, the VH FWR4 has an amino acid sequence set forth in SEQ ID NO: 43.

Preferably, the VH comprises an amino acid sequence set forth in any one of SEQ ID NOs: 74-77, SEQ ID NOs: 79-80 and SEQ ID NOs: 82-92 or an amino acid sequence which has at least 80%, 85%, 88%, 90%, 92%, 95%, 97%, 98%, 99% or 100% identity thereto.

In some embodiments, the isolated antigen-binding protein specifically binding to PD-L1 and/or a fragment thereof further comprises an Fc region, or a region equivalent to the Fc region of an immunoglobulin, and preferably, the Fc region is human Fc. More preferably, the human Fc is human IgG1 Fc.

Preferably, the Fc comprises mutations L234A, L235A and P329G, or mutations L234Aand L235 A, or comprises one, two or three mutations selected from S298A, E333A and K334A, and more preferably comprises three mutations S298A, E333A and K334A simultaneously.

In some embodiments, the isolated antigen-binding protein specifically binding to PD-L1 or a fragment thereof comprises a heavy chain with an amino acid sequence having at least 80%, 85%, 88%, 90%, 92%, 95%, 97%, 98%, 99% or 100% identity to SEQ ID NO: 99, 100, 101, 102, 104, 105, 107, 108, 109, 110, 111, 112, 113, 114, 115, 116 or 117. In some embodiments, the isolated antigen-binding protein specifically binding to PD-L1 or a fragment thereof comprises an amino acid sequence of the heavy chain set forth in any one of SEQ ID NOs: 99-102, SEQ ID NOs: 104-105 and SEQ ID NOs: 107-117.

In some embodiments, the antigen-binding protein may also be an antigen-binding fragment, and for example, may be in the form of Fab, Fab', F(ab')₂, Fv, scFv, VH, or HCAb. The number of the Fab, the Fab', the F(ab')₂, the Fv, the scFv or the VH is preferably one or more.

In a second aspect, the present invention provides an isolated nucleic acid encoding the isolated antigen-binding protein according to the first aspect of the present invention.

In a third aspect, the present invention provides an expression vector comprising the isolated nucleic acid according to the second aspect of the present invention, and capable of expressing the isolated nucleic acid as the isolated antigen-binding protein according to the first aspect of the present invention.

The expression vector may be an eukaryotic cell expression vector and/or a prokaryotic cell expression vector, such as plasmid.

In a fourth aspect, the present invention provides a host cell comprising the isolated nucleic acid according to the second aspect, or the expression vector according to the third aspect.

In a fifth aspect, the present invention provides an antibody-drug conjugate comprising the isolated antigen-binding protein according to the first aspect of the present invention; and a drug covalently linked to the antigen-binding protein. In some embodiments, the drug is selected from a chemotherapeutic agent, a radiotherapeutic agent, an immunosuppressant and a cytotoxic drug.

In a sixth aspect, the present invention provides a chimeric antigen receptor comprising an extracellular antigen-binding domain, a transmembrane domain, and an intracellular signaling domain, wherein the extracellular antigen-binding domain comprises the isolated antigen-binding protein according to the first aspect of the present invention.

In a seventh aspect, the present application provides a modified immune cell comprising the chimeric antigen receptor according to the sixth aspect of the present invention.

In an eighth aspect, the invention provides a multispecific antibody comprising two or more antigen-binding domains, wherein one antigen-binding domain comprises the isolated antigen-binding protein according to the first aspect of the present invention.

In a ninth aspect, the present application provides a preparation method for the isolated antigen-binding protein according to the first aspect.

In some embodiments, the isolated antigen-binding protein according to the first aspect is prepared by using a hybridoma technique or other conventional techniques in the art, such as humanization techniques.

In some embodiments, the preparation method comprises the step of culturing the host cell according to the fourth aspect.

In some embodiments, the isolated antigen-binding protein according to the first aspect is prepared by using a Harbour HCAb transgenic mouse (hereinafter referred to as HCAb transgenic mouse).

The HCAb transgenic mouse is a transgenic mouse carrying an immune repertoire of human immunoglobulins, capable of producing new "heavy chain"-only antibodies that are only half the size of conventional IgG antibodies. The antibodies produced have only human antibody "heavy chain" variable domains and mouse Fc constant domains.

Preferably, the method for preparing the isolated antigen-binding protein according to the first aspect using the HCAb transgenic mouse comprises the following steps:
(a) the HCAb transgenic mouse is immunized by using a human PD-L1 antigen. More preferably, the human PD-L1 antigen is recombinant human PD-L1-mFc, and particularly, the antigen is a recombinant fusion protein formed by linking human PD-L1 to Fc;
(b) a human VH gene is amplified by cDNA reversely transcribed by RNA of splenic B cells of the immunized HCAb transgenic mouse, and a specific primer;
(c) the amplified VH gene is constructed into a mammalian cell expression vector encoding the sequence of the heavy chain Fc domain of the human IgG antibody; and
(d) the fully human PD-L1 monoclonal antibody acquired in step (c) is purified.

Preferably, the IgG antibody is an IgG1 antibody or an IgG4 antibody.

In some embodiments, the method further comprises the step of performing affinity modification on the fully human PD-L1 monoclonal antibody by mutating the CDR regions.

Preferably, the fully human PD-L1 monoclonal antibody is optimized by germline back mutation and/or post-translational modification (PTM) removal.

In a tenth aspect, the present application provides a pharmaceutical composition comprising the isolated antigen-binding protein according to the first aspect, the antibody-drug conjugate according to the fifth aspect, the chimeric antigen receptor according to the sixth aspect, the modified immune cell according to the seventh aspect or the multispecific antibody according to the eighth aspect, and the pharmaceutically acceptable carrier.

The pharmaceutical composition may also comprise other therapeutic agents including, but not limited to, a chemotherapeutic agent, a radiotherapeutic agent, an immunosuppressant, a cytotoxic drug, and the like. In some embodiments, the chemotherapeutic agent, the radiotherapeutic agent, the immunosuppressant, or the cytotoxic drug in the pharmaceutical composition is the same as or different from the drug in the antibody-drug conjugate according to the fifth aspect.

In an eleventh aspect, the present invention provides use of the isolated antigen-binding protein according to the first aspect, the isolated nucleic acid according to the second aspect, the antibody-drug conjugate according to the fifth aspect, the chimeric antigen receptor according to the sixth aspect, the modified immune cell according to the seventh aspect, the multispecific antibody according to the eighth aspect or the pharmaceutical composition according to the tenth aspect in the preparation of a medicament for preventing, treating and/or diagnosing immune diseases, acute and chronic inflammatory diseases, and tumor diseases.

The tumor may be breast cancer, renal cell carcinoma, melanoma, colon cancer, and B-cell lymphoma, melanoma, head and neck cancer, bladder cancer, stomach cancer, ovarian cancer, malignant sarcoma, urothelial cancer, liver cancer, esophageal cancer, gastroesophageal junction cancer, nasopharyngeal cancer, small cell lung cancer, cervical cancer, endometrial cancer, pancreatic cancer, prostate cancer, glioma, non-small cell lung cancer, acute myeloid leukemia, Hodgkin lymphoma, cutaneous squamous cell carcinoma, locally advanced or metastatic malignancies, and the like.

The inflammatory disease may be atopic dermatitis, ulcerative colitis, and the like.

The immune disease may be graft-versus-host disease, rheumatoid arthritis, systemic lupus erythematosus, asthma, and the like.

In a twelfth aspect, the present invention provides a method for detecting PD-L1 in a sample, and the method comprises the step of detecting the PD-L1 in the sample with the isolated antigen-binding protein according to the first aspect.

The sample may be a biological sample,such as whole blood, red blood cell concentrate, platelet concentrate, white blood cell concentrate, tissue, bone marrow aspirate, plasma, serum, cerebrospinal fluid, feces, urine, cultured cells, saliva, oral secretions, nasal secretions, and the like.

In some embodiments, the method for detecting the PD-L1 in the sample may be for non-diagnosis purposes.

In a thirteenth aspect, the present invention provides a multispecific binding protein comprising at least two domains capable of binding to the PD-L1 or a fragment thereof, and/or CD73 or a fragment thereof.

In some embodiments, the specific binding protein comprises a first domain and a second domain, wherein the first domain binds to CD73 or a fragment thereof, and the second domain binds to PD-L1 or a fragment thereof. The first domain and the second domain are linked to form a bispecific binding protein.

In some embodiments, the first domain is a CD73 antibody or an antigen-binding fragment thereof.

In some embodiments, the second domain is a PD-L1 antibody or an antigen-binding fragment thereof.

In some embodiments, the first domain and/or the second domain is in the form of IgG, Fab, Fab', F(ab')₂, Fv, scFv, VH, or HCAb.

The number of the Fab, Fab', F(ab')₂, Fv, scFv or VH is one or more than one.

In some embodiments, the first domain is in the form of IgG. Preferably, the heavy chain constant region of the IgG is a human heavy chain constant region, more preferably a human IgG1, human IgG2, human IgG3, or human IgG4 heavy chain constant region, wherein the human IgG preferably comprises one, two or three mutations selected from L234A, L235A and P329G, and more preferably comprises mutations L234A and L235A, or comprises one, two or three mutations selected from S298A, E333Aand K334A, and more preferably comprises mutations S298A, E333A and K334A.

Preferably, the Fc of IgG in the first domain is the Fc of the human IgG1. More preferably, the Fc comprises mutations L234A, L235A and P329G, or mutationsL234A and L235A, or comprises one, two or three mutations selected from S298A, E333A and K334A, and more preferably comprises mutations S298A, E333Aand K334A.

In some embodiments, the first domain is in the form of Fab, and more preferably the first domain comprises 2 Fabs.

In some embodiments, the second domain comprises one or more VHs.

Preferably, the VH is a human VH. More preferably, the second domain has two VHs.

In some embodiments, the second domain further comprises the Fc, a region equivalent to the Fc region of immunoglobulin. Preferably the Fc is the Fc of human IgG1, and more preferably, the Fc comprises mutations L234A, L235A and P329G, or mutations L234A and L235A, or comprises one, two or three mutations selected from S298A, E333Aand K334A, and more preferably comprises mutations S298A, E333Aand K334A.

In some embodiments, the first domain is directly linked to the second domain or is linked to the second domain via a linker peptide L to form a bispecific binding protein.

In some embodiments, the second domain is linked to the C-terminus or the N-terminus of the first domain.

In some embodiments, the bispecific binding protein comprises a short chain and a long chain.
Preferably, the short chain has a structure set forth in N'-VL₁-CL₁-C'; the long chain has a structure set forth in N'-VH₁-CH1-h-CH2-CH3-L-VH₂-C';
or the short chain has a structure set forth in N'-VL₁-CL₁-C'; the long chain has a structure set forth in N'-VH₂-L-VH₁-CH1-h-CH2-CH3-C';
or the short chain has a structure set forth in N'-VH₁-CH-C'; and the long chain has a structure set forth in N'-VL₁-CL₁-L-VH₂-CH2-CH3-C',
wherein the VL₁ and the VH₁ are a VL and a VH of the first domain, respectively, the VH₂ is a VH of the second domain, the h is a hinge region, the L is the linker peptide, and the CL₁ is a CL of the first domain. Wherein, the hinge region is a common hinge region in the field of the immunoglobulin, generally containing a large amount of proline and having flexibility, and forming 2-5 disulfide bonds.

Preferably, the L is a peptide having a length of 0-30 amino acids. In some embodiments, the amino acid sequence of the L is set forth in any one of SEQ ID NOs: 136-157.

In some embodiments, in the long chain, CH3 is directly linked to VH₂, i.e., the L is 0 in length.

In some embodiments, in the long chain, the CH3 is linked to the VH₂ via a linker peptide L. In some embodiments, the linker peptide L is a peptide having a length of 0-30 amino acids. In some embodiments, the linker peptide L may be an amino acid sequence set forth in any one of SEQ ID NOs: 136-157.

In some embodiments, in the long chain, the VH₁ is directly linked to VH₂, i.e. the L is 0 in length.

In some embodiments, in the long chain, the VH₁ is linked to the VH₂ via the linker peptide L. In some embodiments, the linker peptide L is a peptide having a length of 0-30 amino acids. In some embodiments, the linker peptide L may be an amino acid sequence set forth in any one of SEQ ID NOs: 136-157.

In some embodiments, in the long chain, the VH₂ is directly linked to CL₁, i.e., the L is 0 in length.

In some embodiments, in the long chain, the VH₂ is linked to CL₁ via the linker peptide L. In some embodiments, the linker peptide L is a peptide having a length of 0-30 amino acids. In some embodiments, the linker peptide L may be an amino acid sequence set forth in any one of SEQ ID NOs: 136-157.

In some embodiments, the first domain comprises a light chain variable region (VL) and a heavy chain variable region (VH), and the VL comprises complementary determining regions selected from:
LCDR1 set forth in an amino acid sequence of any one of SEQ ID NO: 51, SEQ ID NO: 52 and SEQ ID NO: 53;
LCDR2 set forth in an amino acid sequence of any one of SEQ ID NO: 57, SEQ ID NO: 58 and SEQ ID NO: 59; and/or
LCDR3 set forth in an amino acid sequence of any one of SEQ ID NO: 65, SEQ ID NO: 66 and SEQ ID NO: 67; And the VH comprises complementary determining regions selected from:
   HCDR1 set forth in an amino acid sequence of any one of SEQ ID NO: 8, SEQ ID NO: 12 and SEQ ID NO: 10;
   HCDR2 set forth in an amino acid sequence of any one of SEQ ID NO: 22, SEQ ID NO: 25 and SEQ ID NO: 26; and/or
   HCDR3 set forth in an amino acid sequence of any one of SEQ ID NO: 35, SEQ ID NO: 37 and SEQ ID NO: 38.

Preferably, the first domain comprises a light chain variable region (VL) and a heavy chain variable region (VH), and the VL and the VH comprise complementary determining regions selected from:
LCDR1, LCDR2 and LCDR3 set forth in SEQ ID NO: 51, SEQ ID NO: 57 and SEQ ID NO: 65, respectively, and
HCDR1, HCDR2 and HCDR3 set forth in SEQ ID NO: 8, SEQ ID NO: 22 and SEQ ID NO: 35, respectively;
LCDR1, LCDR2 and LCDR3 set forth in SEQ ID NO: 52, SEQ ID NO: 58 and SEQ ID NO: 66, respectively, and
HCDR1, HCDR2 and HCDR3 set forth in SEQ ID NO: 12, SEQ ID NO: 25 and SEQ ID NO: 37, respectively; and
LCDR1, LCDR2 and LCDR3 set forth in SEQ ID NO: 53, SEQ ID NO: 59 and SEQ ID NO: 67, respectively, and
HCDR1, HCDR2 and HCDR3 set forth in SEQ ID NO: 10, SEQ ID NO: 26 and SEQ ID NO: 38, respectively.

In some embodiments, the first domain comprises a VL and a VH, wherein the amino acid sequence of the VL has at least 80%, 85%, 88%, 90%, 92%, 95%, 97%, 98%, 99%, or 100% identity to SEQ ID NO: 94, SEQ ID NO: 95 or SEQ ID NO: 96; and the amino acid sequence of the VH has at least 80%, 85%, 88%, 90%, 92%, 95%, 97%, 98%, 99%, or 100% identity to SEQ ID NO: 73, SEQ ID NO: 78 or SEQ ID NO: 81.

Preferably, the first domain comprises the VL and the VH, wherein the VL has an amino acid sequence set forth in any one of SEQ ID NO: 94, SEQ ID NO: 95 and SEQ ID NO: 96; and the VH has an amino acid sequence set forth in any one of SEQ ID NO: 73, SEQ ID NO: 78 and SEQ ID NO: 81.

Preferably, the first domain comprises the VL and the VH, and amino acid sequences of the VL and the VH are set forth in SEQ ID NO: 94 and SEQ ID NO: 73, respectively, or are set forth in SEQ ID NO: 95 and SEQ ID NO: 78, respectively, or are set forth in SEQ ID NO: 96 and SEQ ID NO: 81, respectively.

In some embodiments, the first domain comprises a light chain with an amino acid sequence having at least 80%, 85%, 88%, 90%, 92%, 95%, 97%, 98%, 99% or 100% identity to SEQ ID NO: 119, SEQ ID NO: 120 or SEQ ID NO: 121, and a heavy chain with an amino acid sequence having at least 80%, 85%, 88%, 90%, 92%, 95%, 97%, 98%, 99% or 100% identity to SEQ ID NO: 98, SEQ ID NO: 103 or SEQ ID NO: 106.

Preferably, the first domain comprises a light chain set forth in any one of SEQ ID NO: 119, SEQ ID NO: 120 and SEQ ID NO: 121; and a heavy chain set forth in any one of SEQ ID NO: 98, SEQ ID NO: 103 and SEQ ID NO: 106.

Preferably, the first domain comprises a light chain set forth in SEQ ID NO: 119 and a heavy chain set forth in SEQ ID NO: 98.

Preferably, the first domain comprises a light chain set forth in SEQ ID NO: 120 and a heavy chain set forth in SEQ ID NO: 103.

Preferably, the first domain comprises a light chain set forth in SEQ ID NO: 121 and a heavy chain set forth in SEQ ID NO: 106.

In some embodiments, the second domain comprises an antibody heavy chain variable region VH, and the VH comprises the complementary determining regions, or mutants thereof:
HCDR1 set forth in an amino acid sequence of SEQ ID NO: 9;
HCDR2 set forth in an amino acid sequence of SEQ ID NO: 23; and/or
HCDR3 set forth in an amino acid sequence of SEQ ID NO: 36.

The mutants are an insertion, deletion or substitution of 1, 2 or 3 amino acids on the amino acid sequences of the HCDR1, the HCDR2 and the HCDR3 of the VH. It should be known to those skilled in the art that insertion, deletion or substitution mutations of 1, 2, or 3 amino acids can be performed on 1, 2, or 3 CDRs in the HCDR1, the HCDR2, and the HCDR3, respectively. For example, a mutation of 1 amino acid may be performed on the HCDR1, but no amino acid mutations on the HCDR2 and the HCDR3; or a mutation of 1 amino acid may be performed on the HCDR1 and the HCDR2, respectively, but no amino acid mutations on the HCDR3.

In some embodiments, the HCDR1 has an amino acid sequence of GFX₁FSX₂Y; the HCDR2 has an amino acid sequence of X₃YX₄GX₅X₆, and the HCDR3 has an amino acid sequence of NRAX₇FGVX₈PDX₉SDI, wherein X₁, X₂, X₃, X₄, X₅, X₆, X₇, X₈, or X₉ is any one selected from N, T, D, S, W, R, K, E, I, A, V and L. In some embodiments, X₁ is T, D or N; X₂ is N or S; X₃ is W or R; X₄ is D or T; X₅ is T or S; X₆ is K, R or E; X₇ is I or L; X₈ is V or I; and/or, X₉ is A or D.

In some embodiments, the amino acid sequence of the HCDR1 is GF(N/T/D)FS(N/S)Y. In some embodiments, the amino acid sequence of the HCDR2 is (W/R)Y(D/T)G(T/S)(K/R/E). In some embodiments, the amino acid sequence of the HCDR3 is NRA(I/L)FGV(V/I)PD(A/D)SDI.

In some embodiments, the mutant of the HCDR1 has an amino acid sequence set forth in any one of SEQ ID NO: 10, SEQ ID NO: 11, SEQ ID NO: 13 and SEQ ID NO: 14.

In some embodiments, the mutant of the HCDR2 has an amino acid sequence set forth in SEQ ID NO: 24 or SEQ ID NO: 27.

In some embodiments, the mutant of the HCDR3 has an amino acid sequence set forth in any one of SEQ ID NO: 39, SEQ ID NO: 40 and SEQ ID NO: 41.

Preferably, the second domain comprises an antibody heavy chain variable region VH, and the VH comprises the complementary determining regions, or mutants thereof:
HCDR1 set forth in an amino acid sequence of any one of SEQ ID NOs: 9-11 and SEQ ID NOs: 13-14;
HCDR2 set forth in an amino acid sequence of any one of SEQ ID NOs: 23-24 and SEQ ID NO: 27; and/or
HCDR3 set forth in the amino acid sequence of any one of SEQ ID NO: 36 and SEQ ID NOs: 39-41.

In some embodiments, the second domain comprises an antibody heavy chain variable region VH, and the VH comprises the HCDR1, the HCDR2 and the HCDR3, whose amino acid sequences are set forth in SEQ ID NO: 9, SEQ ID NO: 23 and SEQ ID NO: 36, respectively.

In some embodiments, the second domain comprises an antibody heavy chain variable region VH, and the VH comprises the HCDR1, the HCDR2 and the HCDR3, whose amino acid sequences are set forth in SEQ ID NO: 10, SEQ ID NO: 23 and SEQ ID NO: 36, respectively.

In some embodiments, the second domain comprises an antibody heavy chain variable region VH, and the VH comprises the HCDR1, the HCDR2 and the HCDR3, whose amino acid sequences are set forth in SEQ ID NO: 11, SEQ ID NO: 24 and SEQ ID NO: 36, respectively.

In some embodiments, the second domain comprises an antibody heavy chain variable region VH, and the VH comprises the HCDR1, the HCDR2 and the HCDR3, whose amino acid sequences are set forth in SEQ ID NO: 11, SEQ ID NO: 23 and SEQ ID NO: 36, respectively.

In some embodiments, the second domain comprises an antibody heavy chain variable region VH, and the VH comprises the HCDR1, the HCDR2 and the HCDR3, whose amino acid sequences are set forth in SEQ ID NO: 13, SEQ ID NO: 23 and SEQ ID NO: 36, respectively.

In some embodiments, the second domain comprises an antibody heavy chain variable region VH, and the VH comprises the HCDR1, the HCDR2 and the HCDR3, whose amino acid sequences are set forth in SEQ ID NO: 10, SEQ ID NO: 23 and SEQ ID NO: 39, respectively.

In some embodiments, the second domain comprises an antibody heavy chain variable region VH, and the VH comprises the HCDR1, the HCDR2 and the HCDR3, whose amino acid sequences are set forth in SEQ ID NO: 10, SEQ ID NO: 23 and SEQ ID NO: 40, respectively.

In some embodiments, the second domain comprises an antibody heavy chain variable region VH, and the VH comprises the HCDR1, the HCDR2 and the HCDR3, whose amino acid sequences are set forth in SEQ ID NO: 10, SEQ ID NO: 23 and SEQ ID NO: 41, respectively.

In some embodiments, the second domain comprises an antibody heavy chain variable region VH, and the VH comprises the HCDR1, the HCDR2 and the HCDR3, whose amino acid sequences are set forth in SEQ ID NO: 10, SEQ ID NO: 27 and SEQ ID NO: 36, respectively.

In some embodiments, the second domain comprises an antibody heavy chain variable region VH, and the VH comprises the HCDR1, the HCDR2 and the HCDR3, whose amino acid sequences are set forth in SEQ ID NO: 10, SEQ ID NO: 27 and SEQ ID NO: 39, respectively.

In some embodiments, the second domain comprises an antibody heavy chain variable region VH, and the VH comprises the HCDR1, the HCDR2 and the HCDR3, whose amino acid sequences are set forth in SEQ ID NO: 10, SEQ ID NO: 27 and SEQ ID NO: 40, respectively.

In some embodiments, the second domain comprises an antibody heavy chain variable region VH, and the VH comprises the HCDR1, the HCDR2 and the HCDR3, whose amino acid sequences are set forth in SEQ ID NO: 10, SEQ ID NO: 27 and SEQ ID NO: 41, respectively.

In some embodiments, the second domain comprises an antibody heavy chain variable region VH, and the VH comprises the HCDR1, the HCDR2 and the HCDR3, whose amino acid sequences are set forth in SEQ ID NO: 14, SEQ ID NO: 27 and SEQ ID NO: 36, respectively.

In some embodiments, the second domain comprises an antibody heavy chain variable region VH, and the VH comprises the HCDR1, the HCDR2 and the HCDR3, whose amino acid sequences are set forth in SEQ ID NO: 14, SEQ ID NO: 27 and SEQ ID NO: 39, respectively.

In some embodiments, the second domain comprises an antibody heavy chain variable region VH, and the VH comprises the HCDR1, the HCDR2 and the HCDR3, whose amino acid sequences are set forth in SEQ ID NO: 14, SEQ ID NO: 27 and SEQ ID NO: 40, respectively.

In some embodiments, the second domain comprises an antibody heavy chain variable region VH, and the VH comprises the HCDR1, the HCDR2 and the HCDR3, whose amino acid sequences are set forth in SEQ ID NO: 14, SEQ ID NO: 27 and SEQ ID NO: 41, respectively.

Preferably, the VH further comprises a heavy chain variable region framework region (VH FWR), and the VH FWR may comprise VH FWR1, VH FWR2, VH FWR3 and VH FWR4.

In some embodiments, the VH FWR1 has an amino acid sequence set forth in any one of SEQ ID NOs: 3-5.

In some embodiments, the VH FWR2 has an amino acid sequence set forth in SEQ ID NO: 17 or SEQ ID NO: 20.

In some embodiments, the VH FWR3 has an amino acid sequence set forth in SEQ ID NO: 30 or SEQ ID NO: 31.

In some embodiments, the VH FWR4 has an amino acid sequence set forth in SEQ ID NO: 43.

Preferably, the VH comprises an amino acid sequence set forth in any one of SEQ ID NOs: 74-77, SEQ ID NOs: 79-80 and SEQ ID NOs: 82-92 or an amino acid sequence which has at least 80%, 85%, 88%, 90%, 92%, 95%, 97%, 98%, 99% or 100% identity thereto.

In some embodiments, the second domain further comprises an Fc region, or a region equivalent to the Fc region of the immunoglobulin, and preferably, the Fc region is human Fc. More preferably, the human Fc is human IgG1 Fc.

Preferably, the Fc comprises mutationsL234A, L235A and P329G, or mutationsL234Aand L235A, or comprises one, two or three mutations selected fromS298A, E333Aand K334A, and more preferably comprises mutationsS298A, E333Aand K334A.

In some embodiments, the second domain comprises a heavy chain comprising an amino acid sequence having at least 80%, 85%, 88%, 90%, 92%, 95%, 97%, 98%, 99% or 100% identity to SEQ ID NO: 99, 100, 101, 102, 104, 105, 107, 108, 109, 110, 111, 112, 113, 114, 115, 116 or 117. In some embodiments, the second domain comprises an amino acid sequence of a heavy chain set forth in any one of SEQ ID NOs: 99-102, SEQ ID NOs: 104-105 and SEQ ID NOs: 107-117.

In some embodiments, the long chain of the specific binding protein comprises an amino acid sequence having at least 80%, 85%, 88%, 90%, 92%, 95%, 97%, 98%, 99%, or 100% identity to SEQ ID NO: 122, 123, 124, 125, 126, 127, 128, 129, 130, 132, or 133; and the short chain of the specific binding protein comprises an amino acid sequence having at least 80%, 85%, 88%, 90%, 92%, 95%, 97%, 98%, 99% or 100% identity to SEQ ID NO: 119, 120, 121 or 131. In some embodiments, the long chain of the specific binding protein comprises an amino acid sequence set forth in any one of SEQ ID NOs: 122-130 and SEQ ID NOs: 132-133; and the short chain of the specific binding protein comprises an amino acid sequence set forth in any one of SEQ ID NOs: 119-121 and SEQ ID NO: 131.

In some embodiments, the specific binding protein has a long chain set forth in SEQ ID NO: 122 and a short chain set forth in SEQ ID NO: 119.

In some embodiments, the specific binding protein has a long chain set forth in SEQ ID NO: 123 and a short chain set forth in SEQ ID NO: 120.

In some embodiments, the specific binding protein has a long chain set forth in SEQ ID NO: 124 and a short chain set forth in SEQ ID NO: 121.

In some embodiments, the specific binding protein has a long chain set forth in SEQ ID NO: 125 and a short chain set forth in SEQ ID NO: 119.

In some embodiments, the specific binding protein has a long chain set forth in SEQ ID NO: 126 and a short chain set forth in SEQ ID NO: 119.

In some embodiments, the specific binding protein has a long chain set forth in SEQ ID NO: 127 and a short chain set forth in SEQ ID NO: 119.

In some embodiments, the specific binding protein has a long chain set forth in SEQ ID NO: 128 and a short chain set forth in SEQ ID NO: 119.

In some embodiments, the specific binding protein has a long chain set forth in SEQ ID NO: 129 and a short chain set forth in SEQ ID NO: 119.

In some embodiments, the specific binding protein has a long chain set forth in SEQ ID NO: 132 and a short chain set forth in SEQ ID NO: 131.

In some embodiments, the specific binding protein has a long chain set forth in SEQ ID NO: 130 and a short chain set forth in SEQ ID NO: 119.

In some embodiments, the specific binding protein has a long chain set forth in SEQ ID NO: 133 and a short chain set forth in SEQ ID NO: 131.

In some embodiments, the bispecific binding protein adopts a tetravalent symmetric structure formed by two first polypeptide chains and two second polypeptide chains.

In a fourteenth aspect, the present invention provides an isolated nucleic acid encoding the specific binding protein or the fragment thereof according to the thirteenth aspect of the present invention.

In a fifteenth aspect, the present invention provides an expression vector capable of expressing the isolated nucleic acid according to the fourteenth aspect as the multispecific binding protein or the fragment thereof according to the thirteenth aspect.

The expression vector may be an eukaryotic cell expression vector and/or a prokaryotic cell expression vector, such as plasmid.

In a sixteenth aspect, the present invention provides a host cell comprising the isolated nucleic acid according to the fourteenth aspect, or the expression vector according to the fifteenth aspect.

The host cell is a conventional host cell in the art, provided that the expression vector according to the fifteenth aspect can stably express the carried nucleic acid as the specific binding protein or a fragment thereof according to the thirteenth aspect. Preferably, the host cell is a prokaryotic cell and/or an eukaryotic cell, wherein the prokaryotic cell is preferably an E. coli cell such as TG1 and BL21 (expressing a single-chain antibody or an Fab antibody), and the eukaryotic cell is preferably an HEK293 cell or a CHO cell (expressing a full-length IgG antibody). The host cell according to the present invention can be obtained by transforming the expression vector according to the fifteenth aspect into the host cell. The transformation method is a conventional transformation method in the art, preferably a chemical transformation method, a heat shock method or an electric transformation method.

In a seventeenth aspect, the present invention provides an antibody-drug conjugate comprising the specific binding protein or the fragment thereof according to the thirteenth aspect. The antibody-drug conjugate further comprises a drug covalently linked to the binding protein or the fragment thereof. In some embodiments, the drug is selected from a chemotherapeutic agent, a radiotherapeutic agent, an immunosuppressant and a cytotoxic drug.
In an eighteenth aspect, the present invention provides a preparation method for the specific binding protein or the fragment thereof according to the thirteenth aspect.

In some embodiments, the specific binding protein or the fragment thereof according to the thirteenth aspect are prepared by using a hybridoma technique or other conventional techniques in the art, such as humanization techniques.

In some embodiments, the preparation method comprises the step of culturing the host cell according to the sixteenth aspect.

In some embodiments, the second domain of the specific binding protein according to the thirteenth aspect is prepared by using a Harbour HCAb transgenic mouse (hereinafter referred to as HCAb transgenic mouse).

The HCAb transgenic mouse is a transgenic mouse carrying an immune repertoire of human immunoglobulins, capable of producing new "heavy chain"-only antibodies that are only half the size of conventional IgG antibodies. The antibodies produced have only "heavy chain" variable domains of human antibody and mouse Fc constant domains.
Preferably, the method for preparing the second domain of the specific binding protein according to the thirteenth aspect using the HCAb transgenic mouse comprises the following steps:
(a) the HCAb transgenic mouse is immunized by using the human PD-L1, more preferably, the human PD-L1 antigen is recombinant human PD-L1-Fc, and particularly, the antigen is a recombinant fusion protein formed by linking the PD-L1 to Fc;
(b) a human VH gene is amplified by cDNA reversely transcribed by RNA of splenic B cells of the immunized HCAb transgenic mouse, and a specific primer;
(c) the amplified VH gene is constructed into a mammalian cell expression vector encoding the sequence of the heavy chain Fc domain of the human IgG antibody; and
(d) the fully human PD-L1 monoclonal antibody acquired in step (c) is purified.

In some embodiments, the method further comprises the step of performing affinity modification on the fully human PD-L1 monoclonal antibody by mutating the CDR regions.
Preferably, the fully human PD-L1 monoclonal antibody may also be optimized by germline back mutation and/or post-translational modification (PTM) removal.

In some embodiments, the first domain of the specific binding protein according to the thirteenth aspect is prepared by using the Harbour H2L2 transgenic mouse (hereinafter referred to as H2L2 transgenic mouse).

The H2L2 transgenic mouse is a transgenic mouse carrying an immune repertoire of human immunoglobulins, and produces antibodies with intact human antibody variable domains and rat constant domains.

Preferably, the method for preparing the first domain of the specific binding protein according to the thirteenth aspect using the H2L2 transgenic mouse comprises the following steps:
(a) the H2L2 transgenic mouse is immunized by using the human CD73, and more preferably, the human CD73 antigen is soluble recombinant human CD73-hFc, and particularly, the antigen is a recombinant fusion protein formed by linking the CD73 to Fc;
(b) spleen cells of the immunized H2L2 transgenic mice are fused with a myeloma cell line to obtain hybridoma cells, and the isolated hybridomas express antibody molecules with heavy and light chains of the intact human variable domains and the rat constant domains;
(c) the sequence of the light chain variable domain (VL) of the antibody acquired in the step (b) is genetically synthesized and cloned into a mammalian cell expression vector encoding the sequence of the κ light chain constant domain of the human antibody to encode a full-length light chain producing the antibody;
(d) the sequence of the heavy chain variable domain (VH) of the antibody acquired in the step (b) is genetically synthesized and cloned into a mammalian cell expression vector encoding the sequence of the heavy chain constant domain of the human IgG antibody to encode a full-length heavy chain producing the IgG antibody; and
(e) the expression vectors obtained in the steps (c) and (d) are simultaneously transfected into a mammalian host cell, and a recombinant antibody with the light chain and the heavy chain correctly assembled in pairs can be obtained by the conventional recombinant protein expression and purification techniques.

Preferably, the sequence of the heavy chain variable domain (VH) of the antibody acquired in the step (b) is genetically synthesized and cloned into a mammalian cell expression vector encoding the sequence of the heavy chain constant domain of the human IgG1 antibody to encode a full-length heavy chain producing the IgG1 antibody.

In some embodiments, the method further comprises the step of performing affinity modification on the fully human CD73 monoclonal antibody by mutating the CDR regions.

Preferably, the fully human CD73 monoclonal antibody may also be optimized by germline back mutation and/or post-translational modification (PTM) removal.

In some embodiments, the prepared second domain and the prepared first domain are combined to form a bispecific binding protein. The bispecific binding protein may bind to two targets simultaneously, with the first domain being capable of recognizing the CD73 specifically expressed on tumor cell surfaces, and the second domain being capable of binding to PD-L1 molecules on T cells, and after being bound to the tumor cell surfaces, the specific binding protein may recruit and activate T cells in the vicinity of tumor cells, thereby killing the tumor cells.

In some embodiments, the prepared second domain and the prepared first domain are constructed into the bispecific binding protein. The bispecific binding protein may bind to two targets simultaneously, with the first domain being capable of recognizing CD73 specifically expressed on tumor cell surfaces and the second domain being capable of binding to PD-L1 molecules expressed in cells. The bispecific binding protein can effectively bind soluble CD73 and PD-L1 on the cell surface, can inhibit the enzyme activity of the CD73, can block the binding of PD-L1 and PD-1, and can activate the T cells.

Preferably, the bispecific binding protein adopts a tetravalent symmetric structure.

Preferably, the bispecific binding protein has the structure and the sequence according to the thirteenth aspect.

In a nineteenth aspect, the present application provides a pharmaceutical composition comprising the specific binding protein according to the thirteenth aspect or the antibody-drug conjugate according to the seventeenth aspect, and a pharmaceutically acceptable carrier.

In some embodiments, the pharmaceutical composition further comprises other ingredients as active ingredients, such as other small molecule drugs, antibodies or polypeptides as active ingredients.

In some embodiments, the pharmaceutical composition further comprises a therapeutic agent selected from a chemotherapeutic agent, a radiotherapeutic agent, an immunosuppressant, and a cytotoxic drug. In some embodiments, the therapeutic agent of the chemotherapeutic agent, the radiotherapeutic agent, the immunosuppressant and the cytotoxic drug of the pharmaceutical composition are different from or the same as the drugs in the antibody-drug conjugate according to the seventeenth aspect.

The pharmaceutically acceptable carrier may be a conventional carrier in the art, and the carrier may be any suitable physiologically or pharmaceutically acceptable auxiliary material. The pharmaceutically acceptable auxiliary material is one conventional in the art, and preferably comprises a pharmaceutically acceptable excipient, a filler, a diluent, or the like. More preferably, the pharmaceutical composition comprises 0.01%-99.99% of the specific binding protein and/or other small molecule drugs or antibodies or polypeptides, and 0.01%-99.99% of a pharmaceutically acceptable carrier, the percentage being the mass percentage of the pharmaceutical composition.

The route of administration of the pharmaceutical composition may be parenteral administration, injection administration or oral administration. The pharmaceutical composition may be made in a form suitable for administration, for example, in the form of a solid, semisolid or liquid, i.e., it may be in the form of an aqueous solution, a non-aqueous solution or a suspension, powder, tablets, capsules, granules, injection or infusion. The pharmaceutical composition may be administrated intravascularly, subcutaneously, intraperitoneally, intramuscularly, by inhalation, intranasally, by airway instillation, or by intrathoracic instillation. The pharmaceutical composition may also be administered in the form of an aerosol or a spray, for example, administered nasally, or administered intrathecally, intramedullarily or intraventricularly, and may also be administrated transdermally, percutaneously, topically, enterally, intravaginally, sublingually or rectally. The pharmaceutical composition may be made into various dosage forms as required, and may be administered by a physician according to the patient's type, age, weight, and general disease state, route of administration, and the like.

In some embodiments, the first domain and the second domain in the specific binding protein may be administered simultaneously or sequentially.

In some embodiments, the specific binding protein and the other active ingredients in the pharmaceutical composition may be administered simultaneously or sequentially.

In some embodiments, the specific binding protein is the bispecific binding protein.

In a twentieth aspect, the invention provides use of the specific binding protein according to the thirteenth aspect, the isolated nucleic acid according to the fourteenth aspect, the antibody-drug conjugate according to the seventeenth aspect or the pharmaceutical composition according to the nineteenth aspect in the preparation of a medicament for preventing, treating and/or diagnosing the immune disease, the acute and chronic inflammatory diseases, and the tumor diseases.

The tumor may be breast cancer, renal cell carcinoma, melanoma, colon cancer, and B-cell lymphoma, melanoma, head and neck cancer, bladder cancer, stomach cancer, ovarian cancer, malignant sarcoma, urothelial cancer, liver cancer, esophageal cancer, gastroesophageal junction cancer, nasopharyngeal cancer, small cell lung cancer, cervical cancer, endometrial cancer, pancreatic cancer, prostate cancer, glioma, non-small cell lung cancer, acute myeloid leukemia, Hodgkin lymphoma, cutaneous squamous cell carcinoma, locally advanced or metastatic malignancies, and the like.

The inflammatory disease may be atopic dermatitis, ulcerative colitis, and the like.

The immune disease may be graft-versus-host disease, rheumatoid arthritis, systemic lupus erythematosus, asthma, and the like.

In a twenty-first aspect, the present invention provides a method for detecting PD-L1 and CD73 in a sample, and the method comprising the step of detecting the PD-L1 and the CD73 in the sample with the specific binding protein according to the thirteenth aspect.

The sample may be a biological sample, such as whole blood, red blood cell concentrate, platelet concentrate, white blood cell concentrate, tissue, bone marrow aspirate, plasma, serum, cerebrospinal fluid, feces, urine, cultured cells, saliva, oral secretions, nasal secretions, and the like.
In a twenty-second aspect, the present invention provides a kit of parts, and the kit of parts comprises one or more kits comprising the antigen-binding protein according to the first aspect, the antibody-drug conjugate according to the fifth aspect or the pharmaceutical composition according to the tenth aspect.

In some embodiments, the kit of parts comprises a first kit and a second kit, wherein the first kit comprises the antigen-binding protein according to the first aspect, the antibody-drug conjugate according to the fifth aspect or the pharmaceutical composition according to the tenth aspect, and the second kit comprises other therapeutic agents including, but not limited to, a chemotherapeutic agent, a radiotherapeutic agent, an immunosuppressant and a cytotoxic drug. In some embodiments, the chemotherapeutic agent, the radiotherapeutic agent, the immunosuppressant, and the cytotoxic drug in the kit of parts are the same as or different from the antibody-drug conjugate according to the fifth aspect.

In some embodiments, the first kit and the second kit may be used simultaneously, or the first kit may be used before the use of the second kit, or the second kit may be used before the use of the first kit. The sequence of use may be determined according to actual requirements in a specific application.

In a twenty-third aspect, the present invention provides a kit of parts, and the kit of parts comprises one or more kits comprising the specific binding protein according to the thirteenth aspect, the antibody-drug conjugate according to the seventeenth aspect or the pharmaceutical composition according to the nineteenth aspect.

In some embodiments, the kit of parts comprises a first kit, and the first kit comprises the bispecific binding protein comprising the first domain and the second domain according to the thirteenth aspect, the antibody-drug conjugate according to the seventeenth aspect or the pharmaceutical composition according to the nineteenth aspect. In some embodiments, the kit of parts may further comprise a second kit comprising other therapeutic agents including, but not limited to, a chemotherapeutic agent, a radiotherapeutic agent, an immunosuppressant, and a cytotoxic drug. In some embodiments, the chemotherapeutic agent, the radiotherapeutic agent, the immunosuppressant and the cytotoxic drug in the kit of parts are the same as or different from the antibody-drug conjugate according to the seventeenth aspect.

In some embodiments, the first kit and the second kit may be used simultaneously, or the first kit may be used before the use of the second kit, or the second kit may be used before the use of the first kit. The sequence of use may be determined according to actual requirements in a specific application.

In a twenty-fourth aspect, the present invention provides an administration device comprising the antigen-binding protein according to the first aspect, the antibody-drug conjugate according to the fifth aspect, or the pharmaceutical composition according to the tenth aspect. In some embodiments, the administration device is a pre-filled syringe.

In a twenty-fifth aspect, the present invention provides the administration device comprising the antigen-binding protein according to the thirteenth aspect, the antibody-drug conjugate according to the seventeenth aspect, or the pharmaceutical composition according to the nineteenth aspect. In some embodiments, the administration device is a pre-filled syringe.

In a twenty-sixth aspect, the present invention provides a method for preventing, treating and/or diagnosing immune diseases, acute and chronic inflammatory diseases and tumor diseases, comprising administering to a subject a therapeutically effective quantity of the antigen-binding protein according to the first aspect, the antibody-drug conjugate according to the fifth aspect, or the pharmaceutical composition according to the tenth aspect.
In a twenty-seventh aspect, the present invention provides a method for preventing, treating and/or diagnosing immune diseases, acute and chronic inflammatory diseases and tumor diseases, comprising administering to the subject a therapeutically effective quantity of the antigen-binding protein according to the thirteenth aspect, the antibody-drug conjugate according to the seventeenth aspect, or the pharmaceutical composition according to the nineteenth aspect.

The bispecific binding protein according to the present invention has one or more of the following properties:
(a) the bispecific binding protein can specifically bind to human PD-L1 and CD73 having high affinity;
(b) the bispecific binding protein can inhibit enzymatic activity of the CD73;
(c) the bispecific binding protein has an inhibitory effect on a PD-1 signaling pathway;
(d) the bispecific binding protein can enhance the activation of T lymphocytes to secrete cytokines IL-2 and IFN-γ; and
(e) the bispecific binding protein shows *in vivo* tumor inhibitory activity.

### Definitions

Unless otherwise defined, technical and scientific terms used in the present invention have the same meaning as generally used in the art to which the present invention belongs. For purposes of interpreting the description, the following definitions will be applied and whenever appropriate, terms used in the singular will also include the plural and vice versa.

The "about" and "approximately" generally refer to an acceptable range of error in the measured value in view of the nature or accuracy of the measurement. Generally, the error range is within 20%, generally within 10%, and even more generally within 5% of a given value or range of values.

The term "antigen-binding molecule" or "specific binding protein" generally refers to a molecule specifically binding to an antigenic determinant. The antigen-binding molecule or the specific binding protein comprises, for example, antibodies, antibody fragments and framework antigen-binding proteins.

The term "antibody" in the present invention encompasses various antibody structures, including but not limited to monoclonal antibodies, polyclonal antibodies, monospecific and multispecific antibodies (e.g., bispecific antibodies or trispecific antibodies), single-chain molecules and antibody fragments so long as they exhibit the desired antigen-binding activity.

The term "monoclonal antibody" in the present invention refers to an antibody acquired from a population of substantially homogeneous antibodies, i.e., except for possible small amounts of mutated antibodies (for example, those containing naturally occurring mutations or arising during production of a monoclonal antibody formulation, which are generally present in minor amounts), the individual antibodies that constitute the antibody population are identical and/or bind to the same epitope. Unlike polyclonal antibody formulations which generally comprise different antibodies directed against different antigenic determinants (epitopes), each monoclonal antibody in the monoclonal antibody formulations is directed against a single determinant on an antigen.

The term "multispecific antibody" in the present invention is used in its widest sense to encompass antibodies having multi-epitope specificity. These multispecific antibodies include, but are not limited to: an antibody comprising a heavy chain variable region (VH) and a light chain variable region (VL), the VH-VL unit having multi-epitope specificity; an antibody having two or more VL and VH regions, each VH-VL unit binding to a different target or a different epitope of the same target; an antibody having two or more single variable regions, each single variable region binding to a different target or a different epitope of the same target; full length antibodies, antibody fragments, bispecific antibodies (diabodies), triabodies, antibody fragments linked together covalently or non-covalently, and the like.

The term "bispecific binding protein" or "bispecific antibody" in the present invention refers to protein or an antibody that can specifically bind to at least two different antigenic determinants. For example, two binding sites each formed by a pair of an antibody heavy chain variable domain (VH) and an antibody light chain variable domain (VL) bind to different epitopes on different antigens or on the same antigen. The bispecific antibody may be in a 1+1 form, a 2+1 form (comprising two binding sites for the first antigen or epitope and one binding site for the second antigen or epitope) or a 2+2 form (comprising two binding sites for the first antigen or epitope and two binding sites for the second antigen or epitope). Generally, the bispecific antibody comprises two antigen-binding sites, each of which is specific for different antigenic determinants.

The term "valent" in the present invention denotes the presence of a specified number of binding domains in the antigen-binding molecule. As such, the terms "bivalent", "tetravalent", and "hexavalent" denote the presence of two binding domains, four binding domains, and six binding domains, respectively, in the antigen-binding molecule. The bispecific antibodies are at least "bivalent", and may be "trivalent", "tetravalent" or "multivalent"). In some cases, the antibodies have two or more binding sites and are bispecific. That is, the antibodies may be bispecific even in cases where there are more than two binding sites (i.e. the antibodies are trivalent or multivalent).

The terms "full-length antibody" and "intact antibody" in the present invention are used herein interchangeably to refer to an antibody having a structure substantially similar to a native antibody structure. "Native antibodies" refer to naturally occurring immunoglobulin molecules. For example, native IgG-class antibodies are heterotetrameric glycoproteins of about 150,000 daltons, consisting of two light chains and two heavy chains, which are disulfide-bonded. From the N-terminus to the C-terminus, each heavy chain has a variable region (VH) (also called a variable heavy chain domain or a heavy chain variable domain), and three constant domains (CH1, CH2, and CH3) (also called a heavy chain constant region). From the N-terminus to the C-terminus, each light chain has a variable region (VL) (also called a variable light chain domain or a light chain variable domain) and a light chain constant domain (CL) (also called a light chain constant region). The heavy chain of the antibody may be one of five types: α (IgA), δ (IgD), ε (IgE), γ (IgG), or µ (IgM), which may be further divided into subtypes, e.g. γ1 (IgG1), γ2 (IgG2), γ3 (IgG3), γ4 (IgG4), α1 (IgA1) and α2 (IgA2). The light chain of the antibody may be one of two types: x light chain and λ light chain, based on the amino acid sequence of its constant domain.

In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of classical complement system.

An "antibody fragment" comprises a portion of the intact antibody. Examples of antibody fragments include, but are not limited to, Fab, Fab', F(ab')₂ and Fv; diabodies, triabodies, tetrabodies, cross-Fab fragments; linear antibodies; single-chain antibody molecules (e.g. scFv); and multispecific antibodies formed from antibody fragments and single-domain antibodies.

The term "antigen-binding domain" or "antigen-binding site" in the present invention refers to the part of the antigen-binding molecule that specifically binds to an antigenic determinant. More particularly, the term "antigen-binding domain" refers to the part of the antibody, which comprises the region which specifically binds to and is complementary to part or all of an antigen. Where an antigen molecule is large, the antigen-binding molecule may only bind to a particular part of the antigen, which is called an epitope. An antigen-binding domain may be provided by, for example, one or more variable domains (also called variable regions). Preferably, an antigen-binding domain comprises an antibody light chain variable region (VL) and an antibody heavy chain variable region (VH). In one aspect, the antigen-binding domain may bind to its antigen and block or partly block functions of the antigen.

The term "antigenic determinant" in the present invention is synonymous with "antigen" and "epitope", and refers to a site (e.g. a contiguous stretch of amino acids or a conformational configuration made up of different regions of non-contiguous amino acids) on a polypeptide macromolecule, to which an antigen-binding moiety binds, thereby forming an antigen-binding moiety-antigen complex. Antigenic determinants may be, for example, on the surfaces of tumor cells, on the surfaces of microorganism-infected cells, on the surfaces of other diseased cells, on the surfaces of immune cells, free substances in serum, and/or in extracellular matrixes (ECM). The proteins useful as antigens in the present invention may be any native form of the proteins from any vertebrate source including mammals such as primates (e.g. humans) and rodents (e.g. mice and rats), unless otherwise indicated. The antigen may also be a human protein, or the antigen may be a "full-length", unprocessed protein, as well as any form of proteins formed from intracellular processing, or a naturally occurring protein variant, such as a splice variant or an allelic variant.

"Specific binding" refers to having selective binding for antigens, and can be distinguished from undesired or non-specific binding regions. The ability of an antigen-binding molecule to bind to a particular antigen can be determined by an enzyme-linked immunosorbent assay (ELISA) or other techniques (such as surface plasmon resonance (SPR) technique and a conventional binding assay) known to those skilled in the art. In one embodiment, the antigen-binding molecule binds to an unrelated protein to an extent less than about 10% of the binding of the antigen-binding molecule to an antigen, as measured, for example, by SPR. In certain embodiments, the dissociation constant (Kd) of the molecule binding to the antigen is ≤ 1 µM, ≤ 100 nM, ≤ 10 nM, ≤ 1 nM, ≤ 0.1 nM, ≤ 0.01 nM or ≤ 0.001 nM (e.g., 10⁻⁷ M or less, such as 10⁻⁷ M to 10⁻¹³ M, for example, 10⁻⁹ M to 10⁻¹³ M).

"Affinity" or "binding affinity" refers to the strength of a non-covalent interaction between a single binding site of a molecule (such as an antibody) and its binding partner (such as an antigen). The binding affinity may generally be represented by a dissociation constant (Kd), which is the ratio of a dissociation rate constant (k_{off}) to an association rate constant (kₒₙ). Therefore, equivalent affinities may include different rate constants, as long as the ratio of the rate constants remains the same. The affinity can be determined by conventional methods known in the art, such as surface plasmon resonance (SPR).

The term "high affinity" in the present invention means that the Kd of an antibody to a target antigen is 10⁻⁹ M or less or even 10⁻¹⁰ M or less. The term "low affinity" in the present invention means that the Kd of an antibody is 10⁻⁸ M or more.

An "affinity-matured" antibody refers to an antibody having one or more modifications in one or more hypervariable regions (HVRs), such modifications resulting in an improvement of the affinity of an antibody to an antigen compared to a parent antibody that does not have such modifications.

The terms "single-domain antibody" and "nanobody" of the present invention have the same meaning and refer to a single-domain antibody consisting of only one heavy chain variable region constructed by a variable region having only an antibody heavy chain, which is the smallest antigen-binding fragment having the full function.
The term "heavy-chain antibody" of the present invention, also known as HCAb antibody, refers to an antibody lacking an antibody light chain and comprising only a heavy chain, specifically comprising a heavy chain variable domain and an Fc constant domain, relative to a diabody (immunoglobulin).

The specific binding protein comprises a first domain and a second domain, wherein the first domain binds to CD73 or a fragment thereof, and the second domain binds to PD-L1 or a fragment thereof

The term "a bispecific antibody comprising a first domain specifically binding to CD73 and a second domain specifically binding to PD-L1" "a bispecific antibody specifically binding to CD73 and PD-L1", "a bispecific antigen-binding molecule specific for CD73 and PD-L1" or "an anti-CD73/anti-PD-L1 antibody" are used interchangeably herein and refer to a bispecific antibody that is capable of binding to CD73 and PD-L1 with sufficient affinity, so that the antibody may be used as a diagnostic and/or therapeutic agent targeting CD73 and PD-L1.

The term "T effector cell" in the present invention refers to T cells having cytolytic activity (for example, CD4+ cells and CD8+ T cells) and T helper (Th) cells. The T effector cells secrete cytokines and activate and guide other immune cells excluding regulatory T cells (Treg cells).

The term "regulatory T cell" or "Treg cell" in the present invention refers to a particular type of CD4+ T cells that block the response of other T cells. The Treg cells are characterized by expres sing CD4, an α subunit of an IL-2 receptor (CD25), and a transcription factor FOXP3, and play a vital role in the induction and maintenance of peripheral autologous tolerance directed against antigens expressed by tumors.

The term "CD73" in the present invention, i.e., the cluster of differentiation 73, also known as 5'-ectonucleotidase, generally refers to an enzyme (nucleotidase) capable of converting extracellular nucleoside 5' monophosphate to a nucleoside, i.e., converting adenosine monophosphate (AMP) to adenosine. CD73 is a cell surface enzyme linked by glycosylphosphatidylinositol (GPI), and CD73 is widely expressed on the surfaces of human endothelial cells and lymphocytes, such as Treg cells, DC cells, MDSC cells, NK cells, and the like. CD73 may also be expressed on cancer cells, including colon cancer, lung cancer, pancreatic cancer, ovarian cancer, bladder cancer, leukemia, glioma, glioblastoma, melanoma, thyroid cancer, esophageal cancer, prostate cancer, breast cancer, and the like. High CD73 expression has been reported to relate to poor prognosis for a variety of cancer indications (e.g., lung cancer, melanoma, breast cancer, squamous head and neck cancer, and colorectal cancer). The primary function of CD73 is to convert extracellular nucleotides (e.g., 5'-AMP) into a highly immunosuppressive molecule, namely adenosine. The term "CD73" in the present invention includes any variant or isoform of CD73 that is naturally expressed by a cell. CD73 or any variants and isoforms thereof may be isolated from cells or tissues in which they are naturally expressed, or may be recombinantly produced using techniques well known in the art and/or those described herein. Accordingly, the antigen-binding protein of the present invention may cross-react with species other than human (e.g., cynomolgus monkey CD73). Or the antigen-binding protein, antibody or domain targeting CD73 in the present invention may be specific for human CD73 and may not exhibit any cross-reactivity with other species.

The term "CD73 antibody" or "isolated antigen-binding protein specifically binding to CD73 and/or a fragment thereof' may bind to CD73, and has sufficient affinity, so that the antibody may be used as a diagnostic and/or therapeutic agent targeting CD73. Generally, the binding ability of the CD73 antibody to unrelated CD73 proteins is less than about 10% of the binding ability of the antibody to CD73, as determined by radioimmunoassay (RIA), or flow cytometry (FACS), or surface plasmon resonance using a biosensor system. In certain embodiments, the dissociation constant (KD) of the antibody binding to CD73 is ≤ 1 µM, ≤ 100 nM, ≤ 10 nM, ≤ 1 nM, ≤ 0.1 nM, ≤ 0.01 nM or ≤ 0.001 nM (e.g., 10⁻⁸ M or less, such as 10⁻¹³ M to 10⁻⁸ M, e.g., 10⁻¹³ M to 10⁻⁹ M).

The term programmed cell death 1 ligand 1 (PD-L1) in the present invention, also known as the cluster of differentiation CD274 or B7 homologue 1 (B7-H1), is a member of the B7 family that modulates activation or inhibition of the PD-1 receptor. The open reading frame of PD-L1 encodes type I transmembrane proteins of 290 amino acids, which comprises extracellular Ig domains (an N-terminal V-like domain and a IgC-like domain), a hydrophobic transmembrane domain and a cytoplasmic tail region of 30 amino acids. The intracellular (cytoplasmic) domain of 30 amino acids contains no obvious signaling, but does have a potential site for protein kinase C phosphorylation. Studies have shown that the expression of PD-L1 on immune cells is enhanced under the stimulation of interferon γ. PD-L1 is also expressed on non-immune cells including islets of the pancreas, Kupffer cells of the liver, vascular endothelium and selected epithelia, for example airway epithelia and renal tubule epithelia, where its expression is enhanced during inflammatory episodes. PD-L1 expression is also found at increased levels on a number of tumors including, but not limited to breast cancer, ovarian cancer, cervical cancer, colon cancer, colorectal cancer, lung cancer including non-small cell lung cancer, kidney cancer including renal cell carcinoma, stomach cancer, esophageal cancer, bladder cancer, hepatocellular cancer, squamous cell carcinoma of the head and neck (SCCHN) and pancreatic cancer, melanoma and uveal melanoma.

The term "PD-L1 antibody" or "isolated antigen-binding protein specifically binding to PD-L1 and/or a fragment thereof' may bind to PD-L1 especially PD-L1 polypeptides expressed on cell surfaces, and has sufficient affinity, so that the antibody may be used as a diagnostic and/or therapeutic agent targeting PD-L1. Generally, the binding ability of the PD-L1 antibody to unrelated non-PD-L1 proteins is less than about 10% of the binding ability of the antibody to PD-L1, as determined by radioimmunoassay (RIA), or flow cytometry (FACS), or surface plasmon resonance using a biosensor system. In certain embodiments, the KD value of the antigen-binding protein binding to human PD-L1 is ≤ 1 µM, ≤ 100 nM, ≤ 10 nM, ≤ 1 nM, ≤ 0.1 nM, ≤ 0.01 nM or ≤ 0.001 nM (e.g., 10⁻⁸ M or less, e.g., 10⁻¹³ M to 10⁻⁸ M, e.g., 10⁻¹³ M to 10⁻⁹ M). In some embodiments, the respective KD value of the binding affinity is determined in the surface plasmon resonance determination using human PD-L1 especially human PD-L1 with histidine tags so as to acquire the binding affinity for PD-L1. The therapeutic strategy of the PD-1/PD-L1 antibody is a standard therapeutic strategy for several metastatic tumors, and has shown their role in early disease stages and adjuvant therapy, especially for melanoma and non-small cell lung cancer.

The term "H2L2 transgenic mouse" or "Harbour H2L2 mouse" in the present application is a transgenic mouse carrying an immune repertoire of human immunoglobulins, and the mouse produces traditional tetramer antibodies consisting of two heavy chains and two light chains (H2L2) with a full human variable region, with a complete human antibody variable domain and a rat constant domain. The antibodies produced by the transgenic mouse are matured in affinity, and fully humanized in the variable regions, and have excellent solubility.

The term "Harbour HCAb mouse" (WO2002/085945A2) in the present application is a transgenic mouse carrying an immune repertoire of human immunoglobulins, capable of producing new "heavy chain"-only antibodies that are only half the size of conventional IgG antibodies. The antibodies produced have only human antibody "heavy chain" variable domains and mouse Fc constant domains. Due to the absence of light chain, the antibody almost solves the problems of light chain mismatch and heterodimerization, allowing the technical platform to develop products that are difficult to realize by the conventional antibody platform.

The term "variable region" or "variable domain" in the present invention refers to the domain of an antibody heavy chain or an antibody light chain that is involved in binding the antigen-binding molecule to the antigen. The variable domains of the heavy chain and the light chain (VH and VL, respectively) of a native antibody generally have similar structures, with each domain comprising four conserved framework regions (FRs) and three hypervariable regions (HVRs). A single VH or VL domain may be sufficient to confer antigen-binding specificity.
The term "variable" in the present invention means that certain segments of the variable domains differ generally in the sequence among antibodies. The V domain mediates antigen binding and defines the specificity of a particular antibody for its particular antigen. However, variability is not evenly distributed throughout the entire variable domain. Instead, the variability is concentrated in three segments called hypervariable regions (HVRs) both in the light chain variable domain and the heavy chain variable domain. The more highly conserved portions of the variable domains are called framework regions (FR). The variable domains of native heavy and light chains each comprise four FR regions largely in a β-sheet configuration. Every four FRs are connected by three HVRs to form a loop connection, and in some cases to form part of a β-sheet structure. The HVRs in each chain are tightly held together through the FR regions and, together with the HVRs from the other chain, contribute to the formation of the antigen-binding site of antibodies (see Kabat et al., Sequences of Immunological Interest, 5th Ed., National Institute of Health, Bethesda, MD. (1991)). The constant domains do not participate directly in the binding of the antibody to the antigen, but have other effector functions, such as participation of the antibody in antibody-dependent cellular toxicity.

The term "hypervariable region" or "HVR" in the present invention refers to the region where the sequence is hypervariable and/or a structurally defined loop ("hypervariable loop") is formed in the antibody variable domain region. Generally, native four-chain antibodies comprise six HVRs; three in the VH (H1, H2, and H3), and three in the VL (L1, L2, and L3). HVRs generally comprise amino acid residues from the hypervariable loops and/or from the "complementary determining regions" (CDRs), and the amino acid residues from the "complementary determining regions" (CDRs) are of highest sequence variability and/or participate in antigen recognition. Exemplary CDRs (CDR-L1, CDR-L2, CDR-L3, CDR-H1, CDR-H2 and CDR-H3) occur at the amino acid residues 26-32 (L1), 50-52 (L2), 91-96 (L3), 26-32 (H1), 53-55 (H2) and 96-101 (H3) (Chothia et al, J. Mol. Biol. 196:901-917 (1987)). Exemplary CDRs (CDR-L1, CDR-L2, CDR-L3, CDR-H1, CDR-H2 and CDR-H3) occur at the amino acid residues 24-34 (L1), amino acid residues 50-56 (L2), amino acid residues 89-97 (L3), amino acid residues 31-35 (H1), amino acid residues 50-65 (H2) and amino acid residues 95-102 (H3) (Kabat et al, Sequences of Proteins of Immunological Interest, 5th Ed., Public Health Service, National Institutes of Health, Bethesda, MD (1991)). As a comparison, the corresponding amino acid residues which contain the CDRs defined in the references cited above are listed in Table 1 below. In the present application, the amino acid sequences of the listed CDRs are all shown according to the Chothia scheme (the sequences in the claims of the present application are also shown according to the Chothia scheme). However, it is well known to those skilled in the art that the CDRs of an antibody can be defined in the art using a variety of methods, such as the Kabat scheme based on sequence variability, and the Chothia scheme based on the location of the structural loop regions (see J Mol Biol 273: 927-948, 1997). In the present application, the Combined scheme comprising the Kabat scheme and the Chothia scheme can also be used to determine the amino acid residues in a variable domain sequence. The Combined scheme combines the Kabat scheme with the Chothia scheme to obtain a larger range. It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

**Table 1. Positions of CDR schemes of the present invention in antibody light or heavy chains based on different numbering**

| CDR\Numbering scheme | Kabat | Chothia | Combined |
|---|---|---|---|
| LCDR1 | 24-34 | 26-32 | 24-34 |
| LCDR2 | 50-56 | 50-52 | 50-56 |
| LCDR3 | 89-97 | 91-96 | 89-97 |
| HCDR1 | 31-35 | 26-32 | 26-35 |
| HCDR2 | 50-65 | 52-58 | 50-65 |
| HCR3 | 95-102 | 95-102 | 95-102 |

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in VH (or VL): FR1-H1(L1)-FR2-H2(L2)-FR3-H3(L3)-FR4.

The "class" of the antibody refers to the type of the constant domain or constant region possessed by the heavy chain of the antibody. Five classes of antibodies exist: IgA, IgD, IgE, IgG, and IgM, and several of these classes may be further divided into subclasses (isotypes), e.g. IgG1, IgG2, IgG3, IgG4, IgA1, and IgA2. The heavy chain constant domains corresponding to different classes of immunoglobulins are called α, δ, ε, γ and µ respectively.

A "humanized antibody" comprises amino acid residues from non-human HVRs and amino acid residues from human FRs. In certain embodiments, the humanized antibody comprises at least one, or generally two variable domains in which all or substantially all HVRs (e.g., CDRs) correspond to HVRs of a non-human antibody, and all or substantially all FRs correspond to FRs of a human antibody. The humanized antibody may optionally comprise at least a portion of an antibody constant region derived from a human antibody. An antibody in the "humanized form", such as a non-human antibody, refers to an antibody that has been humanized.

A "human antibody" has amino acid sequences that correspond to the amino acid sequences generated by a human or a human cell or derived from a non-human source antibody that utilizes human antibody libraries or other human antibody coding sequences. This definition of a human antibody particularly excludes humanized antibodies comprising non-human antigen-binding residues.

The term "Fc domain" or "Fc region" of the present invention is used to define the C-terminus region of an antibody heavy chain comprising at least a portion of the constant region. The "Fc region" includes Fc regions of native sequences and variant Fc regions. An IgG Fc region comprises an IgG CH2 domain and an IgG CH3 domain. The CH2 domain herein may be a CH2 domain of a native sequence or a variant CH2 domain. The CH3 domain herein may be a CH3 domain of a native sequence or a variant CH3 domain. The CH2 domain may comprise one or more mutations that reduce or eliminate the binding of the CH2 domain to one or more Fcy receptors (for example, FcyRI, FcyRIIa, FcyRIIb and FcyRIII) and/or complements. It is speculated that reducing or eliminating the binding to Fc receptor γ will reduce or eliminate antibody molecule-mediated ADCC. Similarly, it is speculated that reducing or eliminating the binding to complements will reduce or eliminate antibody molecule-mediated CDC. The mutations that reduce or eliminate the binding of the CH2 domain to one or more Fcy domain receptors and/or complements are known in the art (Wang et al., 2018). These mutations include the "LALA mutation", which involves the replacement of the leucine residues at IMGT positions 1.3 and 1.2 of the CH2 domain with alanine (L1.3A and L1.2A). Alternatively, it is also known that a conserved N-linked glycosylation site is mutated to produce α-glycosyl antibodies by mutating the asparagine (N) at IMGT position 84.4 of the CH2 domain to alanine, glycine or glutamine (N84.4A, N84.4G or N84.4Q), thereby reducing the function of IgG1 effectors (Wang et al., 2018). Alternatively, it is known that complement activation (C1q binding) and ADCC can be reduced by mutating the proline at IMGT position 114 of the CH2 domain to alanine or glycine (P114A or P114G) (Idusogie et al., 2000; Klein et al., 2016). These mutations can be combined to produce antibody molecules with further reduced or no ADCC or CDC activity.

A "region equivalent to the Fc region of immunoglobulin" comprises naturally occurring allelic variants of the Fc region of the immunoglobulin, as well as modified variants which have the ability of producing substitutions, additions or deletions but do not decrease substantially the effector functions (such as antibody-dependent cellular toxicity) mediated by the immunoglobulin. For example, one or more amino acids can be deleted from the N-terminus or the C-terminus of the Fc region of the immunoglobulin, without substantial loss of biological functions. Such variants can be selected according to general rules known in the art so as to have minimal effects on activity (see, e.g., Bowie, J. U. et al., Science 247:1306-10 (1990)).

The term "effector function" in the present invention can be attributed to the biological activities of the Fc region of the antibody, which change with the changes of antibody isotypes. Examples of antibody effector functions comprise: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptors), B cell activation, and the like.

The term "peptide linker" or "linker peptide" in the present invention refers to a peptide comprising one or more amino acids, generally about 2 to 20 amino acids. The peptide linker is known in the art or described herein.

The term "fused to", "fusion-linked" or "linked to" in the present invention means that segments (for example, the antigen-binding domain and the FC domain) are linked to peptide bonds directly or linked to the peptide bonds via one or more peptide linkers.

The present invention also relates to amino acid sequence variants of the bispecific antibodies of the present invention. The amino acid sequence variants of the bispecific antibodies may be prepared by introducing appropriate modifications into the nucleotide sequence encoding the molecules, or by peptide synthesis. Such modifications include, for example, deletion, insertion and/or substitution of residues in the amino acid sequences of the antibodies. Any combination of deletion, insertion, and substitution can be made to acquire the final construct. The final construct possesses the desired characteristics, e.g., antigen-binding activity. Sites for substitution generally comprise HVRs and frameworks (FRs). See Table 2 below for possible substitutions of amino acids.

**Table 2. Conserved amino acid substitutions**

| Amino acid residue | Conserved substitution | Preferred conserved substitution |
|---|---|---|
| Ala(A) | Val; Leu; Ile | Val |
| Arg(R) | Lys; Gln; Asn | Lys |
| Asn(N) | Gln; His; Asp; Lys; Arg | Gln |
| Asp(D) | Glu; Asn | Glu |
| Cys(C) | Ser; Ala | Ser |
| Gln(Q) | Asn; Glu | Asn |
| Glu(E) | Asp; Gln | Asp |
| Gly(G) | Ala | Ala |
| His(H) | Asn; Gln; Lys; Arg | Arg |
| Ile(I) | Leu; Val; Met; Ala; Phe; Nle | Leu |
| Leu(L) | Nle; Ile; Ual; Met; Ala; Phe | Ile |
| Lys(K) | Arg; Gln; Asn | Arg |
| Met(M) | Leu; Phe; Ile | Leu |
| Phe(F) | Trp; Leu; Val; Ile; Ala; Tyr | Tyr |
| Pro(P) | Ala | Ala |
| Ser(S) | Thr | Thr |
| Thr(T) | Val; Ser | Ser |
| Trp(W) | Tyr; Phe | Tyr |
| Tyr(Y) | Trp; Phe; Thr; Ser | Phe |
| Val(V) | Ile; Leu; Met; Phe; Ala; Nle | Leu |

The term "polynucleotide" or "nucleic acid" or "nucleotide sequence" of the present invention refers to an isolated nucleic acid molecule or a construct, such as messenger RNA (mRNA), virus-derived RNA or plasmid DNA (pDNA). The polynucleotide may comprise conventional phosphodiester bonds or unconventional bonds (e.g., amide bonds, such as those found in peptide nucleic acids (PNAs)). The term "nucleic acid molecule" refers to any one or more of nucleic acid segments, e.g., DNA or RNA fragments, present in a polynucleotide.

The term "isolated" nucleic acid molecule or polynucleotide of the present invention refers to a nucleic acid molecule, DNA or RNA, which has been separated from its natural environment. In the present invention, a recombinant polynucleotide encoding polypeptides contained in the vector is also isolated. Other examples of the isolated polynucleotide include recombinant polynucleotides in heterologous host cells or polynucleotides purified in solutions. The isolated polynucleotide includes a polynucleotide molecule contained in a cell that generally comprises the polynucleotide molecule, but the polynucleotide molecule exists extrachromosomally or at a chromosomal location that is different from its natural chromosomal location. The isolated RNA molecule includes *in vivo* or *in vitro* RNA transcripts of the present invention, which are in the forward or reverse strand form, or in the double-stranded form. The isolated polynucleotide or nucleic acid of the present invention further include such molecules generated synthetically. In addition, the polynucleotide or nucleic acid may be or may include regulatory elements such as promoters, ribosome binding sites or transcription terminators.

The term "expression cassette" in the present invention refers to a polynucleotide generated recombinantly or synthetically, with a series of nucleic acid elements that permit transcription of a particular nucleic acid in a target cell. The recombinant expression cassette can be introduced into a plasmid, chromosome, mitochondrial DNA, plastid DNA, virus, or nucleic acid fragments. Typically, in addition to other sequences, the recombinant expression cassette portion of the expression vector comprises a nucleic acid sequence to be transcribed and a promoter. In certain embodiments, the expression cassette of the present invention comprises polynucleotide sequences that encode the bispecific antigen-binding molecule or a fragment thereof of the present invention.

The terms "vector" or "expression vector" and "expression construct" of the present invention are used interchangeably and refer to a DNA molecule for introducing a particular gene operably linked thereto into a target cell and directing the expression. The vector includes vectors that serve as self-replicating nucleic acid structures as well as vectors incorporating into the genome of a host cell into which they have been introduced. The expression vector of the present invention comprises an expression cassette. The expression vector can perform a large number of stable transcriptions of mRNA. Once the expression vector is within the target cell, a ribonucleic acid molecule or protein encoded by the gene is generated by cellular transcription and/or translation mechanism. In one embodiment, the expression vector of the present invention includes an expression cassette comprising a polynucleotide sequence encoding the bispecific antigen-binding molecule or the fragment thereof of the present invention.

The terms "host cell", "host cell line" and "host cell culture" are used interchangeably and refer to cells into which exogenous nucleic acids have been introduced, including progenies of such cells. Host cells include "transformant/transformants" and "transformed cells," including primary transformed cells and progenies derived therefrom. The nucleic acids of progenies may not be exactly identical to those of parent cells, and may contain mutations. The host cells are any type of cells that can be used to generate the bispecific antigen-binding molecules of the present invention. The host cells include cultured cells, for example, cultured mammalian cells such as CHO cells, HEK293 cells, BHK cells, NS0 cells, SP2/0 cells, YO myeloma cells, P3X63 mouse myeloma cells, PER cells, PER.C6 cells or hybridoma cells, yeast cells, insect cells and plant cells, and further include cells contained within transgenic animals, transgenic plants or cultured plant or animal tissues.

Germline back mutation refers to the process of back mutating hyper-somatic mutations of antibodies to corresponding germline sequences. The sequence of the heavy chain variable domain of the antibody was derived from events such as gene rearrangements of germline gene V, D and J segments of heavy chain gene clusters and somatic hypermutations on chromosomes; the sequences of the light chain variable domain were derived from the events such as gene rearrangements of germline gene V, D and J segments of light chain gene clusters and somatic hypermutations. Gene rearrangement and somatic hypermutation are major factors in increasing antibody diversity. Antibodies derived from the same germline V gene segment may also produce different sequences, but with relatively high similarity overall. The germline gene segments that are likely to undergo gene rearrangement can be deduced from the antibody variable region sequences using algorithms such as IMGT/DomainGapAlign (http://imgt.org/3Dstructure-DB/cgi/DomainGapAlign.cgi) or NCBI/IgBLAST (https://www.ncbi.nlm.nih.gov/igblast/).

The term "post-translational modification (PTM)|"refers to the process of introducing chemical modifications to proteins or polypeptide amino acid chains after translation and synthesis in cells. For antibodies, some PTM sites are very conservative. For example, the conservative amino acid asparagine (Asn) at position 297 (EU numbering) of the constant domain of the human IgG1 antibody is often glycosylated to form a saccharide chain whose structure is critical for antibody structure and associated effector functions. However, PTMs may have a greater effect on antigen binding or result in changes in the physicochemical properties of the antibody, if they are present in the variable regions, particularly in the antigen-binding regions (e.g., CDRs) of an antibody. For example, glycosylation, deamidation, isomerization, oxidation, and the like may increase the instability or heterogeneity of antibody molecules, thereby increasing the difficulty and risk of antibody development. Thus, the process of removing PTM to reduce sequence instability, thereby avoiding some potential PTMs, is very important for the development of therapeutic antibodies. As experience has accumulated, it has been found that some PTMs are highly correlated with the composition of amino acid sequences, especially the "pattern" of the composition of adjacent amino acids, which makes it possible to predict potential PTMs from the primary amino acid sequences of a protein. For example, it can be predicted that there is an N-linked glycosylation site from the sequence pattern of N-x-S/T (asparagine at the first position, any amino acid other than non-proline at the second position, and serine or threonine at the third position). The amino acid sequence patterns leading to PTMs may be derived from germline gene sequences, e.g., the human germline gene fragment IGHV3-33 naturally having a glycosylation pattern NST in the FR3 region; or they may also be derived from somatic hypermutations. The amino acid sequence patterns of PTMs may be disrupted by amino acid mutations, thereby reducing or eliminating the formation of specific PTMs. There are different methods for designing mutations depending on the antibody sequences and PTM sequence patterns. One method is to replace a "hot spot" amino acid (e.g., N or S in the NS pattern) with an amino acid with similar physicochemical properties (e.g., to mutate N into Q). If the PTM sequence pattern is derived from somatic hypermutations and is not present in the germline gene sequence, the other method can be to replace the sequence pattern with the corresponding germline gene sequence. In practice, a variety of methods for designing mutations may be used for the same PTM sequence pattern.

The term "antibody-drug conjugate" or "ADC" refers to a binding protein (e.g., an antibody or an antigen-binding fragment thereof) that is chemically linked to one or more chemical drugs. In a preferred embodiment, the ADC comprises a binding protein, a drug, and a linker linking the binding protein to the drug.

The term "chimeric antigen receptor" or "CAR" refers to receptors having desired antigen specificity and signaling domains to propagate intracellular signals upon antigen binding. For example, T lymphocytes recognize specific antigens through interaction between T cell receptors (TCRs) and short peptides presented by class I or II major histocompatibility complex (MHC) molecules. For initial activation and clonal expansion, initial T cells are dependent on antigen presenting cells (APCs) that provide additional co-stimulatory signals. In some embodiments, monocytes and macrophages can be engineered to, for example, express the chimeric antigen receptors (CAR). The modified cells can be recruited to the tumor microenvironment, where they serve as a potent immune effector by infiltrating tumors and killing target cancer cells. The CAR may comprise the antigen-binding domain, the transmembrane domain and the intracellular domain. The antigen-binding domain binds to an antigen on a target cell. Examples of cell surface markers that can serve as an antigen binding to the antigen-binding domain of the CAR comprise those associated with viral infection, bacterial infection, parasitic infection, autoimmune diseases and cancer cells (e.g., tumor antigens).

The term "modified immune cells" refer to immune cells that have been genetically modified to express the CAR. In some embodiments, the immune cells are T cells or cells derived therefrom. In some embodiments, the immune cells are natural killer (NK) cells, or cells derived therefrom. In some embodiments, the immune cells are B cells or cells derived therefrom. In some embodiments, the immune cells are B cells or cells derived therefrom. In some embodiments, the immune cells are monocytes or macrophages, or cells derived therefrom.

The term "kit of parts" refers to a combination in which one or more active ingredients are present in more than one unit. When multiple active ingredients are present in the kit of parts, the active ingredients may be administered simultaneously or sequentially.

The term "administration device" refers to any tool for administrating to a subject.

The term "pre-filled syringe" refers to a syringe that has been loaded with drugs prior to access or use by an operator of the syringe. The pre-filled syringe may be of any material (e.g., glass, plastic, or metal). In some embodiments, the pre-filled syringe is a glass syringe.

An "effective amount" of the drugs refers to the amount that is necessary to result in a physiological change in the cells or tissue to which the drugs are administered. An "effective amount" comprises an amount sufficient to ameliorate or prevent symptoms or conditions of a medical disease. An effective amount also refers to an amount sufficient to allow or facilitate diagnosis. The effective amount for a particular patient or veterinary subject can vary depending on the following factors such as the conditions to be treated, the overall health of the patient, the method, route and dosage of the administration, and the severity of side effects. An effective amount may be the maximum dosage or administration regimen that avoids significant side effects or toxic effects.

The bispecific antibodies according to the present invention have a synergistic effect. The "synergistic effect" means that the combined effect of the two drugs is greater than the sum of their individual effects, and is statistically different from that of the control and that of the single drug. The superimposed effect of the present invention means that the combined effect of the two drugs is the sum of their individual effects, and is statistically different from that of the control and/or that of the single drug.

A "therapeutically effective amount" of a drug (e.g., the pharmaceutical composition) refers to the amount necessary to effectively achieve the desired therapeutic or prophylactic effect, in dose and in the administration interval and time. For example, a therapeutically effective amount of a drug eliminates, alleviates/decreases, delays, minimizes or prevents adverse effects of diseases.

The term "individual" or "subject" refers to mammals. The mammals include, but are not limited to, domesticated animals (e.g. cows, sheep, cats, dogs, and horses), primates (e.g. humans and non-human primates (such as monkeys)), rabbits, and rodents (e.g. mice and rats). Particularly, the individual or the subject is a human.

The term "pharmaceutical composition" refers to a mixture containing one or more of the antibodies or the antigen-binding fragments thereof disclosed herein and other chemical components, such as physiologically/pharmaceutically acceptable carriers or excipients. The pharmaceutical composition is intended to promote the administration to an organism, which facilitates the absorption of the active ingredient, thereby exerting biological activities.

The term "pharmaceutically acceptable carrier" refers to a component of the pharmaceutical composition that is not toxic to a subject in addition to the active ingredients. The pharmaceutically acceptable excipients include, but are not limited to a buffer, a stabilizer and/or a preservative.

The term "cancer" refers to or describes a disease in mammals that is characterized by unregulated cell growth. Examples of cancers include, but are not limited to, tumors, lymphomas, blastomas, sarcomas and leukemias or lymphoid malignancies. More specific examples of cancers include, but are not limited to, squamous cell carcinoma (such as epithelial squamous cell carcinoma), lung cancer (including small-cell lung cancer, non-small cell lung cancer and adenocarcinoma and squamous carcinoma of the lung), peritoneal cancer, hepatocellular carcinoma, gastric cancer (including gastrointestinal cancer and gastrointestinal stromal cancer), bone cancer, pancreatic cancer, glioblastoma, cervical cancer, ovarian cancer, liver cancer, bladder cancer, urinary tract cancer, breast cancer, colon cancer, rectal cancer, colorectal cancer, endometrial cancer or cervical cancer, salivary gland carcinoma, kidney cancer or ureteral cancer, prostate cancer, vaginal cancer, vulval cancer, thyroid cancer, anal cancer, penile cancer, melanoma, cholangiocarcinoma, central nervous system (CNS) tumor, spinal axis tumor, brain stem glioma, glioblastoma multiforme, astrocytoma, schwannoma, ependymoma, myeloblastoma, meningioma, squamous cell carcinoma, pituitary adenoma and Ewing's sarcoma, superficial spreading melanoma, lentigo maligna melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloblastic leukemia and post-transplant lymphoproliferative disorder (PTLD), and abnormal vascular proliferation associated with phakomatoses, edema (such as those associated with brain tumors) and Meigs syndrome, brain tumor and brain cancer, and head or neck cancer, and related metastatic carcinomas.

The term "treating" refers to administering a therapeutic agent, such as a composition comprising any one of the antibodies or the antigen-binding fragments thereof disclosed herein or a nucleic acid molecule encoding the antibody or the antigen-binding fragment thereof, either internally or externally to a patient with one or more diseases or symptoms on which the therapeutic agent has a therapeutic effect. Typically, the therapeutic agent is administered in an amount effective to alleviate one or more diseases or symptoms to the patient or population being treated to induce regression of such symptoms or to inhibit the development of such symptoms to any clinically measurable degree.

The term "preventing cancer" refers to delaying, inhibiting or preventing the onset of cancers in mammals in which the initiation of carcinogenesis or tumorigenesis has not been confirmed, but a susceptibility to cancer has been identified as determined, for example, by genetic screening or other methods. The term also includes treating mammals having pre-cancerous disorders to terminate the progression of the pre-cancerous disorders to malignancies or to cause the regression thereof.

The sequence numbers (SEQ ID NO:) of the antibodies of the present application are shown in Table 3 below.

**Table 3:**

| Protein No. | Light chain | Heavy chain | V L | V H | LCDR 1 | LCDR 2 | LCDR 3 | HCDR 1 | HCDR 2 | HCDR 3 |
|---|---|---|---|---|---|---|---|---|---|---|
| PR000151 | 118 | 97 | 93 | 72 | 50 | 56 | 64 | 7 | 21 | 34 |
| PR000960 | | 99 | | 74 | | | | 9 | 23 | 36 |
| PR001058 | | 100 | | 75 | | | | 10 | 23 | 36 |
| PR001061 | | 101 | | 76 | | | | 11 | 24 | 36 |
| PR001065 | | 102 | | 77 | | | | 11 | 23 | 36 |
| PR002079 | | 104 | | 79 | | | | 13 | 23 | 36 |
| PR002082 | | 105 | | 80 | | | | 10 | 23 | 36 |
| PR005867 | | 108 | | 83 | | | | 10 | 23 | 39 |
| PR005868 | | 109 | | 84 | | | | 10 | 23 | 40 |
| PR005869 | | 110 | | 85 | | | | 10 | 23 | 41 |
| PR005263 | | 107 | | 82 | | | | 10 | 27 | 36 |
| PR005872 | | 111 | | 86 | | | | 10 | 27 | 39 |
| PR005875 | | 112 | | 87 | | | | 10 | 27 | 40 |
| PR005878 | | 113 | | 88 | | | | 10 | 27 | 41 |
| PR006245 | | 114 | | 89 | | | | 14 | 27 | 36 |
| PR006246 | | 115 | | 90 | | | | 14 | 27 | 39 |
| PR006247 | | 116 | | 91 | | | | 14 | 27 | 40 |
| PR006248 | | 117 | | 92 | | | | 14 | 27 | 41 |
| PR000846 | 119 | 98 | 94 | 73 | 51 | 57 | 65 | 8 | 22 | 35 |
| PR001408 | 120 | 103 | 95 | 78 | 52 | 58 | 66 | 12 | 25 | 37 |
| PR003836 | 121 | 106 | 96 | 81 | 53 | 59 | 67 | 10 | 26 | 38 |

The sequence numbers (SEQ ID NO:) of the bispecific antibodies of the present application are shown in Table 4 below.

**Table 4:**

| Protein No. | Polypeptide chain 1 | Polypeptide chain 2 |
|---|---|---|
| PR003569 | 122 | 119 |
| PR003739 | 123 | 120 |
| PR004295 | 124 | 121 |
| PR006268 | 125 | 119 |
| PR006269 | 126 | 119 |
| PR006270 | 127 | 119 |
| PR006271 | 128 | 119 |
| PR006663 | 129 | 119 |
| PR006667 | 132 | 131 |
| PR006664 | 130 | 119 |
| PR006668 | 133 | 131 |

### BRIEF DESCRIPTION OF THE DRAWINGS

FIGs. 1A and 1B show the results of binding of the PD-L1 antigen-binding protein of the present application to CHO-K1 cells overexpressing human PD-L1. FIG. 1C shows the results of binding of the post-translational modification (PTM) variants of PR000960 of the present application to the CHO-K1 cells overexpressing human PD-L1. FIG. 1D, FIG. 1E, and FIG. 1F show the results of binding of the affinity-matured variants of PR002082 of the present application to the CHO-K1 cells overexpressing human PD-L1.
FIG. 2A shows the results of binding of the PD-L1 antigen-binding protein of the present application to the CHO-K1 cells overexpressing cynomolgus monkey PD-L1. FIG. 2B shows the results of binding of the post-translational modification (PTM) variants ofPR000960 of the present application to the CHO-K1 cells overexpressing cynomolgus monkey PD-L1. FIG. 2C shows the results of binding of the affinity-matured variants of PR002082 of the present application to the CHO-K1 cells overexpressing monkey PD-L1.
FIG. 3A shows inhibitory effects of the PD-L1 antigen-binding protein of the present application on a PD-1 signaling pathway. FIG. 3B shows the inhibitory effects of the post-translational modification (PTM) variants of PR000960 of the present application on the PD-1 signaling pathway. FIG. 3C and FIG. 3D show the inhibitory effects of the affinity-matured variants of PR002082 of the present application on the PD-1 signaling pathway.
FIG. 4 shows the results that the PD-L1 antigen-binding protein of the present application enhances the activation of T lymphocytes to secrete cytokines IL-2 (FIG. 4A) and IFN-γ (FIG. 4B) in donor pairing #1 MLR experiments.
FIG. 5 shows the results that the PD-L1 antigen-binding protein of the present application enhances the activation of T lymphocytes to secrete cytokines IL-2 (FIG. 5A) and IFN-γ (FIG. 5B) in donor pairing #2 MLR experiments.
FIG. 6 shows the results that the post-translational modification (PTM) variants of PR000960 of the present application enhances the activation of T lymphocytes to secrete cytokines IL-2 (FIG. 6A) and IFN-γ (FIG. 6B) in donor pairing #3 MLR experiments.
FIG. 7 shows the results that the affinity-matured variants of PR002082 of the present application enhance the activation of T lymphocytes to secrete cytokines IL-2 (FIG. 7A) and IFN-γ (FIG. 7B) in donor pairing #4 MLR experiments.
FIG. 8 shows the results that the affinity-matured variants of PR002082 of the present application enhance the activation of T lymphocytes to secrete cytokines IL-2 (FIG. 8A) and IFN-γ (FIG. 8B) in donor pairing #5 MLR experiments.
FIG. 9 shows the results that the affinity-matured variants of PR002082 of the present application enhance the activation of T lymphocytes to secrete cytokines IL-2 (FIG. 9A) and IFN-γ (FIG. 9B) in donor pairing #6 MLR experiments.
FIG. 10 shows the results that the affinity-matured variants of PR002082 of the present application enhance the activation of T lymphocytes to secrete cytokines IL-2 (FIG. 10A) and IFN-γ (FIG. 10B) in donor pairing #7 MLR experiments.
FIG. 11 shows the results that the affinity-matured variants of PR002082 of the present application enhance the activation of T lymphocytes to secrete cytokines IL-2 (FIG. 11A) and IFN-γ (FIG. 11B) in donor pairing #8 MLR experiments.
FIG. 12 shows the results that the affinity-matured variants of PR002082 of the present application enhance the activation of T lymphocytes to secrete cytokines IL-2 (FIG. 12A) and IFN-γ (FIG. 12B) in donor pairing #9 MLR experiments.
FIG. 13 shows the *in vivo* anti-tumor effect in an NCG mouse A375 tumor model with reconstitution of a human PBMC immune system. FIG. 13B shows the results of body weight change in the animals of each group in the course of the experiment.
FIG. 14 shows schematic structural diagrams of the bispecific molecules according to the present invention.
FIG. 15 shows binding of antibodies to CHO-K1 cells stably expressing human CD73 and human PD-L1.
FIG. 16 shows the results of experiments in which the antibodies inhibit the enzymatic activity of CD73.
FIG. 17 shows inhibitory effects of the antibodies on the PD-1/PD-L1 signaling pathway detected by a reporter gene cell line.
FIG. 18 shows activation effect of fusion proteins on T cells detected by an MLR method *in vitro.*

### DETAILED DESCRIPTION

The examples shown below are intended to illustrate specific embodiments of the present invention and are not intended to limit the scope of the specification or claims in any way. The examples do not include detailed descriptions of conventional methods, such as those methods for constructing vectors and plasmids, methods for inserting genes encoding proteins into such vectors and plasmids, or methods for introducing plasmids into host cells. Such methods are well known to those of ordinary skill in the art and are described in numerous publications, including Sambrook, J., Fritsch, E. F. and Maniais, T. (1989) MolecuLar Cloning: A Laboratory Manual, 2nd edition, Cold spring Harbor Laboratory Press.

### Examples

### Example 1. Preparation of PD-L1 Antigen-Binding Protein

To obtain antibody molecules specifically binding to PD-L1, typically the PD-L1 antigen can be used to immunize experimental animals such as mice, rats, rabbits, sheep and camels, but the obtained antibody molecules are non-human. After obtaining non-human antibodies, these molecules need to be humanized by antibody engineering technology to reduce immunogenicity and improve druggability. However, the humanization of antibodies is complex in terms of the technology, and the humanized molecules tend to have reduced affinity for antigens. On the other hand, advances in transgenic technology have made it possible to develop genetically engineered mice that carry a human immunoglobulin immune repertoire and have the endogenous murine immune repertoire deleted. The antibodies produced by the transgenic mice have fully human sequences, so that further humanization is not needed, and the efficiency of developing therapeutic antibodies is greatly improved.

The Harbour HCAb mouse (Harbour Antibodies BV, WO 2002/085945 A3) is a transgenic mouse carrying an immune repertoire of human immunoglobulins, capable of producing novel "heavy chain"-only antibodies that are only half the size of conventional IgG antibodies. The antibodies produced have only human antibody "heavy chain" variable domains and mouse Fc constant domains. Due to the absence of light chain, this antibody almost solves the problems of light chain mismatch and heterodimerization, allowing the technical platform to develop products that are difficult to realize by the conventional antibody platform.

### 1.1 Immunization of HCAb mice with PD-L1

Harbour HCAb human antibody transgenic mice of 6-8 weeks old were subjected to multiple rounds of immunization with a soluble recombinant human PD-L1-mFc fusion protein (Novo Protein Inc. #CM06, batch No. 0330837). In each round of immunization, each mouse received a subcutaneous inguinal injection or intraperitoneal injection of 100 µL in total. In the first round of immunization, each mouse received an immunization with an immunogenic reagent prepared by mixing 50 µg of antigenic protein with complete Freund's adjuvant (Sigma, #F5881) in a 1:1 volume ratio. In each subsequent round of booster immunization, each mouse received an immunization with an immunogenic reagent prepared by mixing 25 µg of antigenic protein with Ribi adjuvant (Sigma Adjuvant System, #S6322). The interval between rounds of booster immunization was at least two weeks. In general, there are no more than five rounds of booster immunizations. The immunization was performed on days 0, 14, 28, 42, 56 and 70, and the antibody titer in serum of mice was detected on days 49 and 77. The last round of booster immunization was performed at a dose of 25 µg of antigenic protein per mouse 5 days before the isolation of HCAb mouse splenic B cells.

### 1.2 Acquisition of sequences of anti-PD-L1 HCAb antibodies

When the titer of the PD-L1-specific antibody in the serum of mice was determined to reach a certain level, spleen cells of the mice were taken, from which B cells were isolated, and the CD138-positive plasma cells and human PD-L1 antigen-positive B cell populations were sorted using a BD FACS AriaII cell sorter. The RNA of the B cells was extracted and reversely transcribed into cDNA (SuperScript IV First-Strand synthesis system, Invitrogen, #18091200), and human VH genes were amplified by PCR using specific primers. PCR primers were 5'-GGTGTCCA GTGTSAGGTGCAGCTG-3' (SEQ ID NO: 134), and 5'-AATCCCTGGGCACTGAAGAGACGG TGACC-3' (SEQ ID NO: 135). The amplified VH gene fragments were constructed into a mammalian cell expression plasmid pCAG vectors encoding the sequence of the heavy chain Fc domain of the human IgG1 antibody.

Mammal host cells (e.g., human embryonic kidney cell HEK293) were transfected with the constructed plasmids to express acquired HCAb antibodies. The binding of the HCAb-expressing supernatant to a stable cell line CHO-K1/hPD-L1 (GenScript, #M00543) overexpressing human PD-L1 was detected, while screening was performed by a Miroball fluorescent flow cytometer (Sptlabtech) with a positive antibody PR000151 (Atezolizumab analog, synthesized and expressed by the applicant according to an Atezolizumab sequence) used as a positive control.

The specific procedures were as follows: CHO-K1/hPD-L1 cells were washed with a serum-free F12K medium (Thermofisher, #21127022) and resuspended in a serum-free medium to 1×10⁶ cells/mL. Draq5 fluorescence probe (CTS, #4048L) (1 µL of Draq5 added to 1 mL of CHO-K1/hPD-L1 cells, diluted in a 1:1000 ratio) was added, and the mixture was incubated for 30 min away from the light. After centrifugation, the cells were washed with a medium and the cell density was adjusted to 1.0×10⁵ cells/mL. Then, Alexa Fluor^{®} 488, AffiniPure Goat Anti-Human IgG, Fcγ Fragment Specific secondary antibody (Jackson, ImmunoResearch, #109-545-098) diluted in a 1:1000 ratio was added, and the mixture was added to a 384-well plate (Greiner, #781091) at 30 µL/well. Then, the positive control or HCAb-expressing supernatant was added to the 384-well plate at 10 µL/well, and the mixture was incubated for 2 h. Fluorescence values were read on a Miroball fluorescent flow cytometer. The positive clone antibodies were further detected by FACS for the binding to CHO-K1/hPD-L1 cells, and detected by ELISA to verify cross-binding activity to cynomolgus monkey PD-L1-his proteins (Acrobiosystems, #PD-1-C52H4). The nucleotide sequences encoding the variable domains of the antibody molecules and the corresponding amino acid sequences were obtained by sequencing the positive antibodies using conventional sequencing means.

### 1.3 Preparation of recombinant fully human antibodies

After obtaining the sequences of heavy chain variable domains encoding the antibody molecules in Example 1.2, the sequences of the heavy chain variable domains can be fused with the corresponding sequences of the heavy chain constant domains of the human antibody and expressed by conventional recombinant DNA techniques to obtain recombinant antibody molecules.

The plasmid encoding the heavy chain of the antibody was transfected into a mammalian host cell (e.g., human embryonic kidney cell HEK293), and a recombinant antibody with HCAb heavy chain can be obtained using conventional recombinant protein expression and purification techniques.

Specifically, HEK293 cells were expanded in FreeStyle^{™} F17 Expression Medium (Thermo, #A1383504). Before the transient transfection, the cells were adjusted to a concentration of (6-8)×10⁵ cells/mL, and cultured in a shaker at 37 °C with 8% CO₂ for 24 h to make a concentration of 1.2×10⁶ cells/mL. 30 mL of cultured cells were taken. The above plasmids encoding the heavy chains of HCAbs were dissolved in 1.5 mL of Opti-MEM reduced serum medium (Thermo, #31985088), and sterilized through a 0.22 µm membrane filter. Then, 1.5 mL of Opti-MEM was dissolved in 120 µL of 1 mg/mL PEI (Polysciences Inc, #23966-2), and the mixture was left to stand for 5 min. PEI was slowly added into the plasmids, and the mixture was incubated at room temperature for 10 min. The mixed solution of plasmids and PEI was slowly added dropwise while shaking the culture flask, and the cells were cultured in a shaker at 37 °C with 8% CO₂ for 5 days. Cell viability was measured after 5 days. The culture was collected and centrifuged at 3300 g for 10 min, and then the supernatant was collected and centrifuged at high speed to remove impurities. A gravity column (Bio-Rad, #7311550) containing MabSelect^{™} (GE Healthcare Life Science, #71-5020-91 AE) was equilibrated with PBS (pH 7.4) and rinsed with 2-5 column volumes of PBS. The supernatant sample was loaded onto a column. The column was rinsed with 5-10 column volumes of PBS. The target protein was eluted with 0.1 M glycine (pH 3.5). The eluate was adjusted to neutrality with Tris-HCl (pH 8.0), and concentrated and buffer exchanged into PBS buffer with an ultrafiltration tube (Millipore, #UFC901024) to obtain a purified antibody solution. Then, the purified antibody solution was subjected to concentration determination using NanoDrop (Thermo Scientific^{™} NanoDrop^{™} One), subpackaged and stored for later use.

### 1.4 Sequences of PD-L1 fully human recombinant antibodies

The amino acid sequences of the heavy chain variable domains, the full-length amino acid sequences of the heavy chains and the amino acid sequences of the CDRs defined according to the Chothia scheme of the PD-L1 antibodies in this example are listed in Table 5. Meanwhile, the corresponding amino acid sequence of the positive control antibody Atezolizumab analog of the anti-PD-L1 of the present application is derived from an IMGT database, the heavy chain sequence of the antibody is SEQ ID NO: 97, and the light chain sequence of the antibody is SEQ ID NO: 118.

**Table 5. Sequence numbers (SEQ ID NO:) of anti-PD-L1 HCAb antibodies**

| **Recombinant antibody** | **Heavy chain sequence** | **VH sequence** | **VH CDR1** | **VH CDR2** | **VH CDR3** |
|---|---|---|---|---|---|
| **PR000960** | 99 | 74 | 9 | 23 | 36 |
| **PR001058** | 100 | 75 | 10 | 23 | 36 |
| **PR001061** | 101 | 76 | 11 | 24 | 36 |
| **PR001065** | 102 | 77 | 11 | 23 | 36 |

### 1.5 Analysis of protein purity and polymers by HPLC-SEC

Analytical size-exclusion chromatography (SEC) was used to analyze the protein sample for purity and polymer form. An analytical chromatography column TSKgel G3000SWxl (Tosoh Bioscience, #08541, 5 µm, 7.8 mm × 30 cm) was linked to a high-pressure liquid chromatograph (HPLC) (model: Agilent Technologies, Agilent 1260 Infinity II) and equilibrated with a PBS buffer at room temperature for at least 1 h. A proper amount of the protein sample (at least 10 µg, with the concentration adjusted to 1 mg/mL) was filtered through a 0.22 µm filter membrane and then injected into the system, and an HPLC program was set: the sample was passed through the chromatography column with PBS buffer (pH 7.4) at a flow rate of 1.0 mL/min for a maximum of 25 min. The detection wavelength was 280 nm. After being recorded, the chromatogram was integrated using ChemStation software and relevant data were calculated. An analysis was generated, with the retention time of the components with different molecular sizes in the sample reported.

The results of yield and purity analysis of PD-L1 antibodies are shown in Table 6.

**Table 6. Yield and purity analysis of PD-L1 antibodies**

| **No.** | **Recombinant antibody** | **Expression system and volume** | **Yield after first purification (mg/L)** | **HPLC-SEC Purity (%)** |
|---|---|---|---|---|
| 1 | **PR000960** | HEK293-6E (40 mL) | 48.25 | 95 |
| 2 | **PR001058** | HEK293-F (30 mL) | 26 | 100 |
| 3 | **PR001061** | HEK293-F (30 mL) | 60.33 | 71.75 |
| 4 | **PR001065** | HEK293-F (30 mL) | 66.67 | 71 |

### Example 2. Optimization of Antibody Sequences

### 2.1 Removal of PTM site

The antibody sequences in Example 1 were analyzed, and germline gene V gene segments for the heavy chain variable domain (VH) and predicted PTMs for the variable domain VH are listed in Table 7.

**Table 7. Germline gene analysis of sequences of anti-PD-L1 HCAb antibodies**

| **No.** | **Recombinant antibody** | **Molecular structure** | **IgG subtype** | **VH germline V gene** | **VH PTM** |
|---|---|---|---|---|---|
| 1 | **PR000960** | HCAb | Human IgG | IGHV3-33 | NxS/T (HCDR1); |
| | | | | | DG (HCDR2) |
| 2 | **PR001058** | HCAb | Human IgG | IGHV3-33 | DG (HCDR2) |
| 3 | **PR001061** | HCAb | Human IgG | IGHV3-33 | NxS/T (HCDR1); |
| | | | | | DG (HCDR2) |
| 4 | **PR001065** | HCAb | Human IgG | IGHV3-33 | NxS/T (HCDR1); |
| | | | | | DG (HCDR2) |

The new antibody molecules (referred to as PTM variants) resulting from amino acid mutations to the sequences of the PR000960 antibodies with potential PTM sites from Example 1 are listed in Table 8. The amino acid sequences of CDRs, the VH and the HC of the PTM variants of PR000960, and the amino acid sequences of the CDRs defined according to the Chothia scheme are listed in Table 9. All designed PTM variants were purified by the method described in Example 1.3 to obtain the purified recombinant antibodies, and further validated in subsequent functional experiments. The results of yield and purity analysis of PTM variants of PR000960 are shown in Table 10.

**Table 8. Mutation site designs for PR000960**

| Initial antibody | PTM variant | Mutation site | VH PTM of variant molecule |
|---|---|---|---|
| PR000960 | PR002079 | N28T | DG (HCDR2) |
| | PR002082 | N28T, N31S | DG (HCDR2) |

**Table 9. Amino acid sequences of CDR, VH and HC of PTM variants of PR000960**

| Initial antibody | PTM variant | HC | VH | HCDR1 | HCDR2 | HCDR3 |
|---|---|---|---|---|---|---|
| PR000960 | PR002079 | 104 | 79 | 13 | 23 | 36 |
| | PR002082 | 105 | 80 | 10 | 23 | 36 |

**Table 10. Yield and purity analysis of post-translational modification (PTM) variants of PR000960**

| **Antibody No.** | **Concentration (mg/mL)** | **Purity HPLC-SEC (%)** | **Endotoxin level (EU/mg)** | **yield (mg/L)** | **HPLC-HIC retention time (min)** | **DSF Tml** |
|---|---|---|---|---|---|---|
| **PR002079** | 3.64 | 97.85 | 0.1 | 182 | 18.342 | 54.00 |
| **PR002082** | 2.27 | 97.25 | 0.1 | 113.5 | 18.456 | 52.20 |

### 2.2 Optimization of HCAb antibody sequences to improve binding affinity for PD-L1

A yeast display antibody mutation library was established by a method of randomly mutating a CDR region, and the affinity of PR002082 molecules was modified by means of a BD FACS AriaIII sorting platform. This method of affinity maturation was divided into three rounds.

Firstly, mutations were randomly introduced into three CDRs of female parent molecules PR002082 to establish yeast display mutation libraries of 3 CDRs (CDR1, CDR2 and CDR3), and then enrichment was performed by an MACS sorting method; and subsequently, multiple rounds of sorting were performed with flow FACS to enrich mutant molecules having high affinity. In this example, the CDR sequences of the antibody variable domains were analyzed according to the Chothia scheme.

In the first round, 0.1 nM biotinylated PD-L1-his was used to sort out yeast cells having higher binding ability; and then, the culture induction was continued.

In the second round, 0.01 nM biotinylated PD-L1-his was used to further sort out yeast cells with higher binding ability; and then, the culture induction was continued.

In the third round, reducing the concentration of antigens during sorting was continued, and the last round of sorting was performed with 0.003 nM biotinylated PD-L1-his.

Finally, molecules sorted out from the previous three rounds were sequenced, and mutation sites were found and randomly combined to establish a combinatorial mutation library. FACS was then used to continue to sort out mutant molecules having higher affinity at lower antigen concentrations. In this example, the sequence of the heavy chain variable domain (VH) of the antibody acquired by the affinity maturation was genetically synthesized and cloned into a mammalian cell expression plasmid vector encoding Flag and 6×His tag to encode and produce a recombinant HCAb single-domain antibody molecule.

The sequences of the obtained PR002082 variants (all single-domain antibodies) are shown in Table 11 below.

**Table 11. Sequence numbers (SEQ ID NO:) of PR002082 variants**

| **Initial antibody** | **Recombinant antibody (Variant)** | **Variable region mutations** | **IgG subtype** | **Fc mutation** | **VH (SEQ ID NO:)** | **HC (SEQ ID NO:)** |
|---|---|---|---|---|---|---|
| PR002082 | **PR005867** | V106I, A109D | HCAb | none | 83 | 108 |
| PR002082 | **PR005868** | I102L, V106I | HCAb | none | 84 | 109 |
| PR002082 | **PR005869** | I102L, V106I, A109D | HCAb | none | 85 | 110 |
| PR002082 | **PR005263** | I50S, W52R, D54T, K57E | HCAb | none | 82 | 107 |
| PR002082 | **PR005872** | I50S, W52R, D54T, K57E, V106I, A109D | HCAb | none | 86 | 111 |
| PR002082 | **PR005875** | I50S, W52R, D54T, K57E, I102L, V106I | HCAb | none | 87 | 112 |
| PR002082 | **PR005878** | I50S, W52R, D54T, K57E, I102L, V106I, A109D | HCAb | none | 88 | 113 |
| PR002082 | **PR006245** | T28D, 150S, W52R, D54T, K57E, | HCAb | none | 89 | 114 |
| PR002082 | **PR006246** | T28D, I50S, W52R, D54T, K57E, V106I, A109D | HCAb | none | 90 | 115 |
| PR002082 | **PR006247** | T28D, I50S, W52R, D54T, K57E, I102L, V106I | HCAb | none | 91 | 116 |
| PR002082 | **PR006248** | T28D, I50S, W52R, D54T, K57E, I102L, V106I, A109D | HCAb | none | 92 | 117 |

### 2.3 Production and purification of affinity variants of PR002082

After obtaining the sequences encoding variable domains of the affinity variants of PR002082 in Example 22 above, the sequences of the heavy chain variable domains can be fused with the corresponding purification tags and expressed by conventional recombinant DNA techniques to obtain recombinant HCAb single-domain antibody molecules.

The plasmids encoding recombinant HCAb single-domain antibodies were transfected into mammalian host cells (e.g., Chinese Hamster Ovary (CHO) cells), and the corresponding purified recombinant antibodies can be obtained using conventional recombinant protein expression and purification techniques.

Specifically, ExpiCHO-S^{™} cells (Gibco, #A29127) were expanded in ExpiCHO^{™} Expression Medium (Gibco, #A2910001). Before the transient transfection, the cells were adjusted to a concentration of (3-4)×10⁶ cells/mL, and cultured in a shaker at 37 °C with 8% CO₂ for 24 h to make a concentration of (7-10)×10⁶ cells/mL. The cells were then diluted to 6×10⁶ cells/mL, and 10 mL of the cultured cells was prepared. 8 µg of the above plasmids encoding HCAb single-domain antibodies (the ratio of the plasmids to cells was 0.8 µg:1 mL) were dissolved in 0.4 mL of OptiPRO^{™} SFM medium (Gibco, #12309019), and the mixture was filtered through a 0.22 µm filter membrane for sterilization. Then 32 µL of ExpiFectamine^{™} CHO reagent (Gibco, #A29129) was added to 0.37 mL of OptiPRO^{™} SFM medium (Gibco, #12309019). The ExpiFectamine^{™} CHO reagent solution was immediately added slowly to the plasmid solution. The mixture was inverted to be well mixed. The mixed solution of plasmid and transfection reagent was slowly added dropwise while shaking the flask. The cells were cultured in an 8% CO₂ shaker at 37 °C for 8-9 days. Cell viability was measured after 8 days. The culture was collected and centrifuged at 3300 g for 10 min, and then the supernatant was collected and filtered through a 0.22 µm filter membrane to remove impurities. A gravity column (Bio-Rad, #7311550) containing Ni Sepharose excel (GE Healthcare Life Science, #17531802) was equilibrated with PBS (pH 7.4) and rinsed with 2-5 column volumes of PBS. The supernatant sample was loaded onto a column. The column was rinsed successively with 5-10 column volumes of PBS and 5-10 column volumes of a 20 mM imidazole solution (pH 7.4). The target protein was eluted with a 500 mM imidazole solution (pH 7.4), and the eluate was concentrated and buffer exchanged into PBS buffer with an ultrafiltration tube (Millipore, #UFC901024) to obtain a purified antibody solution. Then, the purified antibody solution was subjected to concentration determination using NanoDrop (Thermo Scientific^{™} NanoDrop^{™} One), subpackaged and stored for later use. Preparation of affinity-matured variants of PR002082 is shown in Table 12.

**Table 12. Preparation of affinity-matured variants of PR002082**

| Antibody No. | Concentration (mg/mL) | Molecular weight (kDa) | Buffer |
|---|---|---|---|
| **PR005263** | 6.69 | 78.8 | PBS |
| **PR005867** | 0.78 | 79.1 | PBS |
| **PR005868** | 1.11 | 79.0 | PBS |
| **PR005869** | 1.13 | 79.1 | PBS |
| **PR005872** | 1.96 | 78.9 | PBS |
| **PR005875** | 1.91 | 78.8 | PBS |
| **PR005878** | 129 | 78.9 | PBS |
| **PR006245** | 6.42 | 78.8 | PBS |
| **PR006246** | 6.56 | 78.9 | PBS |
| **PR006247** | 6.42 | 78.9 | PBS |
| **PR006248** | 6.48 | 78.9 | PBS |

### Example 3. Binding of Antigen-Binding Proteins to Cells Overexpressing Human/Cynomolgus Monkey PD-L1 (FACS)

In the example, in order to study the *in vitro* binding activity of the PD-L1 antigen-binding protein to human/cynomolgus monkey PD-L1, cell-level binding experiments were performed using CHO-K1 cell strains overexpressing human PD-L1 (CHO-K1/hPD-L1, GenScript, M00543) or CHO-K1 cell strains overexpressing cynomolgus monkey PD-L1 (CHO-K1/cynoPD-L1, GenScript, M00573). Briefly, the PD-L1 cells were digested and resuspended in an F-12K complete medium, and the cell density was adjusted to 1×10⁶ cells/mL. The cells were seeded in a 96-well V-bottom plate (Corning, 3894) at 100 µL/well, followed by the addition of the test antigen-binding proteins serially diluted 5-fold with concentrations that were twice the final concentrations at 100 µL/well. The mixtures were well mixed. The highest final concentration of the antigen-binding proteins was 100 nM. A total of 8 concentrations were set. An isotype antibody hIgG 1 iso was used as a control. The cells were incubated away from light at 4 °C for 1 h. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (goat anti-human IgG (H+L) secondary antibody, Alexa Fluor 488 conjugate, Invitrogen, A11013, diluted at the ratio of 1: 1000) was added to each well. The plate was incubated away from light at 4 °C for 30 min. The cells in each well were then rinsed twice with 200 µL of pre-cooled PBS and centrifuged at 500 g at 4 °C for 5 min, and then the supernatant was discarded. Finally, a pre-cooled PBS was added at 200 µL/well to resuspend the cells. Fluorescence signal values were read using a BD FACS CANTOII. Fluorescence signal values were read using a flow cytometer BD FACS CANTOII or FACS ACEA, and the data were processed and analyzed using FlowJo v10 (FlowJo, LLC) software. The data were processed and analyzed by plotting using GraphPad Prism 8 software, and binding curves, EC₅₀ values and other parameters were acquired through four-parameter nonlinear fitting.

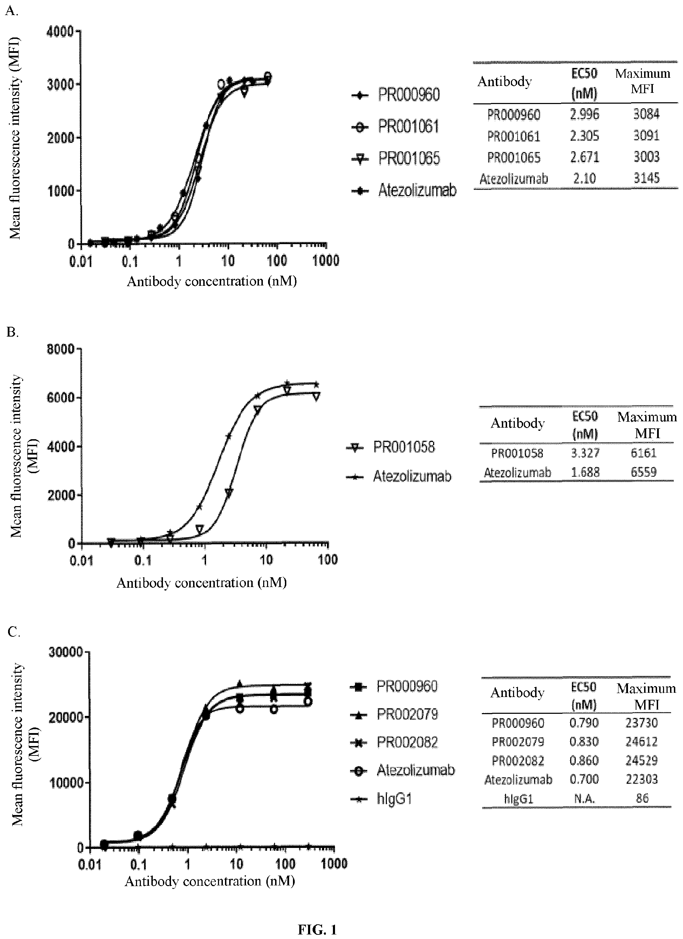

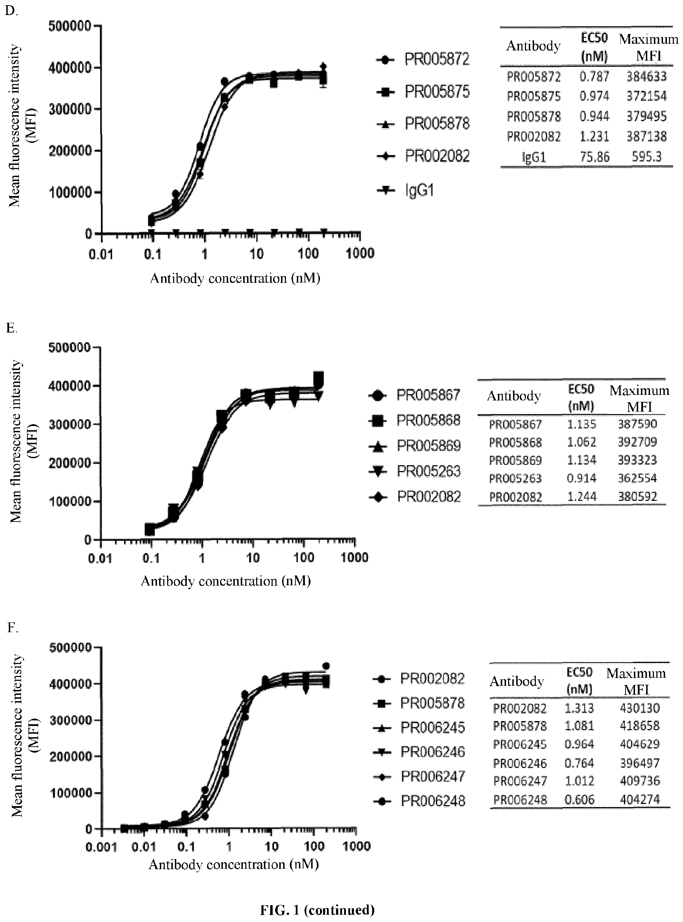

The results in FIG. 1 (A, B) show that the PD-L1 antigen-binding proteins of the present application can all bind to CHO-K1 cells overexpressing human PD-L1.

The results in FIG. 1 (C) show that post-translational modification (PTM) variants of PR000960 of the present application can all bind to CHO-K1 cells overexpressing human PD-L1.

The results in FIG. 1 (D, E, F) show that the affinity-matured variants of PR002082 of the present application can all bind to CHO-K1 cells overexpressing human PD-L1, and the effects of the affinity-matured variants of PR002082 were stronger than those of the parent sequence antibody PR002082.

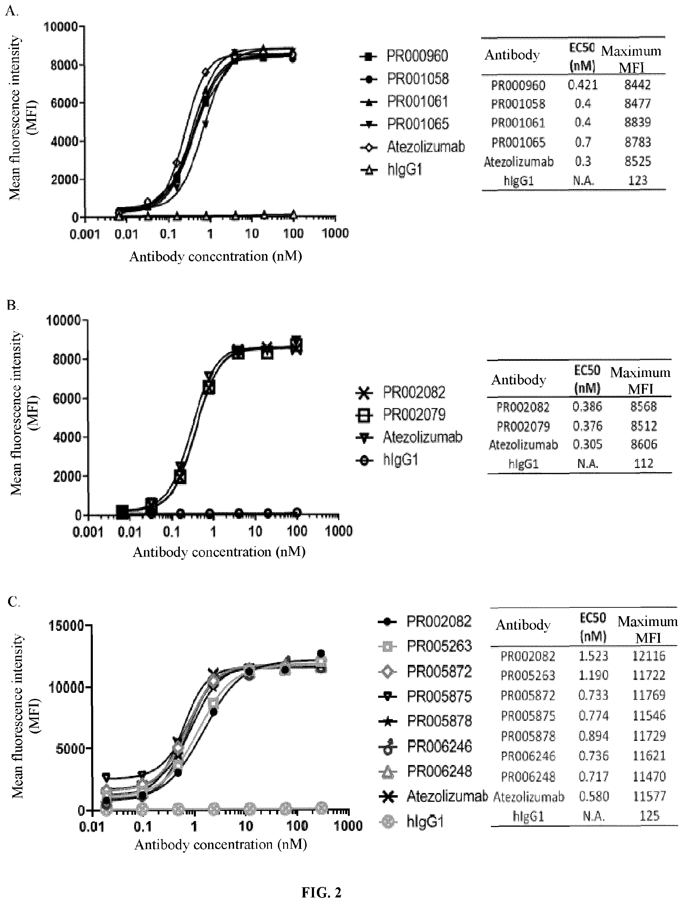

The results in FIG. 2 (A) show that the PD-L1 antigen-binding proteins of the present application can all bind to CHO-K1 cells overexpressing cynomolgus monkey PD-L1.

The results in FIG. 2 (B) show that post-translational modification (PTM) variants of PR000960 of the present application can all bind to CHO-K1 cells overexpressing cynomolgus monkey PD-L1.

The results in FIG. 2 (C) show that the affinity-matured variants of PR002082 of the present application can all bind to CHO-K1 cells overexpressing monkey PD-L1, and the effects were stronger than those of the parent sequence antibody PR002082.

### Example 4. Determination of Affinity of Antigen-Binding Proteins for Human PD-L1 by BLI Method

In this example, affinity determination was performed on the affinity-matured mutants of PR002082 using an Octet Red96e instrument. The specific method was: human PD-L1 proteins with histidine tags were purchased from the manufacturer ACROBiosystems (# PD-1-H5258, and the detection buffer was 1× kinetics buffer (diluted from 10×kinetics buffer (ForteBio, #18-1105))) for affinity test and dilution of antigens and antibodies. The binding kinetics between the antigen and the antibody was analyzed by the biolayer interferometry (BLI) technique using an Octet molecular interaction analyzer (ForteBio, model: Octet Red96e).

When the affinity of the antigen for the antibody was determined, the rotation speed of the sensor was set at 1000 rpm/min. The 2 HIS1K sensors placed in a column were first equilibrated for 10 min in test buffer, then His PD-L1 was captured with the HIS1K sensor at a capture height of 0.9-1.1 nm, then the HIS1K sensor was equilibrated for 2 min in the test buffer, then the HIS1K sensor was bound to the antigen-binding protein (60 nM and 0 nM) for 3 min, and finally the mixture was dissociated for 15 min. The HIS1K sensor was immersed in a 10 mM glycine solution (pH 1.5) for regeneration to elute the proteins bound to the sensor.

When data analysis was performed using Octet Data Analysis software (Fortebio, version 11.0), the reference signals were subtracted by a single reference mode (reference well), the data were fitted by a "1:1 Local/full fitting" method, and the kinetic parameters of the binding of the antigen to the antibody were calculated to obtain Kₒₙ (1/Ms) values, K_{dis} (1/s) values and KD (M) values.

The results are shown in Table 13. The affinity-matured variants of PR02082 were capable of binding to PD-L1, and the affinity of most variants was significantly improved compared to that of PR002082.

**Table 13. Affinity of affinity-matured variants of PR002082 to human PD-L1 proteins**

| Antibody No. | **KD (M)** | **Kₒₙ(1/Ms)** | **K_{dis}(1/s)** | **Full R^2** |
|---|---|---|---|---|
| **PR002082** | 5.61E-09 | 2.10E+05 | 1.18E-03 | 0.9982 |
| **Atezolizumab** | 8.18E-10 | 3.79E+05 | 3.10E-04 | 0.9975 |
| **PR005867** | 1.85E-09 | 2.53E+05 | 4.68E-04 | 0.9991 |
| **PR005868** | 1.92E-09 | 2.01E+05 | 3.86E-04 | 0.9975 |
| **PR005869** | 1.09E-09 | 2.41E+05 | 2.62E-04 | 0.9989 |
| **PR005263** | 1.94E-09 | 1.17E+05 | 2.27E-04 | 0.9983 |
| **PR005872** | 8.61E-10 | 2.18E+05 | 1.87E-04 | 0.9993 |
| **PR005875** | 1.04E-09 | 1.72E+05 | 1.79E-04 | 0.9987 |
| **PR005878** | 6.71E-10 | 1.80E+05 | 1.21E-04 | 0.9990 |
| **PR006245** | 1.09E-09 | 1.49E+05 | 1.62E-04 | 0.9959 |
| **PR006246** | 5.46E-10 | 3.01E+05 | 1.64E-04 | 0.9956 |
| **PR006247** | 1.18E-09 | 1.85E+05 | 2.19E-04 | 0.998 |
| **PR006248** | 6.34E-10 | 1.82E+05 | 1.15E-04 | 0.998 |

### Example 5. Inhibition of PD-1 Signaling Pathway by Antigen-Binding Proteins as Detected Using Reporter Gene Cell Line

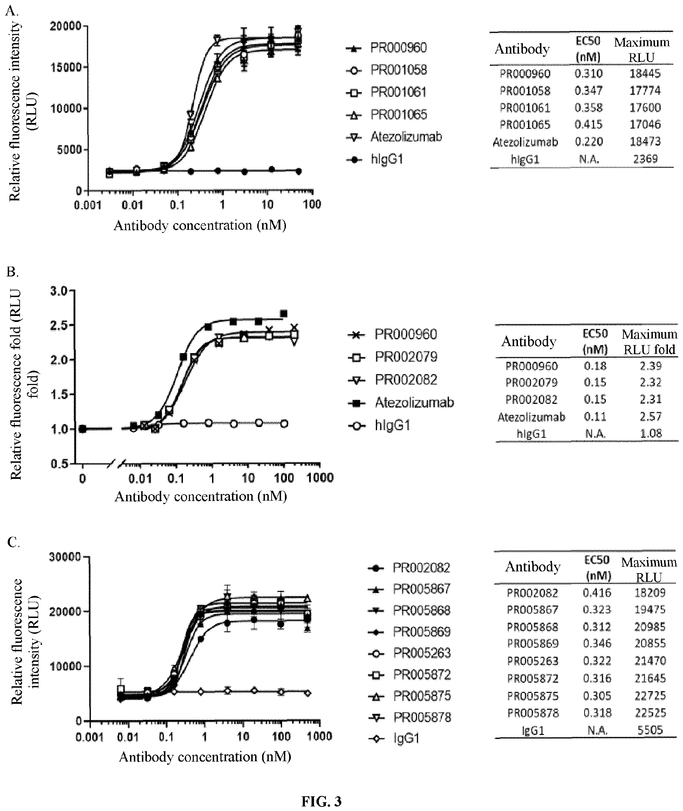

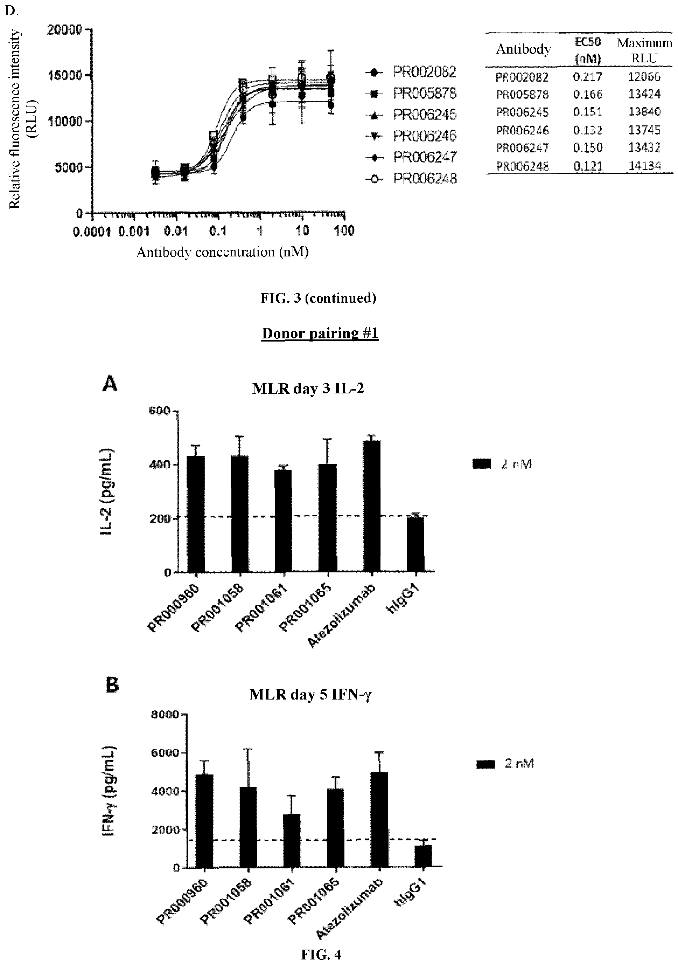

293T (eBioscience) cells expressing PD-L1 and OS8 (CD3 single-chain antibody transmembrane protein) were plated on a 96-well plate at 1.25×10⁴ cells/well and 100 µL/well. The cells were incubated at 37 °C with 5% CO₂ overnight. The supernatant was removed, and a dilution of the test antigen-binding protein was added at 50 µL/well. The initial concentration was 100 nM, and 5-fold dilution was performed. hlgG1 was used as a control group. Jurkat reporter cells capable of constantly expressing PD-1 and NFAT-luciferase reporter genes (UPharm) were added at 5×10⁴ cells/well, 50 µL/well. The cells were incubated at 37 °C with 5% CO₂ for 6 h. ONE-GloTM luciferase reagent (Promega, #E6110) was added. The cells were incubated at room temperature for 5 min, and the luminescence values were measured using a microplate reader. The results are shown in FIG. 3. The results show that the HCAb antibodies of the present application had an inhibitory effect on the PD-1 signaling pathway.

The results in FIG. 3 (A) show that the PD-L1 antigen-binding protein of the present application had an inhibitory effect on the PD-1 signaling pathway.

The results in FIG. 3 (B) show that the post-translational modification (PTM) variants of PR000960 of the present application had an inhibitory effect on the PD-1 signaling pathway.

The results in FIG. 3 (C, D) show that the affinity-matured variants of PR002082 of the present application had an inhibitory effect on PD-1 signaling pathway, and the inhibitory effect of most variants was significantly improved compared with those of PR002082.

### Example 6. Stimulation of Cytokine Secretion by Antigen-Binding Proteins in Mixed Lymphocyte Reaction (MLR)

AllCells PBMC cells were purchased, monocytes were isolated, and recombinant human interleukin 4 (IL-4) (R&D, #204-GMP) and human GM-CSF (R&D, #215-GM/CF) were added. After 6 days of induction, immature human CD14+ dendritic cells (iDC cells) were obtained. 1 µg/mL lipopolysaccharide (LPS; Sigma, #L2630) was then added, and after 24 h of induction, mature dendritic cells (mDC cells) were obtained. T lymphocytes were isolated from PBMC cells of a second donor using a T cell isolation kit (StemCell, #17951). T lymphocytes and mDC cells were seeded in a 96-well plate (T lymphocytes at 1×10⁵ cells/well and mDC cells at 1×10⁴ cells/well) at a ratio of 10:1. The antigen-binding proteins and the negative and positive control antibodies diluted at 10 fold or corresponding fold were added. The cells were incubated in an incubator at 37 °C with 5% CO₂ for 5 days. Supernatants on day 3 and on day 5 were collected and assayed for the secretion of IL-2 (ThermoFisher, #88-7025-88) and IFN-γ (ThermoFisher, #88-7316-88), respectively, using an ELISA kit.

In this example, PBMCs of 18 donors were divided into nine donor pairings for mixed lymphocyte reaction (MLR).

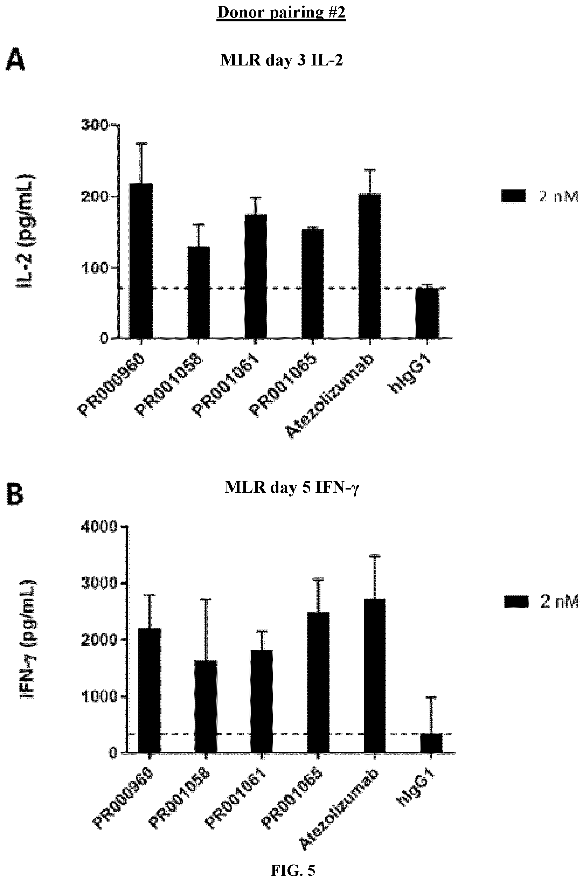

FIG. 4 and FIG. 5 show that the PD-L1 antigen-binding protein of the present application can enhance the activation of T lymphocytes to secrete cytokines IL-2 and IFN-γ in two independent MLR experiments.

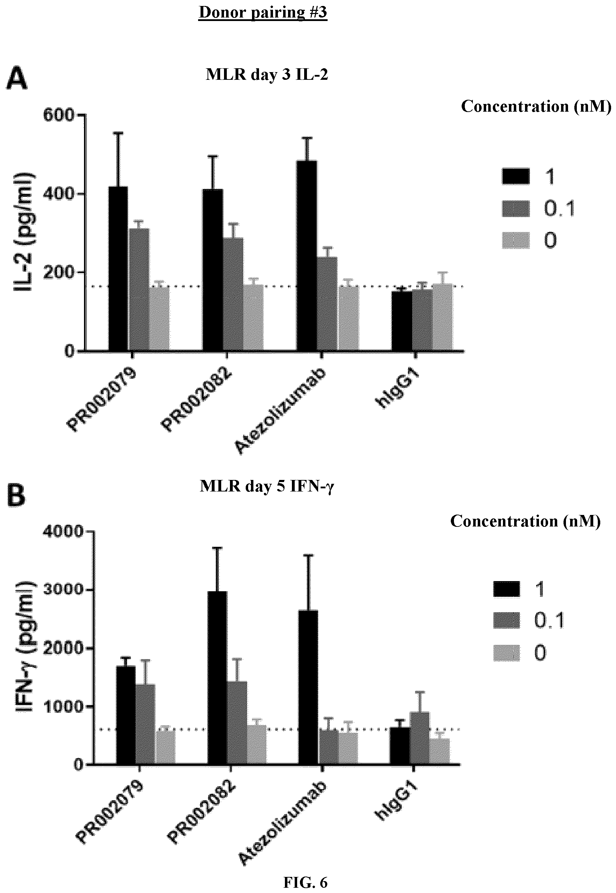

FIG. 6 shows that post-translational modification (PTM) variants of PR000960 of the present application can enhance activation of T lymphocytes to secrete cytokines IL-2 and IFN-γ.

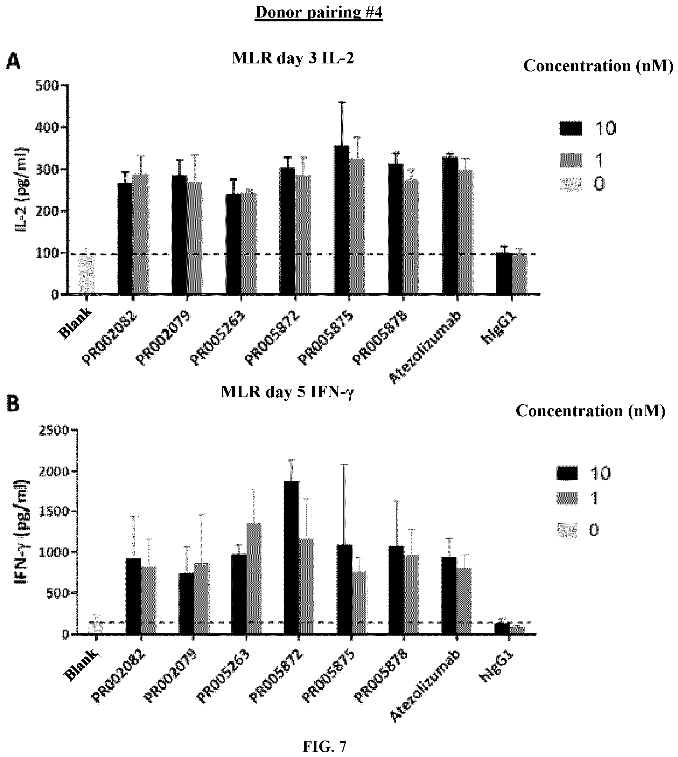

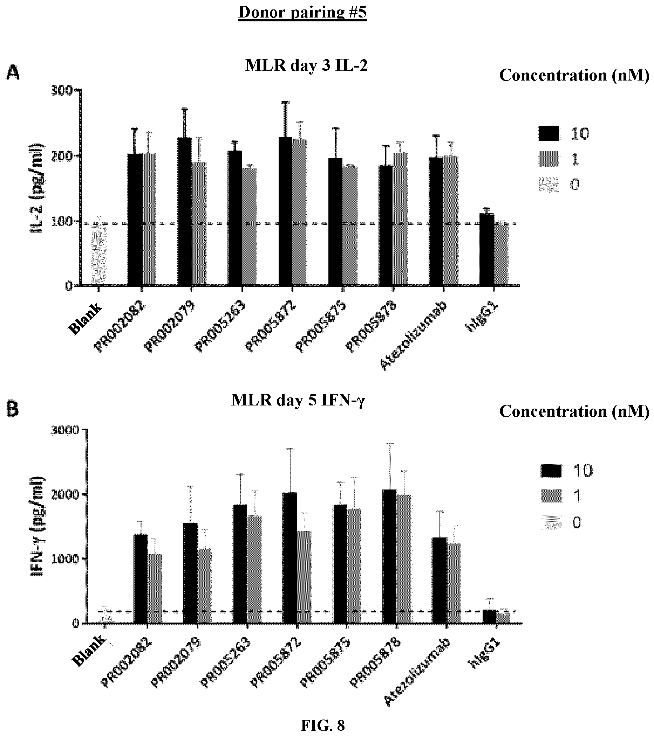

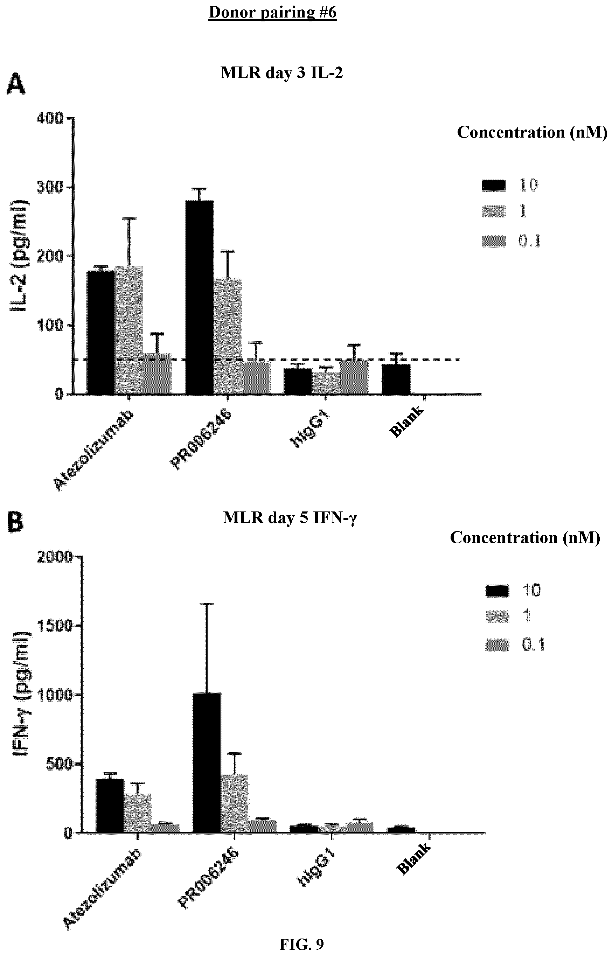

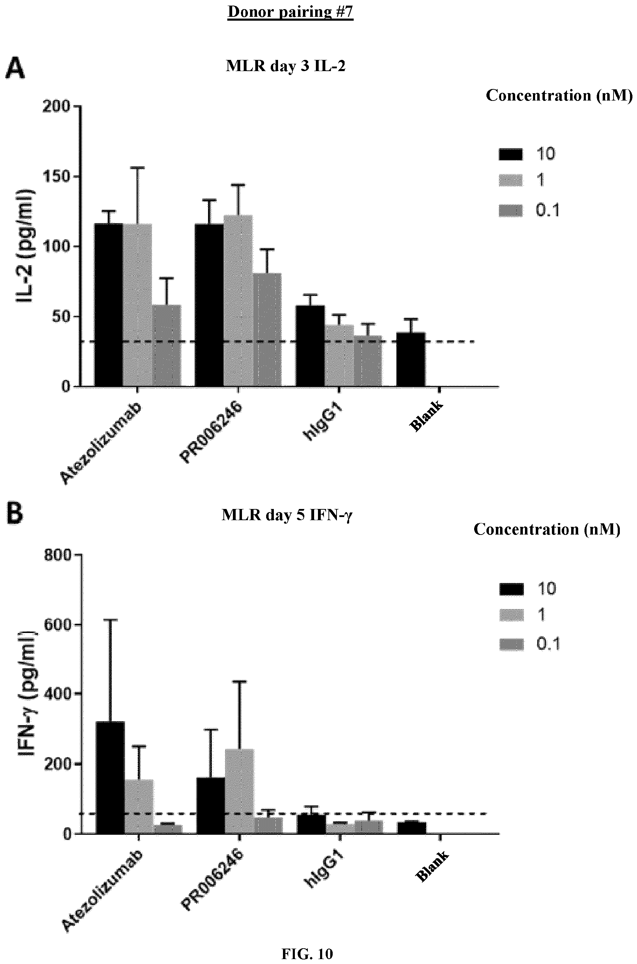

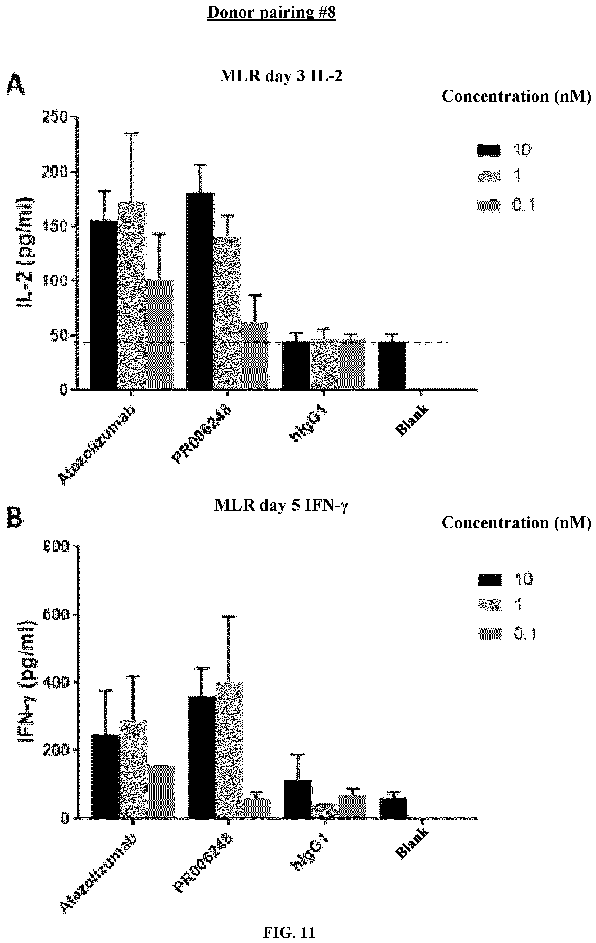

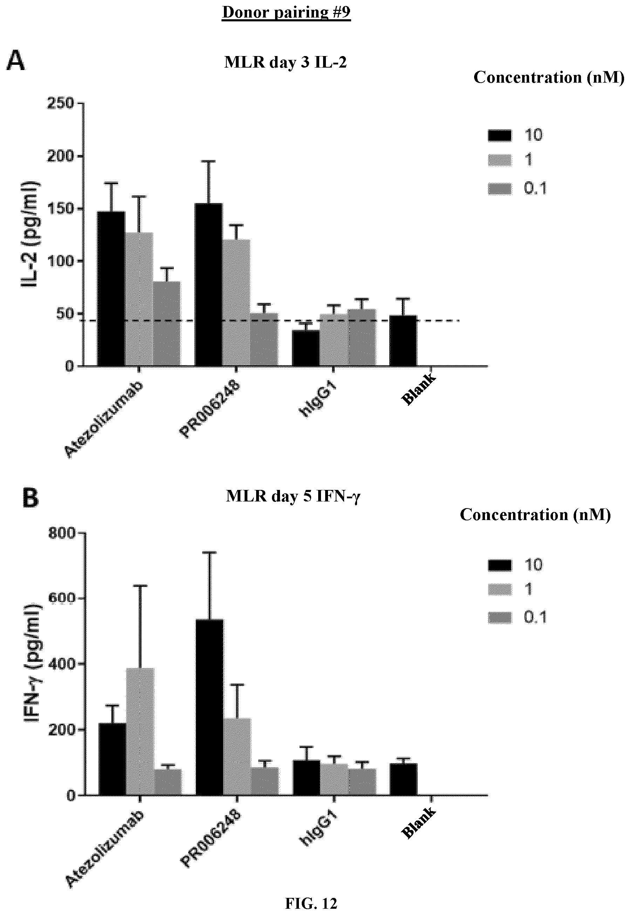

FIG. 7, FIG. 8, FIG. 9, FIG. 10, FIG. 11, and FIG. 12 show that the affinity-matured variants of PR002082 of the present application can enhance activation of the T lymphocytes to secrete the cytokines IL-2 and the IFN-γ in 6 independent MLR experiments.

### Example 7. In Vivo Inhibition of Tumor Activity by Antigen-binding Proteins

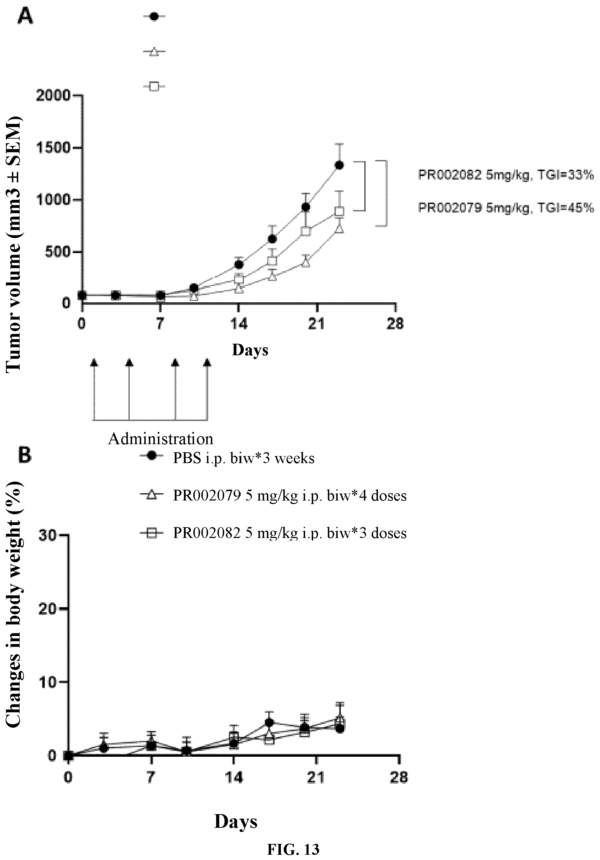

The *in vivo* anti-tumor effect in an NCG mouse A3 75 tumor model with reconstitution of a human PBMC immune system is shown in FIG. 13A. Specifically, the mean tumor volume of the mice in the vehicle control group on day 23 after administration was 1336 mm³. The mean tumor volume of the test drug Atezolizumab (10 mg/kg) treatment group on day 23 after inoculation was 343 mm³, showing a significant difference (p value = 0.002) from that of the vehicle control group, with the tumor growth inhibition rate TGI (%) being 74.33%. The mean tumor volume of the test drug PR002079 (5mg/kg) treatment group at day 23 after inoculation was 728 mm³, showing a significant difference (p value = 0.021) from that of the vehicle control group, with the tumor growth inhibition rate TGI (%) being 45.51%. The mean tumor volume of the test drug PR002082 (5 mg/kg) treatment group on day 23 after inoculation was 891 mm³, showing a significant difference (p value = 0.139) from that of the vehicle control group, with the tumor growth inhibition rate TGI (%) being 33.31%.

The results in FIG. 13B show that the animals in each group gained weight during the experiment, which indicates that the test sample was well tolerable in the animals; the test sample did not produce a significant toxic effect on the animals, which indicates that it has good safety.

### Example 8. Acquisition of CD73 Antibodies

Acquisition of the CD73 antibodies was described in Example 1, Example 2 and Example 3 of the specification of published application WO2021032173A1.

Specifically, CD73 proteins were used as immunogens to generate anti-CD73 antibodies using Harbour H2L2 mice. Each mouse was administrated with 50 µg of the proteins for the first boost and 25 µg for the subsequent boost via subcutaneous and intraperitoneal injection together with an adjuvant (Sigma, S6322). Such immunization was performed once every two weeks, 6 times in total. Final immunization was performed with immunogens diluted in PBS via intraperitoneal injection. Serum titers against human CD73 were tested using ELISA and FACS. At designated time points, mouse serum was sampled and titrated for ELISA and FACS analysis to test binding to the CD73 proteins or CD73 stable cell lines. Good serum titers were observed in protein immunization cohorts.

The mouse spleen cells, isolated from immunized mice, were fused to a mouse myeloma cell line SP2/0 (ATCC, CRL-1581) by an electric field based electroporator using a cell fusion generator (BEX-LF301) to acquire hybridomas. 9-14 days after fusion, individual wells were screened for human anti-CD73 antibodies. After hybridoma sub-cloning, single clones 38H6 showing good binding activities to CD73 were selected for sequencing. The heavy chain variable regions and the light chain variable regions were identified and acquired. Then the heavy chain variable regions and the light chain variable regions were synthesized and cloned into plasmids encoding the human IgG1 constant region and plasmids encoding the human Igκ region, respectively.

The recombinant plasmids encoding target antibodies were transiently co-transfected into HEK293-6E or HEK293-F cell cultures using PEI (Polyscience, 24885). 30 µg of plasmids and 120 µL of PEI were mixed and incubated at room temperature for 15 min. Next, the mixture was added dropwise to 293 cells suspended in Opti-MEM at the concentration of 1×10⁶ cells/mL. After transfection, the cells were cultured on a shaker at 37 °C with 5% CO2 at 120 rpm. The cell culture supernatants collected on day 6-7 were used for purification.

The supernatant containing the target antibodies was harvested by centrifugation and filtration 6-7 days after transfection. Monoclonal antibodies were purified by passing through the rProtein A (GE, 17-1279-02) pre-loaded column (Bio-Rad, 7311550). The Protein A column was prepared by packing 0.2 mL of rProtein A resin in one column and then rinsing with 10 column volumes of ddH2O and 10 column volumes of PBS. The cell culture supernatant was applied to the column followed by washing with 10 column volumes of PBS. Then the proteins were eluted with 8 column volumes of elution buffer (Thermo, 21004), and mixed immediately with 640 µL of neutralization buffer (1 M Tris-HCl, pH 9.0; Teknova, T1090) after elution. In a concentrator (Millipore, UFC903024), the buffer was exchanged with DPBS for more than 500-fold by centrifugation at 3800 rpm (Eppendorf, 5810R) at 4 °C, and finally concentrated to an appropriate volume. The concentration of the purified anti-CD73 antibodies were determined by UV absorbance at 280 nm (NanoDrop). Antibody purity was determined by SEC-HPLC and SDS-PAGE. The recombinant antibody PR000506 was successfully expressed and purified for characterization.

The VH and VL sequences of the anti-CD73 antibodies were further optimized by germline back mutation and PTM removal operations.

In the germline procedure, the VH or VL sequence of the antibodies was firstly aligned to the closest human germline sequence by for example NCBI/Ig-BLAST algorithms, and then residues, in the framework regions, different from the germline sequence were reversed to the corresponding residues in the germline sequence. The antibodies consisting of the sequence variants after germlining were then recombinantly produced by well-established molecular biology techniques.

Post-translational modification (PTM) was widely observed in proteins expressed in mammalian cells. Except for conserved PTM sites in antibodies (for example, conserved N-glycosylation sites on CH2 domains of IgG1 antibodies), other PTM sites occurred within antigen-binding sites of the antibodies (i.e. CDR regions) may reduce antigen-binding activity or reduce chemical stability. For example, deamidation or isomerization may make the molecules unstable and heterogenous. To reduce sequence instability, PTM motifs could be removed by mutations. PTM motifs, e.g., isomerization motifs (e.g. DG) of the VH or VL sequence were searched. Then "hotspot" residues (e.g., D or G in DG motifs) were mutated into the corresponding residues in the germline sequence or other residues with similar biophysical properties. The antibodies consisting of the sequence variants after PTM removal were then recombinantly produced by well-established molecular biology techniques.

The following anti-CD73 antibodies in Table 14 were acquired:

**Table 14:**

| Antibody No. | PTM removal | Light chain | Heavy chain | VL | VH |
|---|---|---|---|---|---|
| PR000846 | Removed | 119 | 98 | 94 | 73 |
| PR001408 | Removed | 120 | 103 | 95 | 78 |
| PR003836 | Removed | 121 | 106 | 96 | 81 |

### Example 9. Preparation of CD73/PD-L1 Bispecific Antibodies

In this example, the antigen-binding domain Fab of anti-CD73 IgG antibodies PR000846, PR001408 and PR003836, as well as the antigen-binding domain VH of the anti-PD-L1 HCAb antibody PR002082 and affinity-matured variants PR005263, PR005872, PR005875, PR005878, PR006246 and PR006248 were used to construct CD73 × PD-L1 bispecific antibody molecules.

The sequence numbers of anti-CD73 H2L2 antibodies are shown in Table 15, and the sequence numbers of anti-PD-L1 HCAb antibodies are shown in Table 16.

**Table 15. Sequence numbers (SEQ ID NO:) of anti-CD73 H2L2 antibodies**

| Antibody No. | Light chain | Heavy chain | VL | VH |
|---|---|---|---|---|
| PR000846 | 119 | 98 | 94 | 73 |
| PR001408 | 120 | 103 | 95 | 78 |
| PR003836 | 121 | 106 | 96 | 81 |

**Table 16. Sequence numbers (SEQ ID NO:) of anri-PD-L1 HCAb antibodies**

| **Antibody No.** | **Heavy chain** | **VH** |
|---|---|---|
| **PR005263** | 107 | 82 |
| **PR005872** | 111 | 86 |
| **PR005875** | 112 | 87 |
| **PR005878** | 113 | 88 |
| **PR006246** | 115 | 90 |
| **PR006248** | 117 | 92 |
| **PR002082** | 105 | 80 |

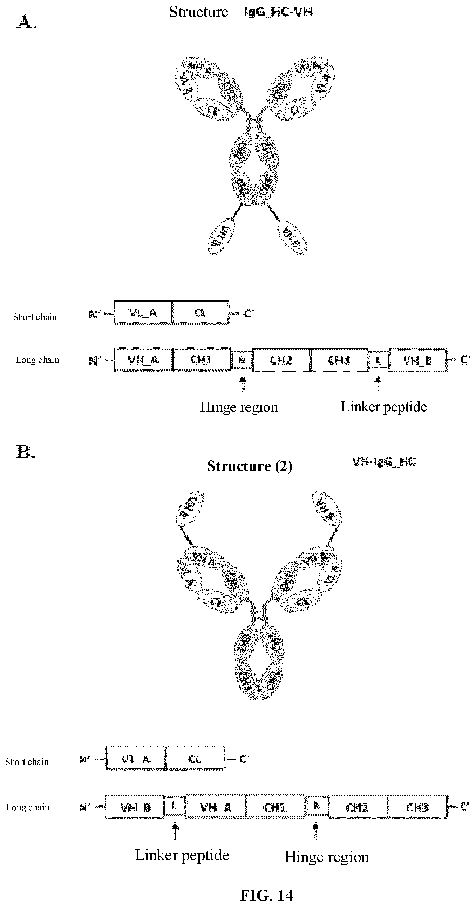

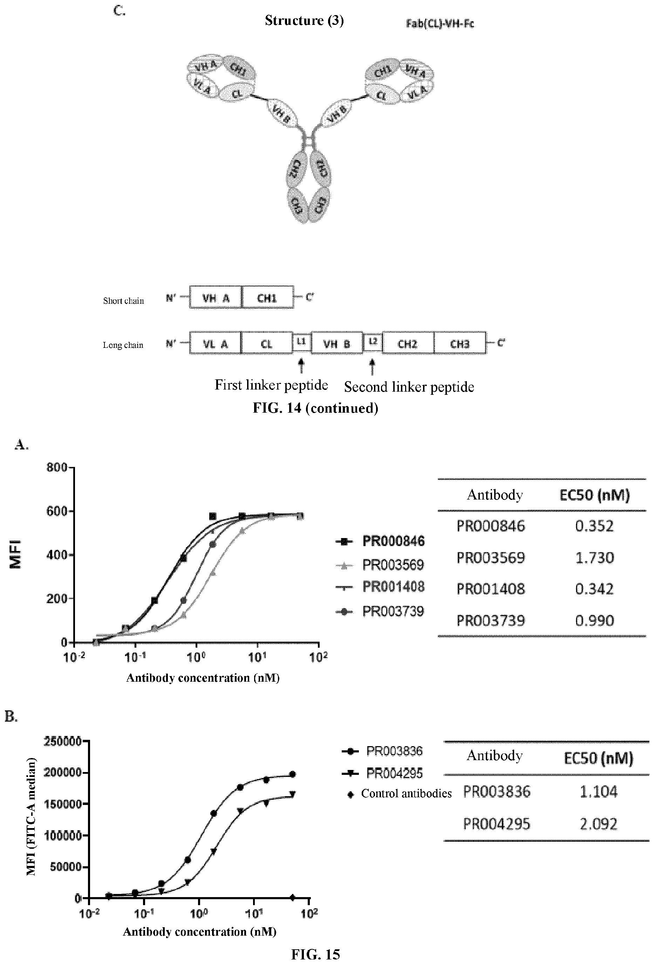

The molecular structures of the bispecific binding proteins included in the present application are listed in FIG. 14, each of which will be further described below. In this example and other parts of the present application, when referring to the number of polypeptide chains contained in a molecular structure, it generally refers to the number of "different polypeptide chains". For example, a conventional IgG antibody has two different polypeptide chains, i.e., a heavy chain and a light chain, and although the IgG antibody molecule itself is a tetrapeptide chain protein molecule containing two identical heavy chains and two identical light chains, its structural characteristics are described with specific reference to its two different polypeptide chains. Binding valence refers to the number of antigen-binding sites in the molecular structure. For example, a conventional IgG antibody can bind to two identical antigen molecules simultaneously, with a binding valence of two.

The sequences of the linker peptides that may be used in the structural design of the present application are listed in Table 17.

**Table 17. Sequence listing for linker peptides**

| **Names of linker peptides** | **Length of linker peptide** | **Sequence of linker peptide** | **Sequence number SEQ ID NO:** |
|---|---|---|---|
| GS_4 | 4 | GSGS | 136 |
| GS_5 | 5 | GGGGS | 137 |
| GS_6 | 6 | GGSGGS | 138 |
| GS_7 | 7 | GGGGSGS | 139 |
| GS_15 | 15 | GGGGSGGGGSGGGGS | 140 |
| GS_20 | 20 | GGGGSGGGGSGGGGSGGGGS | 141 |
| GS_25 | 25 | GGGGSGGGGSGGGGSGGGGSGGGGS | 142 |
| Human IgG1 hinge | 15 | EPKSCDKTHTCPPCP | 143 |
| Human IgG1 hinge (C220S) | 15 | EPKS**S**DKTHTCPPCP | 144 |
| H1_15 | 15 | EPKSSDKTHTPPPPP | 145 |
| G5-LH | 15 | GGGGGDKTHTCPPCP | 146 |
| H1_15-RT | 17 | EPKSSDKTHTPPPPPRT | 147 |
| L-GS_15-RT | 18 | LGGGGSGGGGSGGGGSRT | 148 |
| L-H1_15-RT | 18 | LEPKSSDKTHTPPPPPRT | 149 |
| KL-H1_15-RT | 19 | KLEPKSSDKTHTPPPPPRT | 150 |
| KL-H1_15-AS | 19 | KLEPKSSDKTHTPPPPPAS | 151 |
| RT-GS_5-KL | 9 | RTGGGGSKL | 152 |
| RT-GS_15-KL | 19 | RTGGGGSGGGGSGGGGSKL | 153 |
| RT-GS_25-KL | 29 | RTGGGGSGGGGSGGGGSGGGGSGGGGSKL | 154 |
| EPKSSD | 6 | EPKSSD | 155 |
| AS-GS_15 | 17 | ASGGGGSGGGGSGGGGS | 156 |
| GS_2 | 2 | GS | 157 |

### 9.1 Construction of bispecific antibodies of IgG_HC-VH tetravalent symmetric structure

The bispecific antibodies of IgG-VH tetravalent symmetric structure were constructed using anti-CD73 H2L2 antibodies and anti-PD-L1 HCAb antibodies. The binding protein of the **IgG_HC-VH** tetravalent symmetric structure (as shown in FIG. 14A) comprises two polypeptide chains: a polypeptide chain 1, also known as a short chain, from amino-terminus to carboxyl-terminus, comprising VL_A-CL; and a polypeptide chain 2, also known as a long chain, from amino-terminus to carboxyl-terminus, comprising VH_A-CH1-h-CH2-CH3-L-VH_B. h is a hinge region or derived sequence of an IgG antibody. In one embodiment, CH3 of the polypeptide chain 2 is fusion-linked directly to VH_B, i.e., L is 0 in length. In another embodiment, CH3 of the polypeptide chain 2 is linked to VH_B via the linker peptide L; L may be the sequence listed in Table 17.

### 9.2 Construction of bispecific antibodies of VH-IgG_HC tetravalent symmetric structure

The bispecific antibodies of IgG-VH tetravalent symmetric structure were constructed using anti-CD73 H2L2 antibodies and anti-PD-L1 HCAb antibodies. The binding protein of the **VH-IgG_HC** tetravalent symmetric structure (as shown in FIG. 14B) comprises two polypeptide chains: a polypeptide chain 1, also known as a short chain, from amino-terminus to carboxyl-terminus, comprising VL_A-CL; and a polypeptide chain 2, also known as a long chain, from amino-terminus to carboxyl-terminus, comprising VH_B-L-VH_A-CH1-h-CH2-CH3. h is a hinge region or derived sequence of an IgG antibody. VH_A of the polypeptide chain 2 is linked to VH_B via the linker peptide L; the linker peptide L may be the sequence listed in Table 17. In one embodiment, VH_A of the polypeptide chain 2 is fusion-linked directly to VH_B, i.e., L is 0 in length.

### 9.3 Construction of bispecific antibody molecules of Fab(CL)-VH-Fc structure

The bispecific antibodies of IgG-VH tetravalent symmetric structure were constructed using anti-CD73 H2L2 antibodies and anti-PD-L1 HCAb antibodies. The binding protein of an **Fab(CL)-VH-Fc** symmetric structure (as shown in FIG. 14C) comprises two polypeptide chains: a polypeptide chain 1, also known as a short chain, from amino-terminus to carboxyl-terminus, comprising VH_A-CH1; and a polypeptide chain 2, also known as a long chain, from amino-terminus to carboxyl-terminus, comprising VL_A-CL-L1-VH_B-L2-CH2-CH3. The linker peptides L1 and L2 of polypeptide chain 2 may be the sequences listed in Table 17. In one embodiment, CL of the polypeptide chain 2 is fusion-linked directly to VH_B, i.e., L1 is 0 in length.

CD73 × PD-L1 bispecific antibodies are IgG1, having Fc mutations L234Aand L235Aor L234Aand L235Aand G237A (numbered according to the EU index).

The CD73 × PD-L1 bispecific antibodies prepared by the present invention are summarized in Table 18; the amino acid sequence of the polypeptide chain of the resulting bispecific antibody molecules has sequence numbers shown in Table 19. The CDRs of the antigen-binding domains of bispecific antibody molecules have sequence numbers shown in Table 20. The prepared CD73 × PD-L1 bispecific antibody molecule samples were analyzed for physicochemical properties and summarized in Table 21.

**Table 18. CD73 × PD-L1 bispecific antibody molecule designed using anti-CD73 IgG antibody and anti-PD-L1 heavy-chain antibody**

| **Structure No.** | **Bispecific antibody molecules** | **CD73 antibody (IgG)** | **PD-L1 antibody (VH_B)** | **VH_B position relative to IgG** | **Linker peptide** | **Fc type (mutation)** |
|---|---|---|---|---|---|---|
| **2** | PR006268 | PR000846 | PR005263 | VH-IgG | GS_15 | IgG1_LALA |
| **2** | PR006269 | PR000846 | PR005872 | VH-IgG | GS_15 | IgG1_LALA |
| **2** | PR006270 | PR000846 | PR005875 | VH-IgG | GS_15 | IgG1_LALA |
| **2** | PR006271 | PR000846 | PR005878 | VH-IgG | GS_15 | IgG1_LALA |
| **1** | PR006663 | PR000846 | PR006246 | IgG-VH | GS_6 | IgG1_AAA |
| **3** | PR006667 | PR000846 | PR006246 | Cross Fab-HCAb | None | IGG1_AAA |
| **1** | PR006664 | PR000846 | PR006248 | IgG-VH | GS_6 | IGG1_AAA |
| **3** | PR006668 | PR000846 | PR006248 | Cross Fab-HCAb | None | IGG1_AAA |
| **2** | PR003569 | PR000846 | PR002082 | VH-IgG | GS_15 | IgG1_LALA |
| **2** | PR003739 | PR001408 | PR002082 | VH-IgG | GS_15 | IgG1_LALA |
| **2** | PR004295 | PR003836 | PR002082 | VH-IgG | GS_15 | IgG1_LALA |

**Table 19. Sequence numbers of CD73 × PD-L1 bispecific antibody molecules of the present application**

| **Structure No.** | **Antibody No.** | **Polypeptide chain 1** | **Polypeptide chain 2** |
|---|---|---|---|
| **2** | PR006268 | 125 | 119 |
| **2** | PR006269 | 126 | 119 |
| **2** | PR006270 | 127 | 119 |
| **2** | PR006271 | 128 | 119 |
| **1** | PR006663 | 129 | 119 |
| **3** | PR006667 | 132 | 131 |
| **1** | PR006664 | 130 | 119 |
| **3** | PR006668 | 133 | 131 |
| **2** | PR003569 | 122 | 119 |
| **2** | PR003739 | 123 | 120 |
| **2** | PR004295 | 124 | 121 |

**Table 20. Sequence numbers of CDRs of antigen-binding domain of CD73 × PD-L1 bispecific antibody molecules**

| **Structure No.** | **Antibody No.** | **Antigen-binding domain** | **LCDR 1** | **LCDR 2** | **LCDR 3** | **HCDR1** | **HCDR2** | **HCDR3** |
|---|---|---|---|---|---|---|---|---|
| **2** | PR006268 | CD73 | 51 | 57 | 65 | 8 | 22 | 35 |
| | | PD-L1 | None | None | None | 10 | 27 | 36 |
| **2** | PR006269 | CD73 | 51 | 57 | 65 | 8 | 22 | 35 |
| | | PD-L1 | None | None | None | 10 | 27 | 39 |
| **2** | PR006270 | CD73 | 51 | 57 | 65 | 8 | 22 | 35 |
| | | PD-L1 | None | None | None | 10 | 27 | 40 |
| **2** | PR006271 | CD73 | 51 | 57 | 65 | 8 | 22 | 35 |
| | | PD-L1 | None | None | None | 10 | 27 | 41 |
| **1** | PR006663 | CD73 | 51 | 57 | 65 | 8 | 22 | 35 |
| | | PD-L1 | None | None | None | 14 | 27 | 39 |
| **3** | PR006667 | CD73 | 51 | 57 | 65 | 8 | 22 | 35 |
| | | PD-L1 | None | None | None | 14 | 27 | 39 |
| **1** | PR006664 | CD73 | 51 | 57 | 65 | 8 | 22 | 35 |
| | | PD-L1 | None | None | None | 14 | 27 | 41 |
| **3** | PR006668 | CD73 | 51 | 57 | 65 | 8 | 22 | 35 |
| | | PD-L1 | None | None | None | 14 | 27 | 41 |
| **2** | PR003569 | CD73 | 51 | 57 | 65 | 8 | 22 | 35 |
| | | PD-L1 | None | None | None | 10 | 23 | 36 |
| **2** | PR003739 | CD73 | 52 | 58 | 66 | 12 | 25 | 37 |
| | | PD-L1 | None | None | None | 10 | 23 | 36 |
| **2** | PR004295 | CD73 | 53 | 59 | 67 | 10 | 26 | 38 |
| | | PD-L1 | None | None | None | 10 | 23 | 36 |

**Table 21. Expression and physicochemical properties of CD73 × PD-L1 bispecific antibody molecule proteins**

| **Structure No.** | **Bispecific antibody molecules** | **Expression system and volume** | **Yield (mg/L) after first purification** | **SEC-HPLC purity (%)** | **Endotoxin level (EU/mg)** |
|---|---|---|---|---|---|
| **2** | PR006268 | 100ml | 34.2 | 98.76% | <1 |
| **2** | PR006269 | 100ml | 32.2 | 98.59% | <1 |
| **2** | PR006270 | 100ml | 24.6 | 99.29% | <1 |
| **2** | PR006271 | 100ml | 38.2 | 98.70% | <1 |
| **1** | PR006663 | 40m1 | 43 | 98.41% | <0.1 |
| **3** | PR006667 | 40ml | 47.5 | 97.61% | <0.1 |
| **1** | PR006664 | 40ml | 43.75 | 97.93% | <0.1 |
| **3** | PR006668 | 40m1 | 68 | 96.14% | <0.1 |
| **2** | PR003569 | 1L | 66 | 99.4% | <0.2 |
| **2** | PR003739 | 1L | 40 | 99.42% | <0.2 |
| **2** | PR004295 | 40m1 | 69 | 94.7% | <0.6 |

### Example 10. Binding of CD73 × PD-L1 Bispecific Antibody to Cells Overexpressing Human PD-L1 or Human CD73 (FACS)

In order to investigate the binding activity of the CD73 × PD-L1 bispecific antibody to human CD73 and PD-L1, the binding experiment at the cellular level was performed using CHO-K1 cell strains overexpressing human CD73 or PD-L1 (CHO-K1-hCD73 or CHO-K1-hPD-L1). Briefly, the CHO-K1-hCD73 and CHO-K1-hPD-L1 cells were digested and resuspended in an F-12K complete medium, and the cell density was adjusted to 1×10⁶ cells/mL. The cells were seeded in a 96-well V-bottom plate (Corning, #3894) at 100 µL/well, followed by the addition at 100 µL/well and centrifugation at 500 g at 4 °C for 5 min, and then the supernatant was discarded. Antibodies were 3-fold diluted to a maximum final concentration of 50 nM, and a total of 8 concentrations were set. 100 µL of the diluted antibodies was added to the cells, and the cells were incubated away from light at 4 °C for 1 h. Thereafter, the cells in each well were rinsed twice with 100 µL of pre-cooled PBS, and centrifuged at 500 g at 4 °C for 5 min, and then the supernatant was discarded. Then 100 µL of a fluorescent secondary antibody (goat anti-human IgG (H+L) secondary antibody, Alexa Fluor^{®} 488 conjugate, Invitrogen, # A11013, diluted at the ratio of 1:1000) was added to each well. The plate was incubated away from light at 4 °C for 30 min. The cells in each well were then rinsed twice with 200 µL of pre-cooled PBS and centrifuged at 500 g at 4 °C for 5 min, and then the supernatant was discarded. Finally, the cells in each well were resuspended in 200 µL of pre-cooled PBS, and the fluorescence signal values were read using a BD FACS CANTOII.

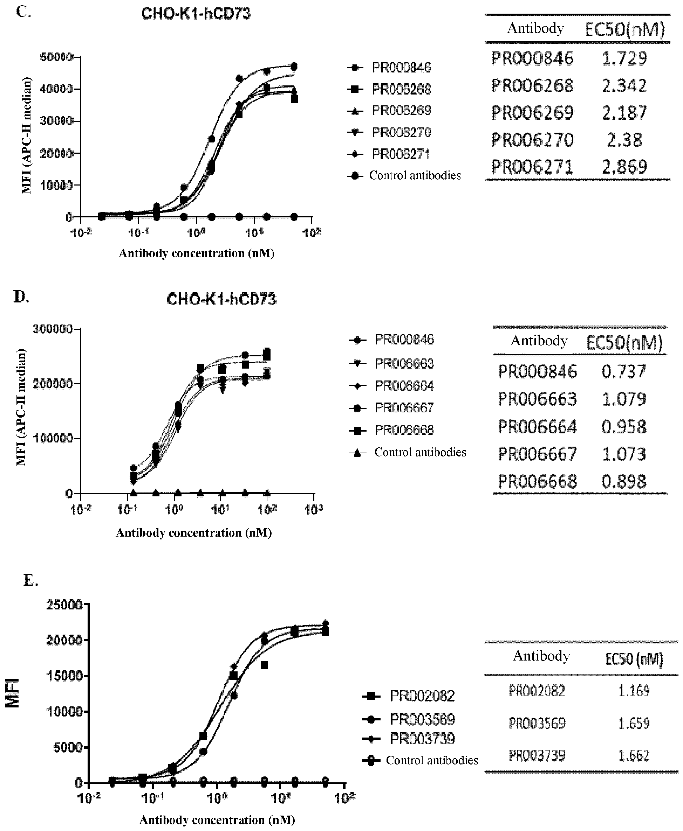

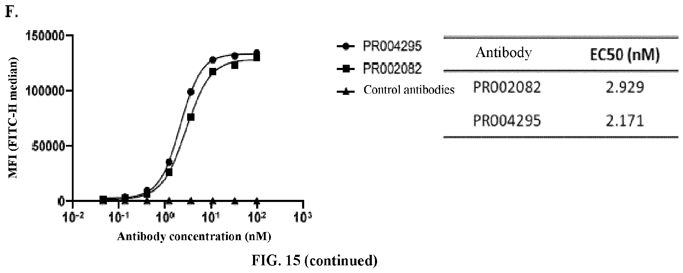

The results in FIG. 15 show that the anti-CD73 antibodies PR000846 and PR001408 of the present application can specifically bind to CHO-K1 cells overexpressing human CD73 (FIG. 15A); the CD73 × PD-L1 bispecific antibodies (PR003569 and PR003739) of the present application can bind to CHO-K1 cells overexpressing human CD73 (FIG. 15A); the anti-CD73 antibody PR003836 of the present application can specifically bind to CHO-K1 cells overexpressing human CD73 (FIG. 15B); the CD73 × PD-L1 bispecific antibody PR004295 of the present application can bind to CHO-K1 cells overexpressing human CD73 (FIG. 15B);
the anti-CD73 antibody PR000846 of the present application can specifically bind to CHO-K1 cells overexpressing human CD73 (FIG. 15C and FIG. 15D); the CD73 × PD-L1 bispecific antibodies (PR006268, PR006269, PR006270, PR006271, PR006661, PR006662, PR006663, PR006664, PR006667 and PR006668) of the present application can bind to CHO-K1 cells overexpressing human CD73 (FIG. 15C and FIG. 15D); the anri-PD-L1 antibody PR002082 of the present application can bind to CHO-K1 cells overexpressing human PD-L1 (FIG. 15E); the CD73 × PD-L1 bispecific antibodies PR003569, PR003739, and PR004295 of the present application can bind to CHO-K1 cells overexpressing human PD-L1 (FIG. 15E and FIG. 15F).

The anti-CD73 antibodies, the anti-PD-L1 antibodies, and the CD73 × PD-L1 bispecific antibodies of the present application had EC50 values in the range of 0.3418 nM-2.929 nM. In FIG. 15C, the initial concentration was 50 nM and diluted in a 3-fold gradient, with a total of 8 concentrations. In FIG. 15D, the initial concentration was 100 nM and diluted in a 3-fold gradient, with a total of 8 concentrations.

### Example 11. Inhibition of Enzymatic Activity of CD73 by CD73 × PD-L1 Bispecific Antibody

The enzymatic activity of antibodies against soluble recombinant CD73 was determined using the malachite green method. First, a 384-well plate (Corning, #3799) was added with 12.5 µL of 1 nM recombinant CD73 proteins and 12.5 µL of 1 nM antibodies (the experimental buffer was 25 mM Tris pH 7.5, 5 mM MgCl₂ and 0.005% Tween-20), and the plate was incubated at room temperature for 1 h. 25 µL of AMP (with a maximum concentration of 200 µM, 2-fold diluted with experimental buffer to 8 concentrations) was added, and the plate was incubated at room temperature for 15 min. The concentration of inorganic phosphate in each well was determined according to the manufacturer's instructions. After the determination was completed, the absorbance values at 620 nm were recorded using a Molecular Devices plate reader (SPECTRAMax plus384). The experimental results were analyzed and plotted using GraphPad Prism 8.0.

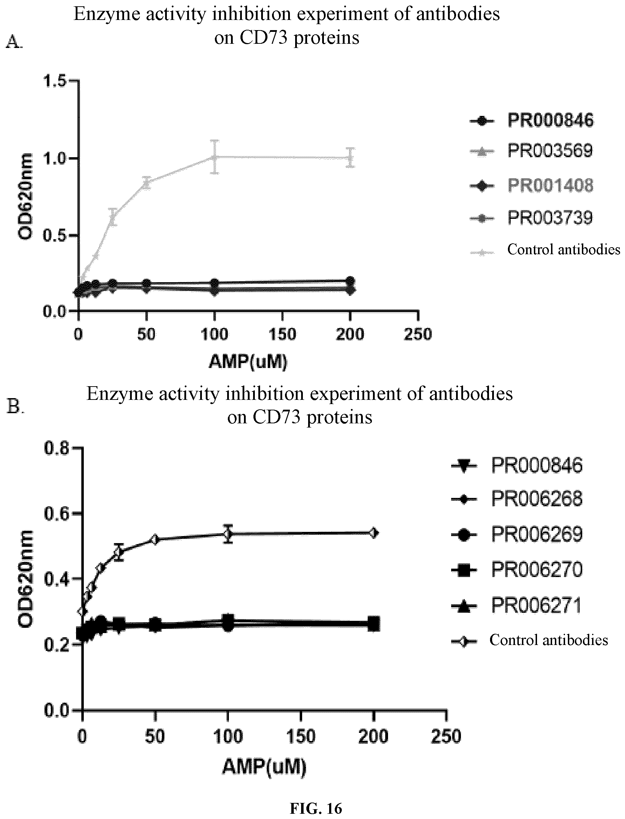

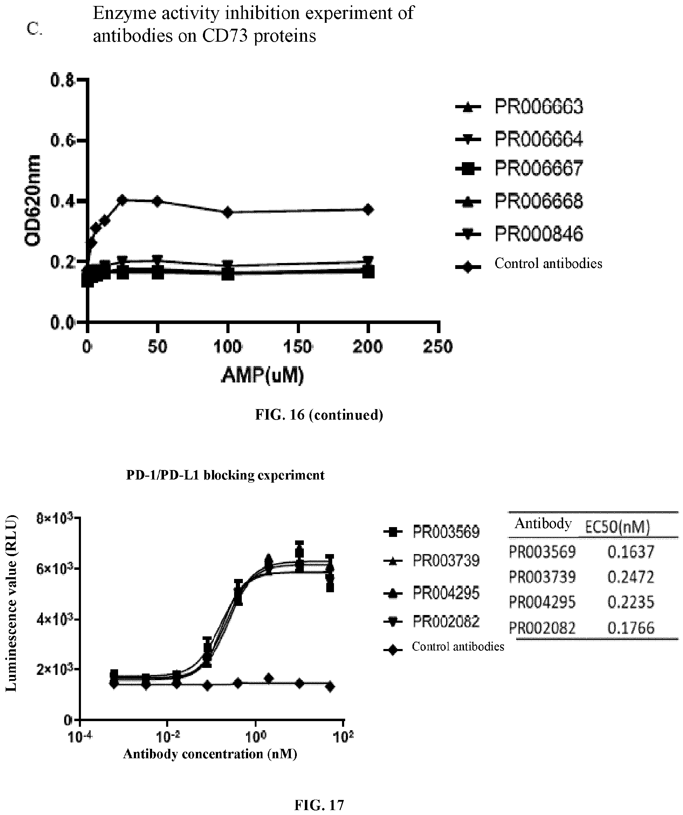

The results in FIG. 16 show that the CD73 × PD-L1 bispecific antibodies PR003569, PR003739, PR006268, PR006269, PR006270, PR006271, PR006663, PR006664, PR006667, and PR006668 of the present application can non-competitively inhibit the enzymatic activity of CD73 proteins, comparable to the activity of the parent monoclonal antibody PR000846.

### Example 12. Inhibition of PD-1 Signaling Pathway by CD73 × PD-L1 Bispecific Antibody Detected Using Reporter Gene Cell Line

HEK293T (eBioscience) cells expressing PD-L1 and OS8 (CD3 single-chain antibody transmembrane protein) were plated on a 96-well plate at 1.25×10⁴ cells/well, 100 µL/well. The cells were incubated at 37 °C with 5% CO₂ overnight. A dilution of the test antigen-binding protein was added at 50 µL/well. The initial concentration was 100 nM, and 5-fold dilution was performed. hlgG1 was used as a control group. Jurkat reporter cells capable of constantly expressing PD-1 and NFAT-luciferase reporter genes (UPharm) were added at 5×10⁴ cells/well, 50 µL/well. The cells were cultured at 37 °C with 5% CO₂ for 6 h. ONE-GloT luciferase reagent (Promega, #E6110) was added. The cells were incubated at room temperature for 5 min, and the luminescence values were measured using a microplate reader.

The results in FIG. 17 show that the CD73 × PD-L1 bispecific antibodies PR003569, PR003739 and PR004295 of the present application detected using the reporter gene cell line had inhibitory effects on the PD-1 signaling pathway.

### Example 13. Activation of T Cells by CD73 × PD-L1 Bispecific Antibody in Mixed Lymphocyte Reaction (MLR)

AllCells PBMC cells were purchased, CD14+ monocytes were isolated, and recombinant human interleukin 4 (IL-4) (R&D, #204-GMP) and human GM-CSF (R&D, #215-GM/CF) were added. After 6 days of induction, immature human dendritic cells (iDC cells) were obtained. 1 µg/mL lipopolysaccharide (LPS; Sigma, #L2630) was then added, and after 24 h of induction, mature dendritic cells (mDC cells) were obtained. T lymphocytes were isolated from PBMC cells of a second donor using a Pan-T cell isolation kit (Miltenyibiotec, #130-096-535). The T lymphocytes and mDC cells were seeded in a 96-well plate (T lymphocytes at 1×10⁵ cells/well and mDC cells at 1×10⁴ cells/well) at a ratio of 10:1. The diluted antigen-binding proteins and the negative and positive control antibodies were added. The cells were incubated in an incubator at 37 °C with 5% CO₂ for 5 days. Supernatants on day 3 and on day 5 were collected and detected for the secretion of IL-2 (ThermoFisher, #88-7025-88) and IFN-γ (ThermoFisher, #88-7316-88), respectively, using an ELISA kit.

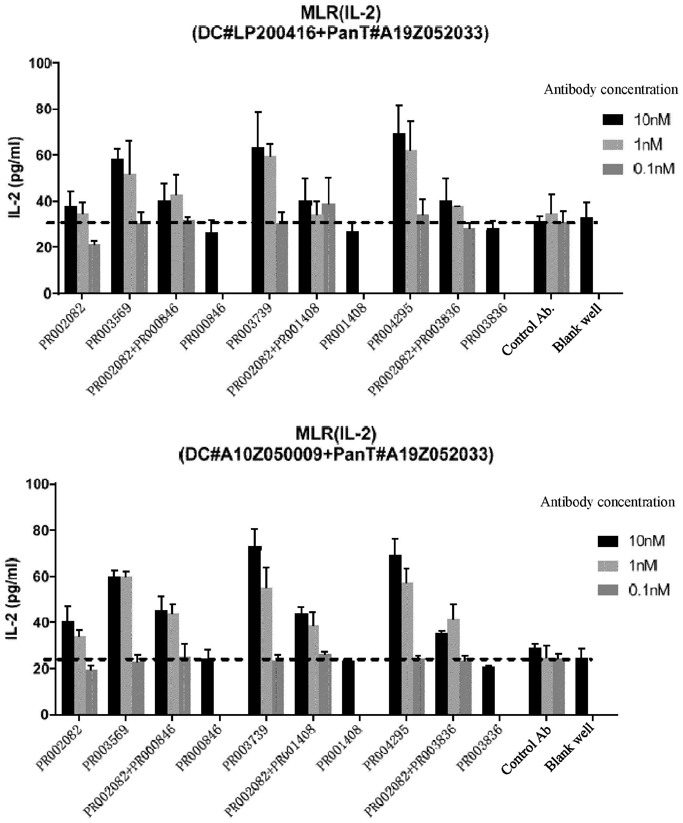

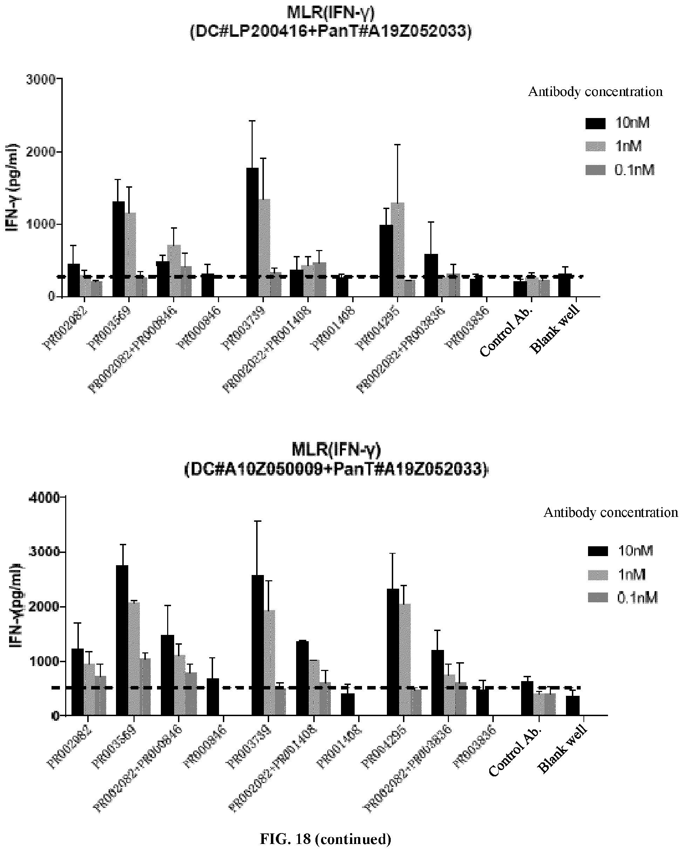

The results in FIG. 18 show that the CD73 × PD-L1 bispecific antibodies PR003569, PR003739 and PR004295 of the present application *in vitro* detected using the MLR method had activation effects on the T cells. The CD73 × PD-L1 bispecific antibodies PR003569, PR003739 and PR004295 are significantly more effective at activating the T cells to secret IL-2 at a concentration of 10 nM compared with human isotype IgG, compared with the anti-PD-L1 monoclonal antibody PR002082 alone, compared with the anti-CD73 monoclonal antibodies PR000846, PR001408 and PR003836 alone, compared with the anti-PD-L1 monoclonal antibody PR002082 in combination with anti-CD73 monoclonal antibody PR000846, and compared with the anti-PD-L1 monoclonal antibody PR002082 in combination with the anti-CD73 monoclonal antibody PR003836. Furthermore, based on the more significant effects of CD73 × PD-L1 bispecific antibodies PR003569, PR003739 and PR004295 compared with the anti-PD-L1 monoclonal antibody PR002082 in combination with the anti-CD73 monoclonal antibody PR000846, and compared with the anti-PD-L1 monoclonal antibody PR002082 in combination with the anti-CD73 monoclonal antibody PR003836, it is considered that the CD73 × PD-L1 bispecific antibodies PR003569, PR003739 and PR004295 have synergistic effects.

Similarly, CD73 × PD-L1 bispecific antibodies PR003569, PR003739 and PR004295 are significantly more effective at activating the T cells to secret IFN-γ at a concentration of 10 nM compared with human isotype IgG, compared with the anti-PD-L1 monoclonal antibody PR002082 alone, compared with the anti-CD73 monoclonal antibodies PR000846, PR001408 and PR003836 alone, compared with the anti-PD-L1 monoclonal antibody PR002082 in combination with anti-CD73 monoclonal antibody PR000846, and compared with the anti-PD-L1 monoclonal antibody PR002082 in combination with anti-CD73 monoclonal antibody PR003836. Furthermore, based on the more significant effects of CD73 × PD-L1 bispecific antibodies PR003569, PR003739 and PR004295 compared with the anti-PD-L1 monoclonal antibody PR002082 in combination with the anti-CD73 monoclonal antibody PR000846, and compared with the anti-PD-L1 monoclonal antibody PR002082 in combination with the anti-CD73 monoclonal antibody PR003836, it is considered that the CD73 × PD-L1 bispecific antibodies PR003569, PR003739 and PR004295 have synergistic effects.

## Claims

1. An isolated antigen-binding protein specifically binding to PD-L1 and/or a fragment thereof, comprising an antibody heavy chain variable region (VH), wherein the VH comprises the following complementary determining regions (HCDRs) or a mutant thereof:
HCDR1 set forth in an amino acid sequence of SEQ ID NO: 9;
HCDR2 set forth in an amino acid sequence of SEQ ID NO: 23; and/or
HCDR3 set forth in an amino acid sequence of SEQ ID NO: 36.

2. The isolated antigen-binding protein according to claim 1, wherein the mutant has an insertion, deletion or substitution of 1, 2, 3 or 4 amino acids in the amino acid sequences of the HCDR1, the HCDR2 and the HCDR3 of the VH,
preferably, the HCDR1 has an amino acid sequence of GFX₁FSX₂Y,
the HCDR2 has an amino acid sequence of X₃YX₄GX₅X₆, and
the HCDR3 has an amino acid sequence of NRAX₇FGVX₈PDX₉SDI,
wherein X₁, X₂, X₃, X₄, X₅, X₆, X₇, X₈ or X₉ is any one selected from N, T, D, S, W, R, K, E, I, A, V and L; and
more preferably, X₁ is T, D or N,
X₂ is N or S,
X₃ is W or R,
X₄ is D or T,
X₅ is T or S,
X₆ is K, R or E,
X₇ is I or L,
X₈ is V or I, and/or
X₉ is A or D.

3. The isolated antigen-binding protein according to claim 1 or 2, wherein the mutant of the HCDR1 has an amino acid sequence set forth in any one of SEQ ID NO: 10, SEQ ID NO: 11, SEQ ID NO: 13 and SEQ ID NO: 14.

4. The isolated antigen-binding protein according to claim 1 or 2, wherein the mutant of the HCDR2 has an amino acid sequence set forth in SEQ ID NO: 24 or SEQ ID NO: 27.

5. The isolated antigen-binding protein according to claim 1 or 2, wherein the mutant of the HCDR3 has an amino acid sequence set forth in any one of SEQ ID NO: 39, SEQ ID NO: 40 and SEQ ID NO: 41.

6. The isolated antigen-binding protein according to claim 1, wherein the VH comprises the following complementary determining regions (HCDRs) or a mutant thereof:
HCDR1 set forth in an amino acid sequence of any one of SEQ ID NOs: 9-11 and SEQ ID NOs: 13-14;
HCDR2 set forth in an amino acid sequence of any one of SEQ ID NOs: 23-24 and SEQ ID NO: 27; and/or
HCDR3 set forth in an amino acid sequence of any one of SEQ ID NO: 36 and SEQ ID NOs: 39-41.

7. The isolated antigen-binding protein according to claim 1 or 2, wherein the VH comprises the following complementary determining regions (HCDRs):
HCDR1, HCDR2 and HCDR3 set forth in SEQ ID NO: 9, SEQ ID NO: 23 and SEQ ID NO: 36, respectively; or
HCDR1, HCDR2 and HCDR3 set forth in SEQ ID NO: 10, SEQ ID NO: 23 and SEQ ID NO: 36, respectively; or
HCDR1, HCDR2 and HCDR3 set forth in SEQ ID NO: 11, SEQ ID NO: 24 and SEQ ID NO: 36, respectively; or
HCDR1, HCDR2 and HCDR3 set forth in SEQ ID NO: 11, SEQ ID NO: 23 and SEQ ID NO: 36, respectively; or
HCDR1, HCDR2 and HCDR3 set forth in SEQ ID NO: 13, SEQ ID NO: 23 and SEQ ID NO: 36, respectively; or
HCDR1, HCDR2 and HCDR3 set forth in SEQ ID NO: 10, SEQ ID NO: 23 and SEQ ID NO: 39, respectively; or
HCDR1, HCDR2 and HCDR3 set forth in SEQ ID NO: 10, SEQ ID NO: 23 and SEQ ID NO: 40, respectively; or
HCDR1, HCDR2 and HCDR3 set forth in SEQ ID NO: 10, SEQ ID NO: 23 and SEQ ID NO: 41, respectively; or
HCDR1, HCDR2 and HCDR3 set forth in SEQ ID NO: 10, SEQ ID NO: 27 and SEQ ID NO: 36, respectively; or
HCDR1, HCDR2 and HCDR3 set forth in SEQ ID NO: 10, SEQ ID NO: 27 and SEQ ID NO: 39, respectively; or
HCDR1, HCDR2 and HCDR3 set forth in SEQ ID NO: 10, SEQ ID NO: 27 and SEQ ID NO: 40, respectively; or
HCDR1, HCDR2 and HCDR3 set forth in SEQ ID NO: 10, SEQ ID NO: 27 and SEQ ID NO: 41, respectively; or
HCDR1, HCDR2 and HCDR3 set forth in SEQ ID NO: 14, SEQ ID NO: 27 and SEQ ID NO: 36, respectively; or
HCDR1, HCDR2 and HCDR3 set forth in SEQ ID NO: 14, SEQ ID NO: 27 and SEQ ID NO: 39, respectively; or
HCDR1, HCDR2 and HCDR3 set forth in SEQ ID NO: 14, SEQ ID NO: 27 and SEQ ID NO: 40, respectively; or
HCDR1, HCDR2 and HCDR3 set forth in SEQ ID NO: 14, SEQ ID NO: 27 and SEQ ID NO: 41, respectively.

8. The isolated antigen-binding protein according to claim 1 or 2, further comprising a heavy chain variable region framework region (VH FWR), wherein the VH FWR comprises: VH FWR1 having an amino acid sequence set forth in any one of SEQ ID NOs: 3-5; VH FWR2 having an amino acid sequence set forth in SEQ ID NO: 17 or SEQ ID NO: 20; VH FWR3 having an amino acid sequence set forth in SEQ ID NO: 30 or SEQ ID NO: 31; and VH FWR4 having an amino acid sequence set forth in SEQ ID NO: 43.

9. The isolated antigen-binding protein according to claim 1 or 2, wherein the VH comprises an amino acid sequence set forth in any one of SEQ ID NOs: 74-77, SEQ ID NOs: 79-80 and SEQ ID NOs: 82-92 or an amino acid sequence having at least 80%, 85%, 88%, 90%, 92%, 95%, 97%, 98%, 99% or 100% identity thereto.

10. The isolated antigen-binding protein according to claim 1 or 2, further comprising an Fc region, or a region equivalent to an Fc region of an immunoglobulin, preferably, the Fc region is a human Fc, and more preferably, the human Fc is a human IgG1 Fc.

11. The isolated antigen-binding protein according to claim 10, wherein the Fc comprises mutations L234A, L235A and P329G, or mutations L234A and L235A, or comprises one, two or three mutations selected from S298A, E333A and K334A, and more preferably comprises mutations S298A, E333A and K334A.

12. The isolated antigen-binding protein according to claim 1 or 2, comprising a heavy chain with an amino acid sequence having at least 80%, 85%, 88%, 90%, 92%, 95%, 97%, 98%, 99% or 100% identity to SEQ ID NO: 99, 100, 101, 102, 104, 105, 107, 108, 109, 110, 111, 112, 113, 114, 115, 116 or 117.

13. The isolated antigen-binding protein according to claim 1 or 2, being an antigen-binding fragment in a form of HCAb or in a form of a nanobody.

14. An isolated nucleic acid, encoding the isolated antigen-binding protein according to any one of claims 1-13.

15. An expression vector, comprising the isolated nucleic acid according to claim 14.

16. A host cell, comprising the isolated nucleic acid according to claim 14 or the expression vector according to claim 15.

17. An antibody-drug conjugate, comprising the isolated antigen-binding protein according to any one of claims 1-13 and a drug covalently linked to the antigen-binding protein.

18. The antibody-drug conjugate according to claim 17, wherein the drug is selected from a chemotherapeutic agent, a radiotherapeutic agent, an immunosuppressant and a cytotoxic drug.

19. A chimeric antigen receptor, comprising an extracellular antigen-binding domain, a transmembrane domain and an intracellular signaling domain, wherein the extracellular antigen-binding domain comprises the isolated antigen-binding protein according to any one of claims 1-13.

20. A modified immune cell, comprising the chimeric antigen receptor according to claim 19.

21. A multispecific antibody, comprising two or more antigen-binding domains, wherein one of the antigen-binding domains comprises the isolated antigen-binding protein according to any one of claims 1-13.

22. A pharmaceutical composition, comprising the isolated antigen-binding protein according to any one of claims 1-13, the antibody-drug conjugate according to claim 17 or 18, the chimeric antigen receptor according to claim 19, the modified immune cell according to claim 20, or the multispecific antibody according to claim 21, and a pharmaceutically acceptable carrier.

23. The pharmaceutical composition according to claim 22, further comprising a therapeutic agent selected from a chemotherapeutic agent, a radiotherapeutic agent, an immunosuppressant and a cytotoxic drug.

24. Use of the isolated antigen-binding protein according to any one of claims 1-13, the isolated nucleic acid according to claim 14, the antibody-drug conjugate according to claim 17 or 18, the chimeric antigen receptor according to claim 19, the modified immune cell according to claim 20, the multispecific antibody according to claim 21, or the pharmaceutical composition according to claim 22 or 23 in the preparation of a medicament for preventing, treating and/or diagnosing immune diseases, acute and chronic inflammatory diseases, and tumor diseases.

25. The use according to claim 24, wherein the tumor is one or more of breast cancer, renal cell carcinoma, melanoma, colon cancer, B-cell lymphoma, melanoma, head and neck cancer, bladder cancer, stomach cancer, ovarian cancer, malignant sarcoma, urothelial cancer, liver cancer, esophageal cancer, gastroesophageal junction cancer, nasopharyngeal cancer, small cell lung cancer, cervical cancer, endometrial cancer, pancreatic cancer, prostate cancer, glioma, non-small cell lung cancer, acute myeloid leukemia, Hodgkin lymphoma, cutaneous squamous cell carcinoma, and locally advanced and metastatic malignancies;
the inflammatory disease is one or more of atopic dermatitis or ulcerative colitis; and
the immune disease is one or more of graft-versus-host disease, rheumatoid arthritis, systemic lupus erythematosus or asthma.

26. A method for detecting PD-L1 in a sample, comprising the step of detecting PD-L1 in the sample with the isolated antigen-binding protein according to any one of claims 1-13, wherein the sample is whole blood, red blood cell concentrate, platelet concentrate, white blood cell concentrate, tissue, bone marrow aspirate, plasma, serum, cerebrospinal fluid, feces, urine, cultured cell, saliva, oral secretion and/or nasal secretion; and preferably, the method is for non-diagnosis purposes.

27. A specific binding protein, comprising at least two domains and capable of binding to PD-L1 or a fragment thereof, and/or CD73 or a fragment thereof.

28. The specific binding protein according to claim 27, comprising a first domain and a second domain, wherein the first domain binds to CD73 or a fragment thereof, the second domain binds to PD-L1 or a fragment thereof, and the first domain is linked to the second domain to form a bispecific binding protein.

29. The specific binding protein according to claim 28, wherein the first domain is a CD73 antibody or an antigen-binding fragment thereof, the second domain is a PD-L1 antibody or an antigen-binding fragment thereof, the first domain and/or the second domain are/is in a form selected from IgG, Fab, Fab', F(ab')₂, Fv, scFv, VH or HCAb, and preferably, the number of the Fab, Fab', F(ab')₂, Fv, scFv or VH is one or more than one.

30. The specific binding protein according to claim 28, wherein the first domain is in the form of IgG, and preferably, a heavy chain constant region of the IgG is a human heavy chain constant region, more preferably a human IgG1, human IgG2, human IgG3 or human IgG4 heavy chain constant region.

31. The specific binding protein according to claim 30, wherein the IgG comprises one, two or three mutations selected from L234A, L235A and P329G, and more preferably comprises mutations L234Aand L235A; or comprises one, two or three mutations selected from S298A, E333Aand K334A, and more preferably comprises mutations S298A, E333A and K334A; and
preferably, an Fc of the IgG is an Fc of human IgG1.

32. The specific binding protein according to claim 28, wherein the first domain is in the form of Fab, and more preferably, the first domain comprises 2 Fabs.

33. The specific binding protein according to claim 28, wherein the second domain is a VH,
preferably, the VH is a human VH, and more preferably, the second domain has two VHs.

34. The specific binding protein according to claim 28, wherein the second domain is in a form of HCAb, and preferably, a constant region of the HCAb comprises mutations L234A, L235A and P329G, or mutations L234Aand L235A, or comprises one, two or three mutations selected from S298A, E333A and K334A, and more preferably comprises mutations selected from S298A, E333A and K334A

35. The specific binding protein according to claim 28, wherein the first domain is linked to the second domain directly or linked to the second domain via a linker peptide L to form the bispecific binding protein, and the second domain is linked to a C- or N-terminus of the first domain.

36. The specific binding protein according to claim 35, wherein the bispecific binding protein comprises a short chain and a long chain, wherein the short chain has a structure set forth in N'-VL₁-CL₁-C' and the long chain has a structure set forth in N'-VH₁-CH₁-h-CH₂-CH₃-L-VH₂-C';
optionally, the short chain has a structure set forth in N'-VL₁-CL₁-C' and the long chain has a structure set forth in N'-VH₂-L-VH₁-CH₁-h-CH₂-CH₃-C';
optionally, the short chain has a structure set forth in N'-VH₁-CH₁-C' and the long chain has a structure set forth in N'-VL₁-CL₁-L-VH₂-CH₂-CH₃-C',
wherein the VL₁ and the VH₁ are a VL and a VH of the first domain, respectively, the VH₂ is a VH of the second domain, the h is a hinge region, the L is the linker peptide, and the CL₁ is a CL of the first domain.

37. The specific binding protein according to claim 35 or 36, wherein the linker peptide L is a peptide having a length of 0-30 amino acids, which preferably has an amino acid sequence set forth in any one of SEQ ID NOs: 136-157.

38. The specific binding protein according to claim 35 or 36, wherein, in the long chain, the CH₃ is linked to the VH₂ via the linker peptide L; the linker peptide L is a polypeptide of 0-30 amino acids, and preferably, the L comprises an amino acid sequence set forth in any one of SEQ ID NOs: 136-157; or, in the long chain, the CH₃ is linked to the VH₂ directly.

39. The specific binding protein according to claim 35 or 36, wherein, in the long chain, the VH₁ is linked to the VH₂ via the linker peptide L; the L is a peptide having a length of 0-30 amino acids, and preferably, the L comprises an amino acid sequence set forth in any one of SEQ ID NOs: 136-157; or in the long chain, the VH₁ is linked to the VH₂ directly.

40. The specific binding protein according to claim 35 or 36, wherein in the long chain, the VH₂ is linked to the CL₁ via the linker peptide L; the L is an amino acid sequence set forth in any one of SEQ ID NOs: 136-157; or in the long chain, the VH₂ is linked to the CL₁ directly.

41. The specific binding protein according to claim 28, wherein the first domain comprises a light chain variable region VL and a heavy chain variable region VH, wherein the VL comprises a complementary determining region selected from:
LCDR1 set forth in an amino acid sequence of any one of SEQ ID NO: 51, SEQ ID NO: 52 and SEQ ID NO: 53;
LCDR2 set forth in an amino acid sequence of any one of SEQ ID NO: 57, SEQ ID NO: 58 and SEQ ID NO: 59; and/or
LCDR3 set forth in an amino acid sequence of any one of SEQ ID NO: 65, SEQ ID NO: 66 and SEQ ID NO: 67; and the VH comprises a complementary determining region selected from:
HCDR1 set forth in an amino acid sequence of any one of SEQ ID NO: 8, SEQ ID NO: 12 and SEQ ID NO: 10;
HCDR2 set forth in an amino acid sequence of any one of SEQ ID NO: 22, SEQ ID NO: 25 and SEQ ID NO: 26; and/or
HCDR3 set forth in an amino acid sequence of any one of SEQ ID NO: 35, SEQ ID NO: 37 and SEQ ID NO: 38.

42. The specific binding protein according to claim 28, wherein the first domain comprises a light chain variable region VL and a heavy chain variable region VH, wherein the VL and the VH comprise complementary determining regions (CDRs) selected from:
LCDR1, LCDR2 and LCDR3 set forth in SEQ ID NO: 51, SEQ ID NO: 57 and SEQ ID NO: 65, respectively, and
HCDR1, HCDR2 and HCDR3 set forth in SEQ ID NO: 8, SEQ ID NO: 22 and SEQ ID NO: 35, respectively;
LCDR1, LCDR2 and LCDR3 set forth in SEQ ID NO: 52, SEQ ID NO: 58 and SEQ ID NO: 66, respectively, and
HCDR1, HCDR2 and HCDR3 set forth in SEQ ID NO: 12, SEQ ID NO: 25 and SEQ ID NO: 37, respectively; and
LCDR1, LCDR2 and LCDR3 set forth in SEQ ID NO: 53, SEQ ID NO: 59 and SEQ ID NO: 67, respectively, and
HCDR1, HCDR2 and HCDR3 set forth in SEQ ID NO: 10, SEQ ID NO: 26 and SEQ ID NO: 38, respectively.

43. The specific binding protein according to claim 28, wherein the first domain comprises a VL and a VH, wherein an amino acid sequence of the VL has at least 80%, 85%, 88%, 90%, 92%, 95%, 97%, 98%, 99% or 100% identity to SEQ ID NO: 94, SEQ ID NO: 95 or SEQ ID NO: 96; and
an amino acid sequence of the VH has at least 80%, 85%, 88%, 90%, 92%, 95%, 97%, 98%, 99% or 100% identity to SEQ ID NO: 73, SEQ ID NO: 78 or SEQ ID NO: 81.

44. The specific binding protein according to claim 28, wherein the first domain comprises a VL and a VH, wherein amino acid sequences of the VL and the VH are set forth in SEQ ID NO: 94 and SEQ ID NO: 73, respectively, or set forth in SEQ ID NO: 95 and SEQ ID NO: 78, respectively, or set forth in SEQ ID NO: 96 and SEQ ID NO: 81, respectively.

45. The specific binding protein according to claim 28, wherein the first domain comprises a light chain with an amino acid sequence having at least 80%, 85%, 88%, 90%, 92%, 95%, 97%, 98%, 99% or 100% identity to SEQ ID NO: 119, SEQ ID NO: 120 or SEQ ID NO: 121, and a heavy chain with an amino acid sequence having at least 80%, 85%, 88%, 90%, 92%, 95%, 97%, 98%, 99% or 100% identity to SEQ ID NO: 98, SEQ ID NO: 103 or SEQ ID NO: 106.

46. The specific binding protein according to claim 28, wherein the first domain comprises:
a light chain set forth in SEQ ID NO: 119 and a heavy chain set forth in SEQ ID NO: 98; or
a light chain set forth in SEQ ID NO: 120 and a heavy chain set forth in SEQ ID NO: 103; or
a light chain set forth in SEQ ID NO: 121 and a heavy chain set forth in SEQ ID NO: 106.

47. The specific binding protein according to claim 28, wherein the second domain is the isolated antigen-binding protein according to any one of claims 1-13.

48. The specific binding protein according to claim 28, wherein a long chain of the specific binding protein comprises an amino acid sequence having at least 80%, 85%, 88%, 90%, 92%, 95%, 97%, 98%, 99% or 100% identity to SEQ ID NO: 122, 123, 124, 125, 126, 127, 128, 129, 130, 132 or 133; and a short chain of the specific binding protein comprises an amino acid sequence having at least 80%, 85%, 88%, 90%, 92%, 95%, 97%, 98%, 99% or 100% identity to SEQ ID NO: 119, 120, 121 or 131.

49. The specific binding protein according to claim 28, wherein the specific binding protein has:
a long chain set forth in SEQ ID NO: 122 and a short chain set forth in SEQ ID NO: 119; or
a long chain set forth in SEQ ID NO: 123 and a short chain set forth in SEQ ID NO: 120; or
a long chain set forth in SEQ ID NO: 124 and a short chain set forth in SEQ ID NO: 121; or
a long chain set forth in SEQ ID NO: 125 and a short chain set forth in SEQ ID NO: 119; or
a long chain set forth in SEQ ID NO: 126 and a short chain set forth in SEQ ID NO: 119; or
a long chain set forth in SEQ ID NO: 127 and a short chain set forth in SEQ ID NO: 119; or
a long chain set forth in SEQ ID NO: 128 and a short chain set forth in SEQ ID NO: 119; or
a long chain set forth in SEQ ID NO: 129 and a short chain set forth in SEQ ID NO: 119; or
a long chain set forth in SEQ ID NO: 132 and a short chain set forth in SEQ ID NO: 131; or
a long chain set forth in SEQ ID NO: 130 and a short chain set forth in SEQ ID NO: 119; or
a long chain set forth in SEQ ID NO: 133 and a short chain set forth in SEQ ID NO: 131.

50. The specific binding protein according to claim 28, wherein the bispecific binding protein comprises two first polypeptide chains and two second polypeptide chains, which form a bivalent structure or a tetravalent symmetric structure.

51. An isolated nucleic acid, encoding the specific binding protein according to any one of claims 28-50 or a fragment thereof.

52. An expression vector, comprising the isolated nucleic acid according to claim 51.

53. Ahost cell, comprising the isolated nucleic acid according to claim 51 or the expression vector according to claim 52.

54. An antibody-drug conjugate, comprising the isolated antigen-binding protein according to any one of claims 28-50 and a drug covalently linked to the antigen-binding protein.

55. The antibody-drug conjugate according to claim 54, wherein the drug is selected from a chemotherapeutic agent, a radiotherapeutic agent, an immunosuppressant and a cytotoxic drug.

56. A pharmaceutical composition, comprising the specific binding protein according to any one of claims 28-50, or the antibody-drug conjugate according to claim 54 or 55, and a pharmaceutically acceptable carrier.

57. The pharmaceutical composition according to claim 56, further comprising a therapeutic agent selected from a chemotherapeutic agent, a radiotherapeutic agent, an immunosuppressant and a cytotoxic drug.

58. Use of the specific binding protein according to any one of claims 28-50, the isolated nucleic acid according to claim 51, the antibody-drug conjugate according to claim 54 or 55, or the pharmaceutical composition according to claim 56 or 57 in the preparation of a medicament for preventing, treating and/or diagnosing immune diseases, acute and chronic inflammatory diseases, and tumor diseases.

59. The use according to claim 58, wherein the tumor is one or more of breast cancer, renal cell carcinoma, melanoma, colon cancer, B-cell lymphoma, melanoma, head and neck cancer, bladder cancer, stomach cancer, ovarian cancer, malignant sarcoma, urothelial cancer, liver cancer, esophageal cancer, gastroesophageal junction cancer, nasopharyngeal cancer, small cell lung cancer, cervical cancer, endometrial cancer, pancreatic cancer, prostate cancer, glioma, non-small cell lung cancer, acute myeloid leukemia, Hodgkin lymphoma, cutaneous squamous cell carcinoma, and locally advanced and metastatic malignancies;
the inflammatory disease is one or more of atopic dermatitis or ulcerative colitis; and
the immune disease is one or more of graft-versus-host disease, rheumatoid arthritis, systemic lupus erythematosus or asthma.

60. A method for detecting PD-L1 and CD73 in a sample, comprising the step of detecting PD-L1 and CD73 in the sample with the specific binding protein according to any one of claims 28-50, wherein the sample is whole blood, red blood cell concentrate, platelet concentrate, white blood cell concentrate, tissue, bone marrow aspirate, plasma, serum, cerebrospinal fluid, feces, urine, cultured cell, saliva, oral secretion and/or nasal secretion.

61. A kit of parts, comprising one or more kits, wherein each kit comprises the antigen-binding protein according to any one of claims 1-13, the antibody-drug conjugate according to claim 17 or 18, or the pharmaceutical composition according to claim 22 or 23.

62. The kit of parts according to claim 61, comprising a first kit and a second kit, wherein the first kit comprises the antigen-binding protein according to any one of claims 1-13, the antibody-drug conjugate according to claim 17 or 18, or the pharmaceutical composition according to claim 22 or 23; and the second kit comprises a therapeutic agent selected from a chemotherapeutic agent, a radiotherapeutic agent, an immunosuppressant and a cytotoxic drug.

63. A kit of parts, comprising one or more kits, wherein each kit comprises the specific binding protein according to any one of claims 28-50, the antibody-drug conjugate according to claim 54 or 55, or the pharmaceutical composition according to claim 56 or 57.

64. The kit of parts according to claim 63, comprising a first kit and a second kit, wherein the first kit comprises the antigen-binding protein according to any one of claims 28-50, the antibody-drug conjugate according to claim 54 or 55, or the pharmaceutical composition according to claim 56 or 57; and the second kit comprises a therapeutic agent selected from a chemotherapeutic agent, a radiotherapeutic agent, an immunosuppressant and a cytotoxic drug.

65. An administration device, comprising the antigen-binding protein according to any one of claims 1-13, the antibody-drug conjugate according to claim 17 or 18, or the pharmaceutical composition according to claim 22 or 23, wherein preferably, the administration device is a pre-filled syringe.

66. An administration device, comprising the specific binding protein according to any one of claims 28-50, the antibody-drug conjugate according to claim 54 or 55, or the pharmaceutical composition according to claim 56 or 57, wherein preferably, the administration device is a pre-filled syringe.

67. A method for preventing, treating and/or diagnosing tumor diseases, acute and chronic inflammatory diseases, and/or immune diseases, comprising administering to a subject a therapeutically effective amount of the antigen-binding protein according to any one of claims 1-13, the antibody-drug conjugate according to claim 17 or 18, or the pharmaceutical composition according to claim 22 or 23.

68. A method for preventing, treating and/or diagnosing tumor diseases, acute and chronic inflammatory diseases, and/or immune diseases, comprising administering to a subject a therapeutically effective amount of the antigen-binding protein according to any one of claims 28-50, the antibody-drug conjugate according to claim 54 or 55, or the pharmaceutical composition according to claim 56 or 57.

69. The method according to claim 67 or 68, wherein the tumor is one or more of breast cancer, renal cell carcinoma, melanoma, colon cancer, B-cell lymphoma, melanoma, head and neck cancer, bladder cancer, stomach cancer, ovarian cancer, malignant sarcoma, urothelial cancer, liver cancer, esophageal cancer, gastroesophageal junction cancer, nasopharyngeal cancer, small cell lung cancer, cervical cancer, endometrial cancer, pancreatic cancer, prostate cancer, glioma, non-small cell lung cancer, acute myeloid leukemia, Hodgkin lymphoma, cutaneous squamous cell carcinoma, and locally advanced and metastatic malignancies;
the inflammatory disease is one or more of atopic dermatitis or ulcerative colitis; and
the immune disease is one or more of graft-versus-host disease, rheumatoid arthritis, systemic lupus erythematosus or asthma.
